# EUROPEAN PATENT SPECIFICATION

(11) **EP 3 147 294 B1**
(45) Date of publication and mention of the grant of the patent: **29.04.2020**
(21) Application number: 15002759.7
(22) Date of filing: 24.09.2015
(51) Int. Cl.: C07K 14/005, G01N 33/569

(54) **DISTINGUISHING DENGUE VIRUS INFECTIONS FROM OTHER FLAVIVIRAL INFECTIONS USING A RECOMBINANT MUTANT ENVELOPE PROTEIN**
UNTERSCHEIDUNG VON DENGUE-VIRUSINFEKTIONEN VON ANDEREN FLAVIVIRALEN INFEKTIONEN MITTELS EINEM REKOMBINANTEN MUTANTEN HÜLLPROTEIN
DIFFÉRENCIATION D'INFECTIONS PAR LE VIRUS DE LA DENGUE PROVENANT D'AUTRES INFECTIONS FLAVIVIRALES À L'AIDE D'UNE PROTÉINE D'ENVELOPPE MUTANTE RECOMBINANTE

(43) Date of publication of application: 29.03.2017
(73) Proprietor: Fraunhofer-Gesellschaft zur Förderung der angewandten Forschung e.V., 80686 München (DE)
(72) Inventor: Ulbert, Sebastian, 04277 Leipzig (DE); Rockstroh, Alexandra, 04157 Leipzig (DE)
(74) Representative: Lahrtz, Fritz

(56) References cited:
- WO-A1-2014/016360
- WO-A1-2015/139784
- WO-A2-2011/146933
- BLOK J ET AL: "Comparison of a dengue-2 virus and its candidate vaccine derivative: Sequence relationships with the flaviviruses and other viruses", VIROLOGY, ELSEVIER, AMSTERDAM, NL, vol. 187, no. 2, 1 April 1992 (1992-04-01) , pages 573-590, XP026462278, ISSN: 0042-6822, DOI: 10.1016/0042-6822(92)90460-7 [retrieved on 1992-04-01]
- STEFAN CHABIERSKI ET AL: "Distinguishing West Nile virus infection using a recombinant envelope protein with mutations in the conserved fusion-loop", BMC INFECTIOUS DISEASES, vol. 14, no. 1, 9 May 2014 (2014-05-09), page 246, XP021185800, ISSN: 1471-2334, DOI: 10.1186/1471-2334-14-246
- CRISTINA DOMINGO ET AL: "International external quality control assessment for the serological diagnosis of dengue infections", BMC INFECTIOUS DISEASES, BIOMED CENTRAL, LONDON, GB, vol. 15, no. 1, 1 April 2015 (2015-04-01), page 167, XP021216922, ISSN: 1471-2334, DOI: 10.1186/S12879-015-0877-0
- CRILL W D ET AL: "Localization and characterization of flavivirus envelope glycoprotein cross-reactive epitopes", JOURNAL OF VIROLOGY, THE AMERICAN SOCIETY FOR MICROBIOLOGY, US, vol. 78, no. 24, 1 December 2004 (2004-12-01), pages 13975-13986, XP002374044, ISSN: 0022-538X, DOI: 10.1128/JVI.78.24.13975-13986.2004
- KERZHNER A ET AL: "Serological Dengue Diagnosis Using a Modified Envelope Protein", Abstracts, 25. Jahrestagung der Gesellschaft für Virologie e.V. (GfV), 18.3.-21.3.2015, Bochum, 18 March 2015 (2015-03-18), page P169, XP055496301, Retrieved from the Internet: URL:http://www.virology-meeting.de/fileadm in/congress/media/gfv/abstracts-ws/GfV2015 _Abstracts_Diagnostic_Methods.pdf [retrieved on 2018-07-31]
- CRILL W D ET AL: "A detailed mutagenesis study of flavivirus cross-reactive epitopes using West Nile virus-like particles", JOURNAL OF GENERAL VIROLOGY, vol. 88, no. 4, 1 April 2007 (2007-04-01), pages 1169-1174, XP055149549, ISSN: 0022-1317, DOI: 10.1099/vir.0.82640-0
- CHIOU S ET AL: "Enzyme-Linked Immunosorbent Assays Using Novel Japanese Encephalitis Virus Antigen Improve the Accuracy of Clinical Diagnosis of Flavivirus Infections", CLINICAL AND DIAGNOSTIC LABORATORY IMMUNOLOGY, vol. 15, no. 5, 1 May 2008 (2008-05-01), pages 825-835, XP055568880, US ISSN: 1556-6811, DOI: 10.1128/CVI.00004-08
- CHIOU S ET AL: "Mutation analysis of the cross-reactive epitopes of Japanese encephalitis virus envelope glycoprotein", JOURNAL OF GENERAL VIROLOGY, vol. 93, no. Pt_6, 15 February 2012 (2012-02-15), pages 1185-1192, XP055149551, ISSN: 0022-1317, DOI: 10.1099/vir.0.040238-0

## Description

The present disclosure relates to a mutant peptide which is a mutated form of a fragment of a peptide representing the wildtype ectodomain of the E protein of a dengue virus, wherein
(i) the fragment of a peptide representing the wildtype ectodomain of the E protein of a dengue virus:
   (a) consists of amino acids 1 to 396 of the peptide representing the wildtype ectodomain of the E protein of a dengue virus, or
   (b) consists of amino acids 1 to 397 of the peptide representing the wildtype ectodomain of the E protein of a dengue virus, or
   (c) consists of amino acids 1 to 398 of the peptide representing the wildtype ectodomain of the E protein of a dengue virus, or
   (d) consists of amino acids 1 to 399 of the peptide representing the wildtype ectodomain of the E protein of a dengue virus, or
   (e) consists of amino acids 1 to 400 of the peptide representing the wildtype ectodomain of the E protein of a dengue virus, or
   (f) consists of amino acids 1 to 401 of the peptide representing the wildtype ectodomain of the E protein of a dengue virus, or
   (g) consists of amino acids 1 to 402 of the peptide representing the wildtype ectodomain of the E protein of a dengue virus, or
   (h) consists of amino acids 1 to 403 of the peptide representing the wildtype ectodomain of the E protein of a dengue virus,
   and
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
and wherein the dengue virus (DENV) is of serotype 1, 2, 3, 4 or 5. The present disclosure further relates to compositions comprising mutated peptides which are mutated forms of a peptide representing the wildtype ectodomain of the E protein of a dengue virus, as well as kits, kits-of-parts, methods and uses relating thereto.

Dengue virus (DENV) is a mosquito-transmitted pathogen of the family Flaviviridae, a group of small, enveloped and positive stranded RNA-viruses. Besides DENV there are several other human pathogenic vector-borne flaviviruses, such as Yellow Fever virus (YFV), West Nile virus (WNV), Tick-borne Encephalitis virus (TBEV) or Japanese Encephalitis virus (JEV) [1]. DENV is endemic to over a hundred tropical and subtropical countries worldwide, and the numbers of annual infections are strongly increasing with a current estimate of 400 million [2]. High fever is the most common clinical symptom of a DENV-infection, but also severe complications are observed, such as dengue shock syndrome (DHS) or dengue haemorrhagic fever (DHF). Approx. half a million hospitalizations and several thousands of fatalities are caused by DENV every year [3,4] . Dengue viruses include four major distinct serotypes, named DENV-1 to DENV-4, and survival of an infection with one of these serotypes leads to a lifelong immunity to this serotype, but not to the others [5]. In parallel to the increasing distribution of its vector, mosquitos from the genus Aedes, DENV is emerging or re-emerging in several areas including Europe and North America. In Europe local transmission of the virus has been demonstrated in France and Croatia in 2010 [6,7]. In addition, a DENV-outbreak occurred in Madeira in 2012 and resulted in over 2000 cases and transportation of the virus into several other European countries [8].During the acute phase of infection, Dengue can be diagnosed by directly detecting viral RNA or the non-structural protein 1 (NS1), which is secreted by infected cells. About five days after onset of symptoms, these direct infection markers start to decrease in blood and DENV-IgM antibodies appear, followed by IgG a couple of days later. Therefore, except during the acute phase, DENV-infections are usually diagnosed by antibody-measurements, and several serological test systems are available [reviewed in 9,10]. Whereas IgM-detection can diagnose recent primary infections, IgG-measurements are useful for detection of secondary infections, where IgM responses usually remain lower, and for serological surveillance activities. Current dengue infections can be confirmed via a rise in IgM or IgG levels in paired samples [10]. One of the major problems in antibody-based diagnosis of dengue is the similarity of structural proteins of different flaviviruses which leads to cross-reactive antibodies and false positive test results [11-13]. Especially in areas where several flaviviruses co-circulate or in the context of vaccination against e.g. YFV or TBEV, this is of concern [1,14]. As the currently available tests cannot exclude flavivirus cross-reactivity, positive test results have to be confirmed by virus neutralization tests, which are time consuming and require BSL-3 laboratories. The E (envelope) protein is a major target of the human antibody response during DENV infections and is used in most available tests [15]. Cross-reactive antibodies target mainly the highly conserved fusion loop (FL) domain of the E protein which is involved in fusion of the viral and cellular membranes [16-19]. As a consequence, the insertion of mutations into the FL leads to a decrease in binding of cross-reactive antibodies, which has been employed to develop diagnostic methods on the basis of WNV- or JEV- virus-like particles (VLPs) [20-22]. Alternatively, other recombinant antigens than the E protein, such as NS1, have been used to differentiate flavivirus antibodies via titer-determinations [23].

We recently have shown that a bacterially expressed E protein from WNV bearing four point mutations in the FL and an adjacent loop domain can be used to serologically distinguish WNV- from TBEV and DENV-infections [24]. Mutations at these four positions were previously shown to eliminate binding of cross-reactive antibodies from heterologous flavivirus infections [22].

Therefore, there is a need for an improved diagnostic test for specifically diagnosing infections with a Dengue virus and for distinguishing infections with a Dengue virus from other flaviviral infections such as an infection with West Nile virus (WNV), Yellow Fever virus (YFV), Japan Encephalitis virus (JEV), or Tick-borne encephalitis virus (TBEV).

In the present invention, quadruple mutant forms of the E proteins from the different DENV-serotypes were generated in insect cells and analyzed as a diagnostic tool. Surprisingly, the results with these peptides in a diagnostic in vitro method show a high potential of the peptides, methods, kits, kits-of-parts and uses of the present invention for the development of a specific serological DENV-assay.

The problem is solved by the mutant peptides and compositions comprising mutant peptides and methods and uses relating thereto. We generated insect-cell derived recombinant E-proteins of the four major DENV-serotypes which contain point mutations in the FL domain. By using specific mixtures of these mutant antigens, cross-reactivity against heterologous flaviviruses was strongly reduced, enabling sensitive and specific diagnosis of the DENV-infected serum samples in IgG and IgM-measurements.

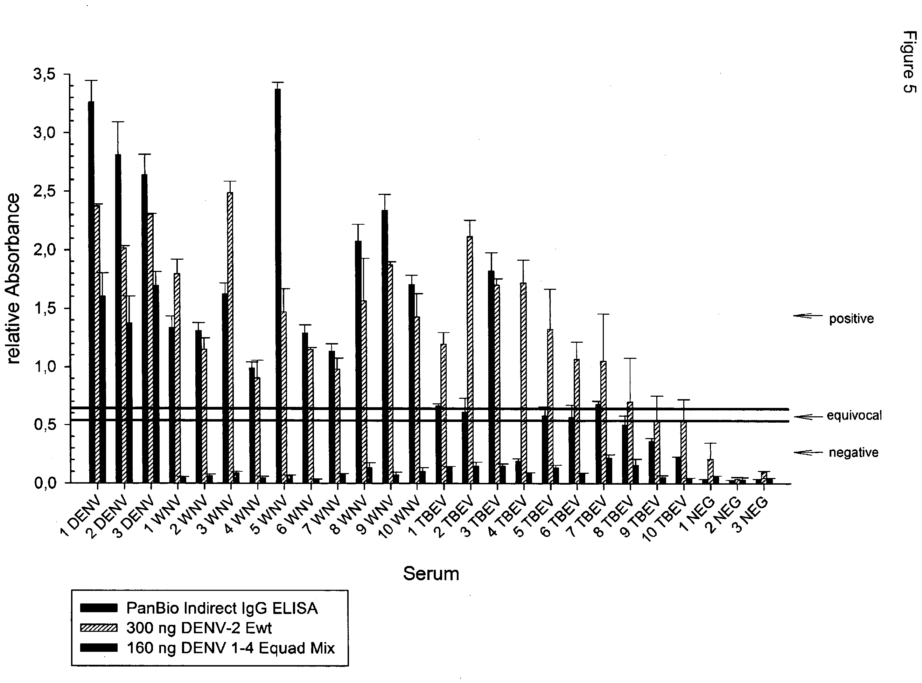

As shown in the Examples, the peptides of the disclosure, compositions, kits and kits-of-parts of the present invention, as well as methods and uses thereto are surprisingly useful for a serological DENV-test with improved specificity. As shown in Figure 5, the serological DENV-test of the present disclosure is clearly superior as compared to the commercially available test for DENV.

In particular, the serological diagnosis of dengue is severely complicated by cross-reactivity between antibodies against different flaviviruses. Currently available tests cannot rule out false positive results due to infections or vaccinations with related pathogens such as West Nile virus or Yellow Fever virus. Most cross-reactive antibodies target the conserved fusion loop (FL) domain in the E protein (the major component of the viral envelope). Therefore, we generated insect-cell derived E proteins of DENV from the four different serotypes with mutations in the FL and set up an ELISA-based platform. Using these antigens we were able to detect DENV-infections with high sensitivity. In addition, cross-reactivity with a variety of heterologous flavivirus-infections was eliminated.

The peptides of the disclosure, compositions, kits and kits-of-parts of the present invention, as well as methods and uses thereto are surprisingly useful for the development of simple and sensitive serological DENV-tests with greatly improved specificity.

In one embodiment, the present disclosure relates to a mutant peptide which is a mutated form of a fragment of a peptide representing the wildtype ectodomain of the E protein of a dengue virus, wherein
(i) the fragment of a peptide representing the wildtype ectodomain of the E protein of a dengue virus:
   (a) consists of amino acids 1 to 396 of the peptide representing the wildtype ectodomain of the E protein of a dengue virus, or
   (b) consists of amino acids 1 to 397 of the peptide representing the wildtype ectodomain of the E protein of a dengue virus, or
   (c) consists of amino acids 1 to 398 of the peptide representing the wildtype ectodomain of the E protein of a dengue virus, or
   (d) consists of amino acids 1 to 399 of the peptide representing the wildtype ectodomain of the E protein of a dengue virus, or
   (e) consists of amino acids 1 to 400 of the peptide representing the wildtype ectodomain of the E protein of a dengue virus, or
   (f) consists of amino acids 1 to 401 of the peptide representing the wildtype ectodomain of the E protein of a dengue virus, or
   (g) consists of amino acids 1 to 402 of the peptide representing the wildtype ectodomain of the E protein of a dengue virus, or
   (h) consists of amino acids 1 to 403 of the peptide representing the wildtype ectodomain of the E protein of a dengue virus,
   and
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
and wherein the dengue virus (DENV) is of serotype 1, 2, 3, 4 or 5.

As summarized and reviewed in Perera R. et al. (Antiviral Res. 2008 Oct; 80(1): 11-22), the flavivirus virion consists of an outer glycoprotein shell and an internal host derived lipid bilayer that surrounds the capsid and viral RNA. During virus entry, envelope (E) proteins forming the glycoprotein shell bind cell to surface receptors that assist in internalizing the virus through clathrin-mediated endocytosis. Following internalization, the low pH of the endosome triggers structural rearrangements in the viral glycoproteins that drive fusion of the viral and endocytic membranes to release the viral RNA into the cytoplasm. This RNA (∼11kb) is directly translated as a single polyprotein that is processed by viral and cellular proteases into 3 structural proteins (C, prM and E) and 7 non-structural proteins (NS1, NS2A/B, NS3, NS4A/B and NS5). The non-structural proteins actively replicate the viral RNA in replication complexes associated with cellular membranes. As further reviewed in Perera, R. et al., the E protein forms the glycoprotein shell of the virus. It is a class II fusion protein that shares about -40% amino acid identity among the flaviviruses. The atomic structure for the ectodomain of E has been solved for DENV-2, DENV-3, WNV and TBEV. The protein consists of three β-barrel domains. Domain I (DI) contains the N-terminus, but is centrally located in the molecule. Domain II (DII) is rather elongated, mediates dimerization of E. The distal end of DII contains the putative fusion loop, a conserved glycine-rich, hydrophobic sequence. Dill of the WNV E protein is an Ig-like domain with seven β-strand at the C-terminal end of the ectodomain.

An ectodomain is understood as a domain of a membrane protein that extends into the extracellular or extraviral space. In a DENV E protein, the ectodomain preferably corresponds to amino acids 1 to 404 of the E protein. A domain preferably forms a compact three-dimensional structure and often can be stable and folded independent from the remaining part of the whole protein.

The present mutant peptides could be obtained successfully by recombinant expression including a heterologous sequence encompassing a purification tag in insect cells, as shown in the examples.

A "peptide" is preferably understood as a linear chain of 10 to 500 amino acid monomers linked by peptide bonds. Preferably, the amino acid monomers are naturally occurring amino acids, more preferably naturally occurring L-amino acids. The peptides may be unmodified, or may contain modifications at the C-terminus, the N-terminus and/or side chains. For example, the peptides may be amidated and/or carboxylated at the termini. Suitable L-amino acids are: Glycine, Alanine, Valine, Leucine, Isoleucine, Serine, Cysteine, Threonine, Methionine, Proline, Phenylalanine, Tyrosine, Tryptophan, Histidine, Lysine, Arginine, Aspartate, Glutamate, Asparagine, and Glutamine. A preferred modification of a side chain is an S-S bond formed between two Cys residues.

A "quadruple mutant" peptide is preferably understood as mutant peptide which contains four mutations as compared to the corresponding wildtype peptide. Therefore, a quadruple mutant peptide differs from the corresponding wildtype peptide in four amino acids. "T76A" is preferably understood as that the T (threonine) residue at position 76 from the N terminus of the wildtype peptide is mutated to A (alanine).

Methods for introducing mutations in a peptide sequence are known in the art. For example, in case of recombinant expression, adequate primers and cloning procedures may be used to express such mutated peptide sequence. Alternatively, the peptides of the invention may be produced by chemical synthesis by methods known in the art.

In a further preferred embodiment, the peptide of the disclosure is produced recombinantly, preferably produced recombinantly by bacterial expression, more preferably by expression in *Escherichia coli,* and/or wherein the peptide is purified after an oxidative refolding protocol.

In a more preferred embodiment, the mutant peptide is produced recombinantly, and is produced in a eukaryotic cell, more preferably a eukaryotic cell selected from an insect cell, yeast cell and mammalian cell. Such eukaryotic expression systems are well known to a skilled person. *S*. *cerevisiae, S*. *pombe* or *Hansenula* strains may be used as yeast host cells. In particular, an insect cell, in particular in a *Drosophila* S2 cell, as described in the Examples may be used. Insect cells were found to be surprisingly useful and efficient for the recombinant production of mutant peptides and were found to be clearly superior as compared to expression in bacterial cells.

In case expression in insect cells is intended, S2 cells, in particular *Drosophila* S2 cells, may be used. In this preferred embodiment, the peptides are preferably produced by stable transfection of insect cells, in particular S2 cells.

The mutant peptides of the disclosure may be glycosylated or unglycosylated. The mutant peptides are preferably glycosylated. Bacterial expression usually results in denatured forms of unglycosylated peptides, which can be refolded into their correct conformation.

A recombinantly produced peptide has the advantage over mammalian cell-culture derived VLPs (virus-like particles) as it can be produced rapidly, inexpensively, and in higher yield, and can be quantified more precisely for diagnostic applications.

Therefore, in one preferred embodiment, the mutant peptides of the disclosure are not part of a virus-like particle and are not part of a virus particle.

In a further preferred embodiment, the mutant peptide exhibits a three-dimensional folding and/or is refolded after recombinant expression, and/or the mutant peptide is purified and/or is not part of a virus-like particle or a virus particle.

In order to determine that the mutant peptides and wildtype peptides employed in kits, methods and uses of the invention exhibit three-dimensional folding, one or more of the following analyses can be performed preferably (see Oliphant T, Nybakken GE, Austin SK, Xu Q, Bramson J, Loeb M, Throsby M, Fremont DH, Pierson TC, Diamond MS for WNV E proteins: Induction of epitope-specific neutralizing antibodies against West Nile virus. J Virol 2007, 81:11828-11839): (i) elution as a monodispersed peak at the appropriate size on a gel filtration column; (ii) immunoreactivity with expected MAbs based on parallel yeast surface display studies; (iii) mass spectrometry analysis; (iv) circular dichroism spectroscopy; and (v) crystallographic analysis.

As shown in the Examples, the ectodomain of the mixture of quadruple mutants T76R, Q77E, W101R, L107R of fragments of the DENV E proteins of serotypes 1, 2, 3 and 4 were used successfully for discriminating a DENV infection from other flaviviral infections. The ectodomain of the DENV E protein preferably corresponds to amino acids 1 to 404 of the E protein as described above.

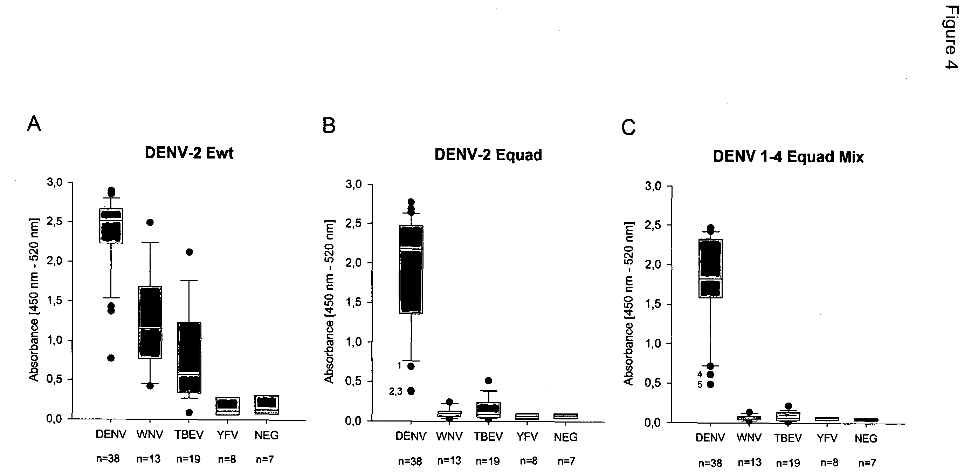

In particular, by quantifying the binding of human antisera to the mixture of T76R, Q77E, W101R, L107R quadruple mutant ectodomains of DENV serotypes 1 to 4, it was surprisingly possible to discriminate between DENV infections on the one hand and WNV, YFV and TBEV infections on the other hand in a statistically significant manner (Figure 4).

A fragment of a peptide representing the wildtype ectodomain of the E protein of a dengue virus is understood as a peptide, which contains at least 300 contiguous amino acids of the E protein of a dengue virus, and wherein at least 1 C-terminal amino acid, and optionally one or more N-terminal amino acids of the wildtype ectodomain of the E protein are deleted, in particular wherein the ectodomain corresponds to amino acids 1 to 404 of the E protein. Preferably, the fragment exhibits three-dimensional folding. In a preferred, embodiment, 1, 2, 3, 4, 5, 6, 7, 8, 9, or 10 C-terminal amino acid are deleted, and optionally 1, 2, 3, 4 or 5 N-terminal amino acids. In the examples, a mutated form of the fragment of a peptide representing the wildtype ectodomain of the E protein of a dengue virus which consists of amino acids 1 to 399 of the peptide representing the wildtype ectodomain of the E protein of a dengue virus was used for serotypes 1, 2 and 4, and a mutated form of the fragment of a peptide representing the wildtype ectodomain of the E protein of a dengue virus which consists of amino acids 1 to 397 of the peptide representing the wildtype ectodomain of the E protein of a dengue virus was used for serotype 3. Accordingly, the C-terminal 4 amino acids (serotypes 1, 2 and 4) or 6 amino acids (serotype 3) were deleted in the fragments of the Examples.

The sequence of a mutant peptide used in the examples which is a mutated form of a fragment of a peptide representing the wildtype ectodomain of the E protein of a dengue virus, wherein
(i) the fragment of a peptide representing the wildtype ectodomain of the E protein of a dengue virus consists of amino acids 1 to 399 of the peptide representing the wildtype ectodomain of the E protein of a dengue virus serotype 1,
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
   is shown in SEQ ID No: 10.

The sequence of a mutant peptide used in the examples which is a mutated form of a fragment of a peptide representing the wildtype ectodomain of the E protein of a dengue virus, wherein
(i) the fragment of a peptide representing the wildtype ectodomain of the E protein of a dengue virus consists of amino acids 1 to 399 of the peptide representing the wildtype ectodomain of the E protein of a dengue virus serotype 2,
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
   is shown in SEQ ID No: 11.

The sequence of a mutant peptide used in the examples which is a mutated form of a fragment of a peptide representing the wildtype ectodomain of the E protein of a dengue virus, wherein
(i) the fragment of a peptide representing the wildtype ectodomain of the E protein of a dengue virus consists of amino acids 1 to 397 of the peptide representing the wildtype ectodomain of the E protein of a dengue virus serotype 3,
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
   is shown in SEQ ID No: 12.

The sequence of a mutant peptide used in the examples which is a mutated form of a fragment of a peptide representing the wildtype ectodomain of the E protein of a dengue virus, wherein
(i) the fragment of a peptide representing the wildtype ectodomain of the E protein of a dengue virus consists of amino acids 1 to 399 of the peptide representing the wildtype ectodomain of the E protein of a dengue virus serotype 4,
(ii) the mutant peptide is mutated at positions 76, 77, 101 and 107 as follows:
   - Threonine at position 76 to mutated to Arginine (T76R),
   - Glutamine at position 77 is mutated to Glutamate (Q77E),
   - Tryptophane at position 101 is mutated to Arginine (W101R), and
   - Leucine at position 107 is mutated to Arginine (L107R),
is shown in SEQ ID No: 13.

In the Examples, the mutant peptides further contained the heterologous sequence of SEQ ID No: 9 at the C-terminus. The heterologous sequence in the Examples consists of a short linker sequences GSG and a His-tag.

Even among a DENV serotype, the sequences of the ectodomain of the E proteins of different virus strains may differ. Accordingly, it is possible to use various strains of a specific Dengue virus serotype.

In the examples, the DENV-1 (or DENV serotype 1) is the Nauru/West Pac/1974 strain. The ectodomain of the E protein of this strain has the sequence pursuant to SEQ ID No: 1. This strain is particularly preferred. Alternative DENV-1 strains are known in the art. For example, the E protein with the sequence of SEQ ID No: 18 may be used.

In the examples, the DENV-2 (or DENV serotype 2) is the 16681 strain. The ectodomain of the E protein of this strain has the sequence pursuant to SEQ ID No: 2. This strain is particularly preferred. Alternative DENV-2 strains are known in the art. For example, the E protein with the sequence of SEQ ID No: 19 may be used.

In the examples, the DENV-3 (or DENV serotype 3) is the Sri Lanka/1266/2000 strain. The ectodomain of the E protein of this strain has the sequence pursuant to SEQ ID No: 3. This strain is particularly preferred. Alternative DENV-3 strains are known in the art. For example, the E protein with the sequence of SEQ ID No: 20 may be used.

In the examples, the DENV-4 (or DENV serotype 4) is the Dominica/814669/1981 strain. The ectodomain of the E protein of this strain has the sequence pursuant to SEQ ID No: 4.

This strain is particularly preferred. Alternative DENV-4 strains are known in the art. For example, the E protein with the sequence of SEQ ID No: 21 may be used.

Accordingly, in one preferred embodiment of the present disclosure, the fragment of a peptide representing the wildtype ectodomain of the E protein of a dengue virus consists of amino acids 1 to 397 of the peptide representing the wildtype ectodomain of the E protein of a dengue virus.

Accordingly, in another preferred embodiment of the present disclosure the fragment of a peptide representing the wildtype ectodomain of the E protein of a dengue virus consists of amino acids 1 to 398 of the peptide representing the wildtype ectodomain of the E protein of a dengue virus.

Accordingly, in yet another preferred embodiment of the present disclosure the fragment of a peptide representing the wildtype ectodomain of the E protein of a dengue virus consists of amino acids 1 to 399 of the peptide representing the wildtype ectodomain of the E protein of a dengue virus,

In another preferred embodiment of the present disclosure the mutant peptide does not contain further mutations than the mutations at positions 76, 77, 101 and 107. A mutant peptide which does not contain further mutations than the four mutations at positions 76, 77, 101 and 107 is also referred to as quadruple mutant in the present application.

Various serotypes of Dengue virus are known, such as DENV-1 or serotype 1, DENV-2 or serotype 2, DENV-3 or serotype 3, DENV-4 or serotype 4, and DENV-5 or serotype 5. DENV-1, DENV-2, DENV-3 and DENV-4 are the major serotypes. As shown in the examples, a mixture of mutant peptides which are mutated forms of a fragment of an ectodomain of an E protein of Dengue viruses of serotypes 1, 2, 3 and 4 were surprisingly useful for discriminating a Dengue virus infection from various other flaviviral infections.

Accordingly, in another preferred embodiment the dengue virus (DENV) is of serotype 1, 2, 3, or 4.

In a further preferred embodiment the wildtype ectodomain of the E protein of a dengue virus (DENV) of serotype 1 has the sequence pursuant to SEQ ID No: 1.

In a further preferred embodiment, the wildtype ectodomain of the E protein of a dengue virus (DENV) of serotype 2 has the sequence pursuant to SEQ ID No: 2.

In a further preferred embodiment, the wildtype ectodomain of the E protein of a dengue virus (DENV) of serotype 3 has the sequence pursuant to SEQ ID No: 3.

In a further preferred embodiment, the wildtype ectodomain of the E protein of a dengue virus (DENV) of serotype 4 has the sequence pursuant to SEQ ID No: 4.

According to the present invention, the expression "T76R, Q77E, W101R, L107R mutant of the peptide" is understood as that the peptide is mutated at positions 76, 77, 101 and 107 as compared to the wildtype sequence as follows:
- Threonine at position 76 to mutated to Arginine (T76R),
- Glutamine at position 77 is mutated to Glutamate (Q77E),
- Tryptophane at position 101 is mutated to Arginine (W101R), and
- Leucine at position 107 is mutated to Arginine (L107R).

In a further embodiment, the present disclosure relates to a mutant peptide having the sequence of the T76R, Q77E, W101R, L107R mutant of the peptide representing the wildtype ectodomain of the E protein of a dengue virus (DENV) or a fragment thereof, wherein
the peptide representing the wildtype ectodomain consists of a sequence selected from SEQ ID No: 1, SEQ ID No: 2, SEQ ID No: 3 and SEQ ID No: 4,
and wherein the fragment thereof consists of a sequence wherein
- 1, 2, 3, 4, 5, 6, 7, 8, 9, or 10 of the N-terminal amino acids of the wildtype ectodomain of the E protein are deleted, and/or
- 1, 2, 3, 4, 5, 6, 7, 8, 9 or 10 of the C-terminal amino acids of the wildtype ectodomain of the E protein are deleted.

For example, 1, 2, 3, 4, 5, 6, 7, 8, 9, or 10 of the N-terminal amino acids of the wildtype ectodomain of the E protein of SEQ ID No: 1, and/or 1, 2, 3, 4, 5, 6, 7, 8, 9 or 10 of the C-terminal amino acids of the wildtype ectodomain of the E protein of SEQ ID No: 1 may be deleted in the T76R, Q77E, W101 R, L107R mutant form thereof.

The mutant peptides of the disclosure may contain one or more further moieties. In a more preferred embodiment, the one or more further moieties are selected from a heterologous sequence, in particular comprising or consisting of a heterologous peptide tag, such as a purification tag, a labeling moiety, such as a fluorescent moiety, and a separation moiety, such as a biotin moiety. Preferably, the one or more further moieties is not a homologous sequence. The one or more further moieties may be bound covalently or non-covalently, preferably covalently. The one or more further moieties may be added to the N-terminal amino acid, to the C-terminal amino acid and/or to one or more internal amino acid(s).

For example the mutant peptides may contain one or more heterologous peptide tags. Such heterologous peptide tags may have a length of 1 to 500 amino acids, preferably of 1, 2, 3, 4, 5, 6, 7, 8, 9, 10 or more and up to 15, 20, 50, 100, 200 or 500 amino acids. For example, the heterologous peptide tag may encompass a purification tag such as a His-tag as used in the examples or a FLAG-tag, myc-tag, GST-tag, MBP-tag, HA-tag or v5-tag. Such tags may be used for detection and/or purification of a peptide by methods skilled in the art. Such tags may be present N-terminally and/or C-terminally, preferably C-terminally.

The one or more further moieties may also be a labeling moiety. Such labeling moieties may be attached N-terminally and/or C-terminally and/or to an amino acid side chain. Such labeling moiety substance is capable of producing a signal via direct or indirect detection. The labeling moiety thus may be detected directly or indirectly. For direct detection, a labeling moiety can be selected from any known detectable marker group, like chromogens, fluorescent groups, chemiluminescent groups (e.g. acridinium esters or dioxetanes), electrochemiluminescent compounds, catalysts, enzymes, enzymatic substrates, dyes, fluorescent dyes (e.g. fluorescein, coumarin, rhodamine, oxazine, resorufin, cyanine and derivatives thereof), colloidal metallic and nonmetallic particles, and organic polymer latex particles. Other examples of labeling moieties are luminescent metal complexes, such as ruthenium or europium complexes, e.g. as used for ECLIA, enzymes, e.g. as used for ELISA, and radioisotopes; e.g. as used for RIA.

Indirect detection systems comprise, for example, that the peptide is labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is labeled to allow for direct detection, e.g. by the labeling moiety as mentioned above.

A particularly preferred labeling moiety is a fluorescent moiety e.g. fluorescein, coumarin, Cy3, Cy5, rhodamine, oxazine, resorufin, cyanine and derivatives thereof.

Depending on the chemical nature of the labeling moiety, the attachment is preferably performed N-terminally, C-terminally or to an amino acid side chain.

In another preferred embodiment, the one or more a separation moiety, such as a biotin moiety may be present. A separation moiety is preferably understood as moiety allowing specific separation of the peptide from a solution comprising such peptide. For example, the peptide may be labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is attached to a solid support, such as an array, chip, well plate or bead, such as a magnetic bead, thereby allowing separation.

In case more than one moieties are present, such as 2, 3, 4, 5, 6, 7, 8, 9, 10 or more moieties, the moieties of the molecule may be of the same moieties, e.g. the mutant peptide may contain 2, 3, 4, 5, 6, 7, 8, 9, 10 or more labeling moieties, such as fluorescent moieties, or 2, 3, 4, 5, 6, 7, 8, 9, 10 or more separation moieties, such as biotin moieties, or the mutant peptide may contain different moieties, such as one or more labeling moieties and one or more separation moieties.

In one preferred embodiment of the present disclosure a mutant peptide described herein contains one or more further moieties. In a more preferred embodiment, the one or more further moieties are selected from a heterologous sequence, in particular comprising or consisting of a heterologous peptide tag, such as a purification tag, a labeling moiety, such as a fluorescent moiety, and a separation moiety, such as a biotin moiety.

In another preferred embodiment 1, 2, 3, 4, or 5 of the N-terminal amino acids of the wildtype ectodomain of the E protein are deleted in a mutant peptide described herein.

In another embodiment, the present disclosure relates to a composition or a kit-of-parts comprising at least two of the mutant peptides selected from:
(a) the T76Y1, Q77Y2, W101Y3, L107Y4 mutant of the peptide representing the wildtype ectodomain of the E protein of a dengue virus (DENV) of serotype 1, or a biologically active variant thereof,
(b) the T76Y1, Q77Y2, W101Y3, L107Y4 mutant of the peptide representing the wildtype ectodomain of the E protein of a dengue virus (DENV) of serotype 2, or a biologically active variant thereof,
(c) the T76Y1, Q77Y2, W101Y3, L107Y4 mutant of the peptide representing the wildtype ectodomain of the E protein of a dengue virus (DENV) of serotype 3, or a biologically active variant thereof,
(d) the T76Y1, Q77Y2, W101Y3, L107Y4 mutant of the peptide representing the wildtype ectodomain of the E protein of a dengue virus (DENV) of serotype 4, or a biologically active variant thereof, and
(e) the T76Y1, Q77Y2, W101Y3, L107Y4 mutant of the peptide representing the wildtype ectodomain of the E protein of a dengue virus (DENV) of serotype 5, or a biologically active variant thereof,
wherein Y1 is independently a naturally occurring amino acid except T, in particular Y1 is independently selected from A, G and R, and
wherein Y2 is independently a naturally occurring amino acid except Q, in particular Y2 is independently selected from A, G, S and E, and
wherein Y3 is independently a naturally occurring amino acid except W, and
wherein Y4 is independently a naturally occurring amino acid except L.

In a more preferred embodiment, Y3 is R and/or Y4 is R, even more preferably Y3 and Y4 are both R.

In another preferred embodiment, Y1 is A or R.

In another preferred embodiment, Y2 is E or G.

In one preferred embodiment Y1 is A, Y2 is G, and Y3 and Y4 are both R.

In a further preferred embodiment Y1 is A, Y2 is E, and Y3 and Y4 are both R.

In a particularly preferred embodiment Y1 is R, Y2 is E, and Y3 and Y4 are both R.

Such mutant peptides were successfully used in the compositions prepared and employed in the Examples.

In a further preferred embodiment Y1 is R, Y2 is G, and Y3 and Y4 are both R.

In a more preferred embodiment, the wildtype ectodomain of the E protein of a dengue virus (DENV) of serotype 1 comprises the sequence according to SEQ ID No: 5 and/or the wildtype ectodomain of the E protein of a dengue virus (DENV) of serotype 2 comprises the sequence according to SEQ ID No: 6, and/or the wildtype ectodomain of the E protein of a dengue virus (DENV) of serotype 3 comprises the sequence according to SEQ ID No: 7 and/or the wildtype ectodomain of the E protein of a dengue virus (DENV) of serotype 4 comprises the sequence according to SEQ ID No: 8.

"Biologically active variants" of peptides are preferably understood as peptides which exhibit at least 70%, at least 80%, at least 90%, at least 91%, 92%, 93%, 94%, 95%, 96%, 97%, 98%, or 99% sequence identity to a mutant peptide described herein. For example, it is known that amino acids at the N- or C-terminus of a peptide may be missing without affecting correct folding of the domain. As shown in the Examples, it is possible to also employ mutant DENV peptides which are mutated forms of a fragment of a peptide representing the wildtype ectodomain of the E protein of a dengue virus, wherein the fragment of a peptide representing the wildtype ectodomain of the E protein of a dengue virus consists of amino acids 1 to 397 of the peptide representing the wildtype ectodomain of the E protein of a dengue virus, namely DENV-3 in the Examples, and wherein the fragment of a peptide representing the wildtype ectodomain of the E protein of a dengue virus consists of amino acids 1 to 399 of the peptide representing the wildtype ectodomain of the E protein of a dengue virus, namely DENV-1, DENV-2 and DENV-4 in the Examples. As further shown in the Examples, such mutant peptides also fold correctly. Also, the mutant peptides may contain 1 to 40 further mutations at positions within the ectodomain, preferably at positions other that 76, 77, 101 and 107, such as 1, 2, 3, 4, 5, 6, 7, 8, 9, 10, 15, 20, 25, 30, 35 or 40 further mutations. The further mutations may be deletions, insertions or substitutions, in particular substitutions. Preferably, the mutations, in particular substitutions are not within the fusion loop domain of the E protein.

Sequence identity can preferably be determined using the sequence comparison program NCBI-BLAST2 (Altschul et al., Nucleic Acids Res. 25:3389-3402 (1997)). NCBI-BLAST2 uses several search parameters, wherein all of those search parameters are set to default values including, for example, unmask = yes, strand = all, expected occurrences = 10, minimum low complexity length = 15/5, mufti-pass e-value = 0.01, constant for mufti-pass = 25, dropoff for final gapped alignment = 25 and scoring matrix = BLOSUM62.

In a further more preferred embodiment, a biologically active variant of a mutant peptide is a peptide wherein
- 1, 2, 3, 4, or 5 of the N-terminal amino acids of the wildtype ectodomain of the E protein are deleted, and/or
- 1, 2, 3, 4, 5, 6, 7 or 8 of the C-terminal amino acids of the wildtype ectodomain of the E protein are deleted, and/or
- the mutant peptide(s) further contain(s) one or more further moieties, preferably selected from 1, 2, 3, 4 or 5 of amino acids of a homologous protein sequence, a heterologous sequence, in particular comprising or consisting of a heterologous peptide tag, such as a purification tag, a labeling moiety, such as a fluorescent moiety, and a separation moiety, such as a biotin moiety, and/or
- the mutant peptide(s) contain(s) 1 to 40 further mutations at positions within the ectodomain.

The mutant peptides may contains one or more further moieties. The one or more further moieties may be bound covalently or non-covalently, preferably covalently. The one or more further moieties may be added to the N-terminal amino acid, to the C-terminal amino acid and/or to one or more internal amino acid(s).

In one preferred embodiment, the one or more further moieties is one more heterologous sequence(s). Such heterologous sequence is a sequence which is not present in the respective DENV E protein sequence. For example, the heterologous sequence may be or encompass a protein or domain of a protein, or a peptide. For example, such heterologous sequence is or encompasses a sequences which is a purification tag, a separation moiety and/or labeling moiety.

For example the mutant peptides may contain one or more heterologous peptide tags. Such heterologous peptide tag may have a length of 1 to 500 amino acids, preferably of 1, 2, 3, 4, 5, 6, 7, 8, 9, 10 or more and up to 15, 20, 50, 100, 200 or 500 amino acids. For example, the heterologous peptide tag may encompass a purification tag such as a His-tag as used in the examples or a FLAG-tag, myc-tag, GST-tag, MBP-tag, HA-tag or v5-tag. Such tags may be used for detection and/or purification of a peptide by methods skilled in the art. Such tags may be present N-terminally and/or C-terminally, preferably C-terminally.

The one or more further moieties may also be a labeling moiety. Such labeling moieties may be attached N-terminally and/or C-terminally and/or to an amino acid side chain. Such labeling moiety substance is capable of producing a signal via direct or indirect detection. The labeling moiety thus may be detected directly or indirectly. For direct detection, a labeling moiety can be selected from any known detectable marker group, like chromogens, fluorescent groups, chemiluminescent groups (e.g. acridinium esters or dioxetanes), electrochemiluminescent compounds, catalysts, enzymes, enzymatic substrates, dyes, fluorescent dyes (e.g. fluorescein, coumarin, rhodamine, oxazine, resorufin, cyanine and derivatives thereof), colloidal metallic and nonmetallic particles, and organic polymer latex particles. Other examples of labeling moieties are luminescent metal complexes, such as ruthenium or europium complexes, e.g. as used for ECLIA, enzymes, e.g. as used for ELISA, and radioisotopes; e.g. as used for RIA.

Indirect detection systems comprise, for example, that the peptide labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is labeled to allow for direct detection, e.g. by the labeling moiety as mentioned above.

A particularly preferred labeling moiety is a fluorescent moiety e.g. fluorescein, coumarin, Cy3, Cy5, rhodamine, oxazine, resorufin, cyanine and derivatives thereof.

Depending on the chemical nature of the labeling moiety, the attachment is preferably performed N-terminally, C-terminally or to an amino acid side chain.

In another preferred embodiment, the one or more a separation moiety, such as a biotin moiety may be present. A separation moiety is preferably understood as moiety allowing specific separation of the peptide from a solution comprising such peptide. For example, the peptide may be labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is attached to a solid support, such as an array, chip, well plate or bead, such as a magnetic bead, thereby allowing separation.

In case more than one moieties are present, such as 2, 3, 4, 5, 6, 7, 8, 9, 10 or more moieties, the moieties of the molecule may be of the same moieties, e.g. the mutant peptide may contain 2, 3, 4, 5, 6, 7, 8, 9, 10 or more labeling moieties, such as fluorescent moieties, or 2, 3, 4, 5, 6, 7, 8, 9, 10 or more separation moieties, such as biotin moieties, or the mutant peptide may contain different moieties, such as one or more labeling moieties and one or more separation moieties.

In one preferred embodiment of the present disclosure a mutant peptide described herein contains one or more further moieties. In a more preferred embodiment, the one or more further moieties are selected from a heterologous sequence, in particular comprising or consisting of a heterologous peptide tag, such as a purification tag, a labeling moiety, such as a fluorescent moiety, and a separation moiety, such as a biotin moiety.

In another preferred embodiment 1, 2, 3, 4, or 5 of the N-terminal amino acids of the wildtype ectodomain of the E protein are deleted in a mutant peptide of a composition or kit-of-parts of the invention described herein.

In one preferred embodiment, the one or more further moieties is 1, 2, 3, 4 or 5 of amino acids of a homologous protein sequence. For example, it is possible to use a mutant peptide which is a mutated form of a fragment of a peptide representing the wildtype ectodomain of the E protein of a dengue virus, wherein the fragment of a peptide representing the wildtype ectodomain of the E protein of a dengue virus consists of amino acids 1 to 405, 1 to 406, 1 to 407, 1 to 408, or 1 to 409 of the peptide representing the wildtype ectodomain of the E protein of a dengue virus serotype 1, 2, 3 or 4.

It was surprisingly found that a composition comprising four mutant peptides which are mutated forms of E protein peptides from DENV-1, DENV-2, DENV-3 and DENV-4 are surprisingly suitable for discriminating a Dengue virus infection from other flaviviral infections. Moreover, the results with such compositions are clearly superior to the commercially available test for Dengue virus infections. As DENV-1, DENV-2, DENV-3 and DENV-4 represent the major serotypes, it is preferred that the compositions comprise peptides derived from at least two of the serotypes DENV-1, DENV-2, DENV-3 and DENV-4.

Therefore, in a preferred embodiment, the composition or the kit-of-parts comprises at least two of the mutant peptides selected from (a), (b), (c), and (d).

For example, the composition or the kit-of-part comprises two of the mutant peptides (a) and (b), two of the mutant peptides (a) and (c), two of the mutant peptides (a) and (d), two of the mutant peptides (b) and (c), two of the mutant peptides (b) and (d), or two of the mutant peptides (c) and (d).

In a further preferred embodiment, the composition or the kit-of-part comprises 3 of the mutant peptides of (a), (b), (c), and (d) above, in particular the mutant peptides (a), (b) and (c the mutant peptides (a), (b) and (d), or the mutant peptides (b), (c) and (d).

In a more preferred embodiment, the composition or the kit-of-parts comprises 2, 3, or 4 of the mutant peptides of (a), (b), (c), and (d) above, even more preferably the composition or the kit-of-parts comprises 4 of the mutant peptides of (a), (b), (c), and (d) above.

In a particularly preferred embodiment Y1 is R, Y2 is E, Y3 is R and Y4 is R in the mutant peptides of the composition or kit-of-parts of the invention.

In a further preferred embodiment the mutant peptides of the composition or kit-of-parts are independently the T76R, Q77E, W101R, L107R quadruple mutants of the wildtype ectodomains of the E protein of DENV serotypes or a biologically active variant thereof.

In another preferred embodiment the biologically active variant thereof is independently the T76R, Q77E, W101R, L107R mutant of amino acids 1 to 396, 397, 398, 399, 400, 401, 402 or 403 of the peptide representing of the wildtype ectodomain of the E protein of the dengue virus (DENV) serotypes. Such biologically active variants were successfully used in the Examples.

The mutant peptides of the compositions and kits-of-parts optionally independently contain one or more further moieties, preferably selected from a heterologous sequence, in particular comprising or consisting of a heterologous peptide tag, such as a purification tag, a labeling moiety, such as a fluorescent moiety, and a separation moiety, such as a biotin moiety.

It was surprisingly found in the examples that a mixture comprising T76R, Q77E, W101R, L107R mutant peptides as described herein derived from DENV-1, DENV-2, DENV-3 and DENV-4 were surprisingly useful for discriminating Dengue infections from a variety of other flaviviral infections, and is clearly superior to the commercially available diagnostic test. Therefore, compositions comprising at least two mutant peptides from different Dengue virus serotypes are particularly useful for diagnostic purposes. Alternatively, the at least two mutant peptides may be comprised spatially separately in a kit or kit-of parts. The comprising at least two mutant peptides from different Dengue virus serotypes may be mixed prior to diagnostic use or may be tested separately for binding to antibodies in a sample.

In one preferred embodiment the composition or kit-of-parts comprises or consists of:
(a) the T76Y1, Q77Y2, W101Y3, L107Y4 mutant of the peptide representing the wildtype ectodomain of the E protein of a dengue virus (DENV) of serotype 1 or amino acids 1 to 396, 397, 398, 399, 400, 401, 402 or 403 thereof,
   more preferably wherein the wildtype ectodomain of the E protein of a dengue virus (DENV) of serotype 1 consists of amino acids 1 to 404 of SEQ ID No: 1, and
(b) the T76Y1, Q77Y2, W101Y3, L107Y4 mutant of the peptide representing the wildtype ectodomain of the E protein of a dengue virus (DENV) of serotype 2 or amino acids 1 to 396, 397, 398, 399, 400, 401, 402 or 403 thereof,
   more preferably wherein the wildtype ectodomain of the E protein of a dengue virus (DENV) of serotype 2 consists of amino acids 1 to 404 of SEQ ID No: 2, and
(c) the T76Y1, Q77Y2, W101Y3, L107Y4 mutant of the peptide representing the wildtype ectodomain of the E protein of a dengue virus (DENV) of serotype 3 or amino acids 1 to 396, 397, 398, 399, 400, 401, 402 or 403 thereof,
   more preferably wherein the wildtype ectodomain of the E protein of a dengue virus (DENV) of serotype 3 consists of amino acids 1 to 404 of SEQ ID No: 3, and
(d) the T76Y1, Q77Y2, W101Y3, L107Y4 mutant of the peptide representing the wildtype ectodomain of the E protein of a dengue virus (DENV) of serotype 4, more preferably wherein the wildtype ectodomain of the E protein of a dengue virus (DENV) of serotype 4 consists of amino acids 1-404 of SEQ ID No: 4,
wherein optionally the mutant peptides independently contain one or more further moieties, preferably selected from a heterologous sequence, in particular comprising or consisting of a heterologous peptide tag, such as a purification tag, a labeling moiety, such as a fluorescent moiety, and a separation moiety, such as a biotin moiety.

In a more preferred embodiment the composition or kit-of-parts comprises:
(a) the T76Y1, Q77Y2, W101Y3, L107Y4 quadruple mutant of the peptide representing the wildtype ectodomain of the E protein of a dengue virus (DENV) of serotype 1 or amino acids 1 to 396, 397, 398, 399, 400, 401, 402 or 403 thereof, more preferably wherein the wildtype ectodomain of the E protein of a dengue virus (DENV) of serotype 1 consists of amino acids 1 to 404 of SEQ ID No: 1,
   more preferably the T76R, Q77E, W101R, L107R quadruple mutant of amino acids 1 to 397, 398 or 399 of the peptide representing of the wildtype ectodomain of the E protein of a dengue virus (DENV) of serotype 1, and
(b) the T76Y1, Q77Y2, W101Y3, L107Y4 quadruple mutant of the peptide representing the wildtype ectodomain of the E protein of a dengue virus (DENV) of serotype 2 or amino acids 1 to 396, 397, 398, 399, 400, 401, 402 or 403 thereof, more preferably wherein the wildtype ectodomain of the E protein of a dengue virus (DENV) of serotype 2 consists of amino acids 1 to 404 of SEQ ID No: 2,
   more preferably the T76R, Q77E, W101R, L107R quadruple mutant of amino acids 1 to 397, 398 or 399 of the peptide representing of the wildtype ectodomain of the E protein of a dengue virus (DENV) of serotype 2, and
(c) the T76Y1, Q77Y2, W101Y3, L107Y4 quadruple mutant of the peptide representing the wildtype ectodomain of the E protein of a dengue virus (DENV) of serotype 3 or amino acids 1 to 396, 397, 398, 399, 400, 401, 402 or 403 thereof, more preferably wherein the wildtype ectodomain of the E protein of a dengue virus (DENV) of serotype 3 consists of amino acids 1 to 404 of SEQ ID No: 3,
   more preferably the T76R, Q77E, W101R, L107R quadruple mutant of amino acids 1 to 397, 398 or 399 of the peptide representing of the wildtype ectodomain of the E protein of a dengue virus (DENV) of serotype 3, and
(d) the T76Y1, Q77Y2, W101Y3, L107Y4 quadruple mutant of the peptide representing the wildtype ectodomain of the E protein of a dengue virus (DENV) of serotype 4, more preferably wherein the wildtype ectodomain of the E protein of a dengue virus (DENV) of serotype 4 consists of amino acids 1 to 404 of SEQ ID No: 4,
   more preferably the T76R, Q77E, W101R, L107R quadruple mutant of amino acids 1 to 397, 398 or 399 of the peptide representing of the wildtype ectodomain of the E protein of a dengue virus (DENV) of serotype 4,
wherein optionally the mutant peptides independently contain one or more further moieties, preferably selected from a heterologous sequence, in particular comprising or consisting of a heterologous peptide tag, such as a purification tag, a labeling moiety, such as a fluorescent moiety, and a separation moiety, such as a biotin moiety.

In the examples, the mutant peptides each contain a His-Tag at the C-terminus. Such heterologous sequences may therefore be attached, e.g. as a fusion protein, without disturbing the folding of the domain.

In one preferred embodiment of a kit-of-parts or kit the at least two mutant peptides are spatially separate.

In another preferred embodiment of a kit-of-parts or kit the at least two mutant peptides are comprised together in at least one composition.

It was further observed that particularly superior results are achieved if a mixture of mutant peptides derived from DENV-1, DENV-2, DENV-3 and DENV-4 are used, wherein the relative amount of DENV-4 is 5-fold less than the amounts of mutant peptides derived from DENV-1, DENV-2 and DENV-3, respectively.

Accordingly, in yet another preferred embodiment, the composition or the kit-of-parts in particular the composition comprises mutant peptides of (a), (b), (c), and (d) above.

More preferably, the mutant peptides are present in the composition or in the kit-of-parts, in particular in the composition, in a weight ratio of (a):(b):(c):(d) of 5 to 10 : 5 to 10 : 5 to 10 : 1 to 5, more preferably the weight ratio is 5 to 7,5 : 5 to 7,5 : 5 to 7,5 : 1 to 1,5, even more preferably the ratio is about 5 : about 5 : about 5 : about 1.
"About" is understood to refer to the indicated value +/- 10% of the indicated value.

In a preferred embodiment, the composition is a gel, in particular hydrogel, or a liquid, more preferably a solution, suspension or emulsion, or solid, such as a dry, dried, freeze-dried or frozen composition.

In one preferred embodiment, the kits or kits-of-parts comprise a gel, in particular hydrogel, or a liquid, more preferably a solution, suspension or emulsion, or solid, such as a dry, dried, freeze-dried or frozen composition, comprising at least one of the mutant peptides (a) to (e).

In a more preferred embodiment, the solution is an aqueous solution, in particular buffered aqueous solution, such as a saline solution or buffered saline solution.

In one preferred embodiment, the pH of a liquid composition is between 6.0 and 8.0, more preferably 6.5 and 7.8. In another preferred embodiment, the pH of a liquid composition is between 8.0 and 10.0, more preferably 9.0 and 9.8.

In one preferred embodiment, the pH of a liquid composition comprising at least one of the mutant peptides (a) to (e) comprised in a kit or kit-of-parts is between 6.0 and 8.0, more preferably 6.5 and 7.8. In another preferred embodiment, the pH of a liquid composition is between 8.0 and 10.0, more preferably 9.0 and 9.8.

In a more preferred embodiment, the composition or the mutant peptide of the kits or kits-of-parts further comprises auxiliary excipients, such as protease inhibitors, buffering compounds, such as phosphate, Tris or HEPES, and stabilizers, in particular wherein the composition is an aqueous liquid, such as an aqueous solution or wherein the kit or kits-of-parts comprises an aqueous liquid.

In another preferred embodiment, the composition is solid, for example, the composition is frozen, or the mutant peptide is in dry form, for example it may be dry, dried or freeze-dried.

In a further preferred embodiment, the mutant peptide of the kits or kits-of-parts or the composition is in a container, such as a vessel, tube, capillary or syringe. For example, the mutant peptide of the kits or kits-of-parts or the composition may be in a unit dose which allows diagnostic use in a method or use of the present disclosure.

The amount of mutant peptide, and optionally the wt peptide, suitable for performing a method of the disclosure depends on the peptide, assay format, detected antibody isotype, solid support if present, and the read-out.

Typically, the total amount of mutant peptides in a non-capture ELISA format performed in a well plate is the range of 10 ng to 1000 ng, more preferably 50 to 500 ng, even more preferably 50 to 300 ng per well.

Typically, the total amount of mutant peptides in a capture assay format performed in a well plate is the range of 10 ng to 1000 ng, more preferably 50 to 500 ng, even more preferably 50 to 400 ng per well.

In a further embodiment, the present disclosure relates to a kit or kit-of-parts comprising, preferably containing:
(a) at least one mutant peptide and/or at least 2, 3 or 4 of the mutant peptides as as described herein and/or a composition as described herein and optionally
(b) at least one peptide representing the ectodomain of the wildtype E protein of the same dengue virus serotype(s), wherein optionally
   - 1, 2, 3, 4, or 5 of the N-terminal amino acids of the ectodomain sequence are deleted, and/or
   - 1, 2, 3, 4, 5, 6, 7 or 8 of the C-terminal amino acids of the ectodomain sequence are deleted, and/or
   - the wildtype peptide further contains one or more further moieties, preferably 1, 2, 3, 4 or 5 of amino acids of the homologous protein sequence, a heterologous sequence, in particular comprising or consisting of a heterologous peptide tag, such as a purification tag, a labeling moiety, such as a fluorescent moiety, and/or a separation moiety, such as a biotin moiety, and/or
   - the peptide contains 1 to 40 mutations at positions within the ectodomain at positions different than 76, 77, 101 and 107.

In a more preferred embodiment, the mutant peptides, and optionally wildtype peptides, are bound to one or more solid support(s), preferably wherein the solid support is selected from a plate, a well plate, an array, such as a microarray or nanoarray, or a bead, such as a magnetic bead.

In yet another embodiment, the present disclosure relates to the use of a kit or kit-of-parts of the present invention, or a mutant peptide of the present disclosure or a composition or kit-of-parts of the present invention, for discriminating infections with a Dengue virus from infections with other flavivirus(es).

Preferably, the present disclosure relates to the use of a kit or kit-of-parts of the present invention, or a mutant peptide of the present disclosure or a composition or kit-of-parts of the present invention, for discriminating infections with a Dengue virus from from infections with other clinically relevant flavivirus infection(s).

In yet a more preferred embodiment, a DENV infection can be discriminated from infections with TBEV and/or WNV and/or YFV.

In a preferred embodiment, the use of the invention is an in vitro use; i.e. the kits or kit-of-parts of the present invention, or the mutant peptide of the present disclosure, or the composition or kit-of-parts of the present invention are used in vitro.

In another embodiment, the present invention relates to a method for discriminating infections with a Dengue virus from infections with other flavivirus(es), preferably with other clinically relevant flavivirus infection(s), comprising the steps:
(1) contacting a composition or kit-of-parts according to any of claims 1 to 7, with an animal sample containing or suspected to contain antibodies binding to the specific flavivirus,
(2) determining the amount of mutant peptide-antibody complex(es) formed in step (1),
wherein the detection of mutant peptide-antibody complex(es) is indicative of a Dengue virus infection.

In one preferred embodiment, the amount of mutant peptide-antibody complex(es) is indicative per se for the presence of a Dengue virus infection.

In one preferred embodiment, the amount of mutant peptide-antibody complex(es) is compared to a control. Such control may be a cut-off value, or a control value for a negative control, such as a value for one or more animals which are not infected with a flavivirus, and/or or a value for one or more animals which are infected with a flavivirus different from Dengue, such as YFV, TBEV, JEV or WNV. Accordingly, a value which is elevated as compared to such control is indicative for the presence of a Dengue virus infection.

Further, the control may be a positive control, such as a cut-off value for a Dengue virus infection, or a positive control such as a value for one or more animals which are infected with a dengue virus. Accordingly, a value which is above a certain cut-off value or similar to a value as compared to such positive control is indicative for the presence of a Dengue virus infection.

Further, it is possible that the method further employs the corresponding wildtype peptides.

In such preferred embodiment, the method of the disclosure comprises the following further steps:
(1a) contacting
   (i) at least one corresponding wildtype peptide of the mutant peptides of step (1),
      wherein optionally
      - 1, 2, 3, 4, or 5 of the N-terminal amino acids of the ectodomain sequence are deleted, and/or
      - 1, 2, 3, 4, or 5 of the C-terminal amino acids of the ectodomain sequence are deleted, and/or
      - the wildtype peptide further contains one or more further moieties, preferably 1, 2, 3, 4 or 5 of amino acids of the homologous protein sequence, a heterologous peptide tag, such as a purification tag, a labeling moiety, such as a fluorescent moiety, and/or a separation moiety, such as a biotin moiety, and/or
      - the peptide contains 1 to 40 mutations within the ectodomain at positions different than 76, 77, 101 and 107,
      and/or
   (ii) at least one peptide representing the ectodomain of the wildtype E protein of the same dengue virus serotype(s) as the mutant peptides of step (1), wherein optionally
      - 1, 2, 3, 4, or 5 of the N-terminal amino acids of the ectodomain sequence are deleted, and/or
      - 1, 2, 3, 4, 5, 6, 7 or 8 of the C-terminal amino acids of the ectodomain sequence are deleted, and/or
      - the wildtype peptide further contains one or more further moieties, preferably 1, 2, 3, 4 or 5 of amino acids of the homologous protein sequence, a heterologous sequence, in particular comprising or consisting of a heterologous peptide tag, such as a purification tag, a labeling moiety, such as a fluorescent moiety, and/or a separation moiety, such as a biotin moiety, and/or
      - the peptide contains 1 to 40 mutations at positions within the ectodomain at positions different than 76, 77, 101 and 107 with an animal sample containing or suspected to contain antibodies binding to the specific flavivirus,
(3a) determining the amount of wildtype peptide-antibody complex formed in step (1a),
(4) determining the ratio (a)/(b) between the amounts measured in step (3a) for (a) the wildtype peptide of step (1a), and (b) the mutant peptide of step (1),
wherein an elevated ratio (a)/(b), in particular a ratio above about 1.5, above about 2, above about 2,5, above about 3, above about 4, above about 4,5 above about 5, above about 5,5 or above about 6, indicates that the animal is not infected with a Dengue virus, or
wherein a low ratio (a)/(b), in particular a ratio of about 1,5 or lower, of about 1,3 or lower, or of about 1,2 or lower indicates that the animal is infected with a Dengue virus.

However, as indicated in the Examples, the discrimination of a dengue virus from other flaviviral infection with a Equad mixture of mutant peptides representing a composition of the invention was surprisingly sufficient to allow for discrimination without comparing to the corresponding wildtype peptides.

The mutant peptides are contacted with antibodies binding to the specific flavivirus from an animal sample, in particular mammalian sample, such as a human sample. This is typically performed by adding either a solution containing a peptide to the animal sample or a solid support, to which antibodies from the animal sample are bound, e.g. by pumping, pipetting or capillary forces, or, by adding an animal sample to the mutant peptides, depending on the assay format.

In case of a non-capture ELISA assay, the mutant peptides are preferably bound to a solid support, and an optionally diluted animal sample, such as a diluted serum or blood sample, is added thereto e.g. by pumping, pipetting or capillary forces. In a preferred embodiment of the embodiment, the mutant peptides of a composition of the invention are bound to a solid support as a mixture.

Accordingly, in such preferred embodiment, the mutant peptides of a composition of the disclosure are bound to a solid support such, that they are not spatially separated. For example, a mixture of peptides derived from 2, 3 or 4 serotypes may be added to a well of a plate or a spot of an array. Such embodiment was successfully performed in the Examples. Alternatively, the mutant peptides of the disclosure or the mutant peptides of a kit-of-part of the disclosure are bound to a solid support spatially separated from each other. In such embodiment, each well of a plate or a spot of an array only contains one mutant peptide or mutant peptide of a kit-of-part or composition of the disclosure.

Typically, one or more washing steps, e.g. with an aqueous buffered solution, are performed after each step to allow for removal of unbound or unspecifically bound entities.

The contacting is preferably performed under conditions conducive to the formation of antibody-peptide complexes.

Preferably, the mutant peptides and antibodies are incubated for at least 10 min, at least 30 min, at least 1 or at least 1.5 hours.

Preferably, the pH in the contacting solution is between 5.5 to 8.5, more preferably between 6.5 and 7.8.

Preferably, the method is performed at a temperature between 15°C and 40°, more preferably between 20°C and 38°C, even more preferably at about 25°C or room temperature.

According to the invention, the amount of mutant peptide-antibody complex formed in step (1) is determined. Various assays known in the art are suitable therefore, such as an enzyme-linked immunoassay (ELISA), more preferably capture ELISA, an (electro-) chemiluminescence immunoassay (ECLIA), and a radioimmunoassay (RIA). Further preferred assays are sandwich fluorescence immunoassay (FIA), and Microparticle capture enzyme immunoassay (MEIA). Also, the assay may be in the form of test strips.

In a particularly preferred embodiment, the method employs an ELISA or capture ELISA assay. Capture ELISA is preferably also known as sandwich ELISA.

In a preferred embodiment of a method of the invention, determining the amount of mutant peptide-antibody complex formed in step (1) is performed by an ELISA assay.

In a yet more preferred embodiment of a method, in particular an ELISA method, of the invention, the at least one mutant peptide or the at least two mutant peptides of the composition or kit-of-parts are bound to a solid support.

In another yet more preferred embodiment of a method of the invention, the amounts of the mutant peptide-antibody complex(es) are determined using an optionally labeled secondary antibody.

In another preferred embodiment of a method of the invention, determining the amount of mutant peptide-antibody complex formed in step (1) is performed by a capture assay.

In a yet more preferred embodiment, the method, preferably the capture assay of the invention is characterized in that following steps are performed:
- providing capture antibodies bound to a solid support,
- contacting the animal sample with the capture antibodies bound to a solid support, whereby antibody-antibody complexes bound to a solid support are formed,
- contacting the at least one mutant peptide or the at least two mutant peptides of the composition or kit-of-parts with the antibody-antibody complexes bound to the solid support, and
- determining the amounts of mutant peptide-antibody complex(es) by an optionally labeled secondary antibody.

In a particularly preferred embodiment of a method of the invention, the optionally labeled secondary antibody specifically recognizes an epitope on the mutant peptide(s), which is at least 5, 10, or 15 amino acids distant from the fusion loop domain, in particular wherein the fusion loop domain corresponds to amino acids 63 to 120 of the E protein of a dengue virus.

In another preferred embodiment of a capture assay method of the invention, the capture antibodies are anti-mammalian IgM antibodies and/or anti-mammalian IgG antibodies, and/or anti-mammalian IgA antibodies and/or anti-avian IgY antibodies. In another preferred embodiment of a method of the invention, the optionally labeled secondary antibodies are anti-mammalian IgM antibodies and/or anti-mammalian IgG antibodies, and/or anti-mammalian IgA antibodies and/or anti-avian IgY antibodies, wherein the antibodies are optionally labeled, as described herein.

According to the present invention, in particular the methods, uses, kits and kits-of-parts, of the present invention, an animal is preferably selected from the group consisting of a mammal and a bird, more preferably the animal is a mammal.

According to the present invention, the mammal is preferably selected from a human, monkey, horse, donkey, cow, pig, dog and cat, more preferably human and horse, most preferably human.

The samples used investigated and/or used in the methods and kits of the invention are from animals, in preferably mammals, such as human or horses.

In a further preferred embodiment, the sample investigated and/or used in the methods, uses, kits and kits-of-parts of the present invention is selected from serum, blood, sputum, saliva and CSF.

In a preferred embodiment, serum, blood, sputum, saliva or CSF samples from mammals, such as humans or horses may be used according to the invention.

In yet another preferred embodiment of a method of the invention, the animal, in particular mammal or human, suffers from an acute infection or a chronic infection or has suffered from an infection in the past.

In yet another preferred embodiment of a method of the invention, the animal sample is fluid or solid bodily sample, in particular wherein the fluid bodily sample is selected from blood, whole blood, serum, plasma, cerebrospinal fluid, saliva, urine, spinal fluid, synovial fluid, amniotic fluid, tear fluid and cranial fluid and/or wherein the solid bodily sample is a skin, muscle or mucosal sample, such as a biopsy.

In yet another preferred embodiment of a method of the invention, IgA, IgM and/or IgG antibodies, or IgY antibodies are detected.

For example, IgY antibodies of birds may be detected.

Accordingly, in yet another preferred embodiment, IgA antibodies may be detected. Further, IgA and/or IgM and/or IgG antibodies in a sample of a mammal, such as a human, typically be employing suitable secondary antibodies in case of an ELISA.

It is understood that preferred embodiments disclosed in the context of a mutant peptide of the disclosure or composition of the disclosure is understood to apply also to the other subject-matter of the disclosure including categories, such as a kit of the disclosure, a kit-of-parts of the disclosure, a method of the disclosure, a nucleic acid of the disclosure and related subject-matter or a use of the disclosure. Further, an embodiment disclosed for a method is understood to also apply to a use and an embodiment disclosed for a use is understood to also apply to a method.

In another embodiment, the present disclosure relates to a nucleic acid encoding a mutant peptide or peptide of the present disclosure.

In yet another embodiment, the present disclosure relates to a gene expression construct comprising a nucleic acid of the present disclosure.

The nucleic acid encoding a mutated peptide or peptide is preferably present in a gene expression construct, which allows expression of the mutated peptide or peptide of the disclosure. Elements of such constructs depend on the vector and host for expression. Typically, such constructs contain suitable promotor sequences, which are preferably inducible, and terminator sequences.

In yet another embodiment, the present disclosure relates to a vector, preferably a plasmid, comprising a nucleic acid of the present disclosure. The nucleic acid encoding a mutated peptide or a biologically active variant thereof is preferably present in a gene expression construct, which allows expression of the mutated peptide. Elements of such constructs depend on the vector and host for expression. Typically, such constructs contain suitable promotor sequences, which are preferably inducible, and terminator sequences.

Therefore, in a yet further embodiment, the present disclosure relates to a gene expression construct comprising a nucleic acid encoding a mutant peptide of the disclosure or a biologically active variant thereof, which allows expression of a mutant peptide or peptide of the disclosure.

In yet another embodiment, the present disclosure relates to a host cell comprising a vector of the present disclosure, preferably wherein the host cell is selected from:
- a eukaryotic host cell, more preferably an insect cell, yeast cell or mammalian cell, in particular an insect cell, even more preferably a *Drosophila* S2 cell, and
- a bacterial host cell, preferably an *Escherichia coli cell.*

In yet another embodiment, the present disclosure relates to a method for producing a mutant peptide of the present disclosure, comprising cultivating a host cell of the disclosure. In a more preferred embodiment, the host cell is an insect cell, even more preferably a *Drosophila* S2 cell.

It was surprisingly found that the mutant peptides of the disclosure and the compositions of the disclosure can be produced in particular efficiently in adequate amounts in insect cells, in particular in S2 cells.

In yet another embodiment, the present disclosure relates to a composition, preferably a solution, or a kit-of-parts comprising at least one mutant peptide of the disclosure.

In yet another embodiment, the present disclosure relates to a peptide:
(a) which consists of amino acids 1 to 396 of the peptide representing the wildtype ectodomain of the E protein of a dengue virus serotype 2, or
(b) which consists of amino acids 1 to 397 of the peptide representing the wildtype ectodomain of the E protein of a dengue virus serotype 2, or
(c) which consists of amino acids 1 to 398 of the peptide representing the wildtype ectodomain of the E protein of a dengue virus serotype 2, or
(d) which consists of amino acids 1 to 399 of the peptide representing the wildtype ectodomain of the E protein of a dengue virus serotype 2, or
(e) which consists of amino acids 1 to 400 of the peptide representing the wildtype ectodomain of the E protein of a dengue virus serotype 2, or
(f) which consists of amino acids 1 to 401 of the peptide representing the wildtype ectodomain of the E protein of a dengue virus serotype 2, or
(g) which consists of amino acids 1 to 402 of the peptide representing the wildtype ectodomain of the E protein of a dengue virus serotype 2, or
(h) which consists of amino acids 1 to 403 of the peptide representing the wildtype ectodomain of the E protein of a dengue virus serotype 2,
wherein the peptide optionally contains one or more further moieties, more preferably selected from a heterologous sequence, in particular comprising or consisting of a heterologous peptide tag, such as a purification tag, a labeling moiety, such as a fluorescent moiety, and/or a separation moiety, such as a biotin moiety.

### Sequences:

### wildtype ectodomain of DENV-1, accession: NP_722460.2

### wildtype ectodomain of DENV-2 Accession, accession NP_056776.2

### wildtype ectodomain of DENV-3 accession, accession YP_001531168.2

### wildtype ectodomain of DENV-4 accession, accession NP_740317.1

Sequence "DENV-1" on Figure 1:
   nttt dsrcptqgea tlveeqdtnf vcrrtfvdrg wgngcglfgk gslitcakfk cv (SEQ ID No: 5)
Sequence "DENV-2" on Figure 1:
   nttt esrcptqgep slneeqdkrf vckhsmvdrg wgngcglfgk ggivtcamfr ck (SEQ ID No: 6)
Sequence "DENV-3" on Figure 1:
   nitt dsrcptqgea vlpeeqdqny vckhtyvdrg wgngcglfgk gslvtcakfqcl (SEQ ID No: 7)
Sequence "DENV-4" on Figure 1:
   nitt atrcptqgep ylkeeqdqqy icrrdvvdrg wgngcglfgk ggvvtcakfs cs (SEQ ID No: 8)
Sequence "WNV" on Figure 1:
Sequence "JEV" on Figure 1:
   distvarcpt tgeahnekra dssyvckqgf tdrgwgngcg lfgkgsidtc akfsct (SEQ ID No: 23)
Sequence "YFV" on Figure 1:
   hvkindkcpstgeahlaee negdnackrt ysdrgwgngc glfgkgsiva cakftca (SEQ ID No: 24)
Sequence "TBEV" on Figure 1:
   dtkv aarcptmgpa tlaeehqggt vckrdqsdrg wgnhcglfgk gsivacvkaace (SEQ ID No: 25)

The mutant peptides in the Examples contain a heterologous sequence, which encompasses a His-tag at the C-terminus of the mutant peptide:
GSGHHHHHH (SEQ ID No: 9)

### mutated form of DENV-1 accession: NP_722460.2; aa 1-399 (mutations in bold)

### mutated form of DENV-2 Accession: NP_056776.2 ; aa 1-399 (mutations in bold)

### mutated form of DENV-3 accession: YP_001531168.2 (mutations in bold)

### mutated form of DENV-4 accession: NP_740317.1 (mutations in bold)

### DENV-1 accession: NP_722460.2

### DENV-2 Accession: NP_056776.2

### DENV-3 accession: YP_001531168.2

### DENV-4 accession: NP_740317.1

Amino acids 1 to 495 of the E protein dengue virus, serotype 1 of GenBank Accession No: AEV66294.1 is:

The E protein of dengue virus, serotype 2 of GenBank Accession No: BAL05267.1 (amino acids 1-495) has the following sequence:

The E protein of dengue virus, serotype 3 of GenBank Accession No: AAD37780.1(amino acids 1-493) has the following sequence:

The E protein of dengue virus, serotype 4 of GenBank Accession No: AEV66313.1 (amino acids 1-495) has the following sequence:

### Figure legend:

- **Figure 1:**: Alignment of the amino acid sequences containing the fusion loop domain from E proteins of DENV 1-4, WNV, JEV, YFV, TBEV and the four point mutations of the QUAD proteins. Amino acids numbering: 1 is start of the E protein.

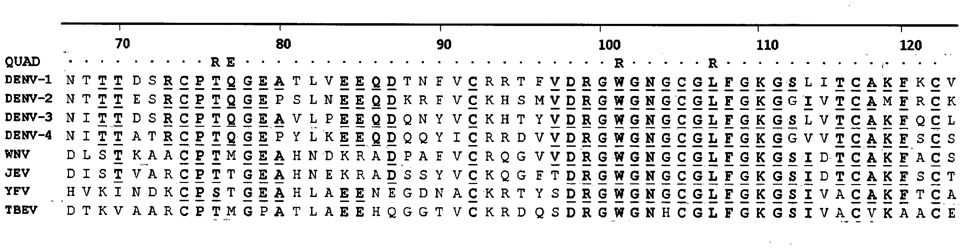

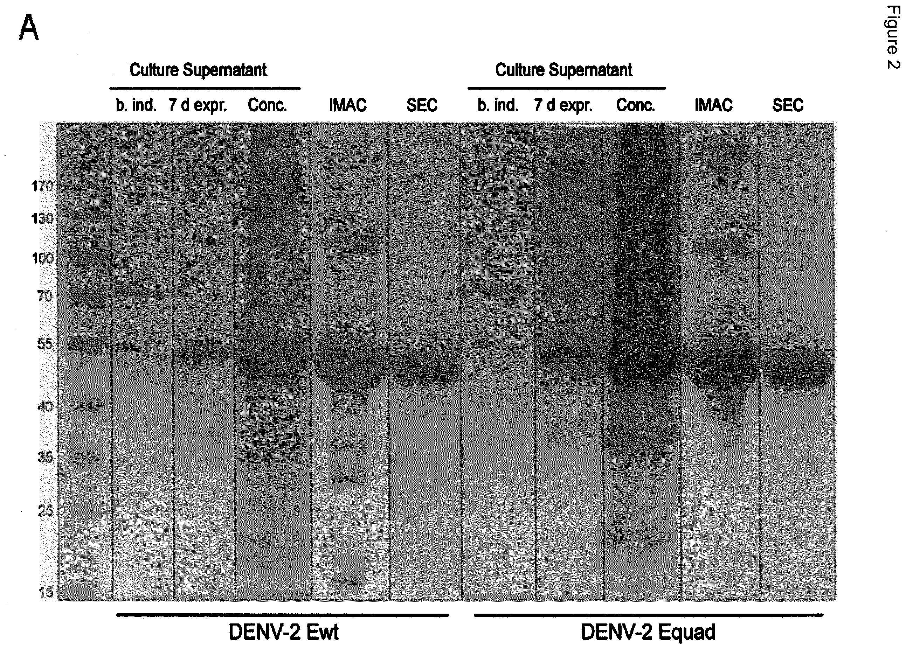

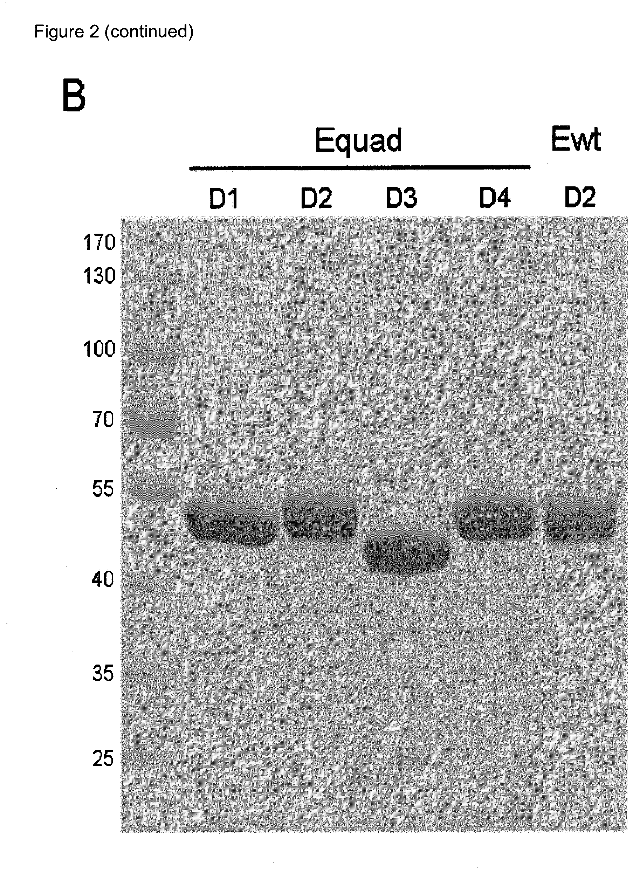

- **Figure 2:**: A: Expression and purification of DENV-2 Ewt and Equad from Drosophila S2 culture supernatants; supernatant before induction (b.ind.), after 7 days of expression culture (7 d expr.), concentrated via tangential flow (Conc.) and the two step purification with immobilized imidazole affinity (IMAC) and size exclusion chromatography (SEC) were separated on a 10 % SDS-PAGE gel under reducing conditions. B: 6 µg of purified DENV1-4 (D1-D4) Equad and DENV-2 Ewt proteins were analyzed with SDS-PAGE. Proteins were stained with Coomassie blue. Size of molecular weight markers in kilo Daltons is indicated on the left.

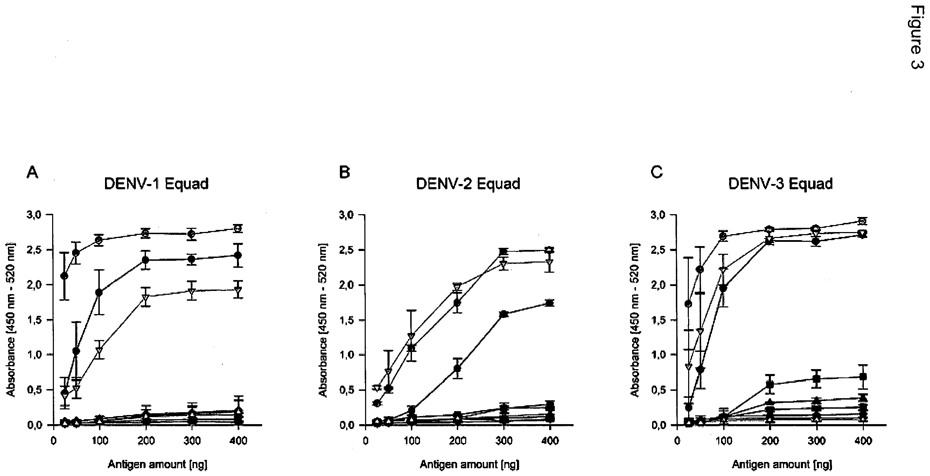

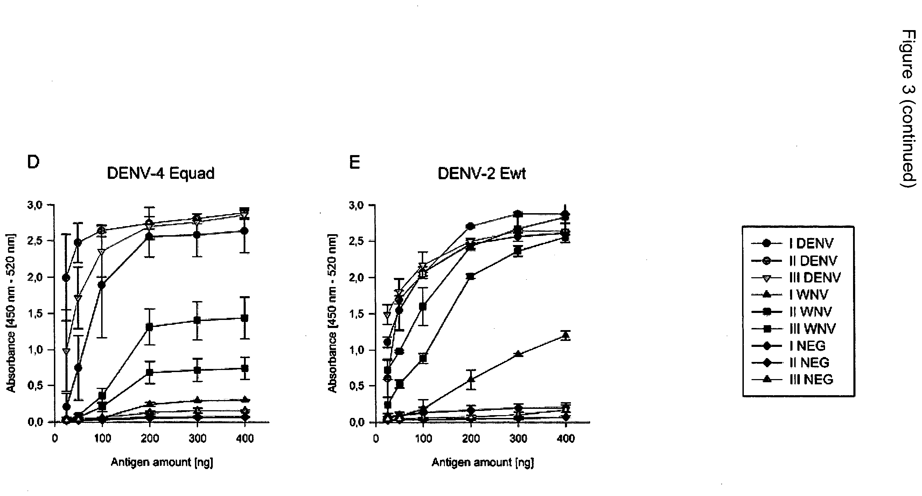

- **Figure 3:**: Antigen titration using the indicated amounts of DENV-1 (A), -2 (B), -3 (C) and -4 (D) Equad and DENV-2 Ewt (E) proteins with three different DENV, WNV and negative (NEG) human sera. Measurements were performed in duplicates in at least two independent experiments.
- **Figure 4:**: 300 ng per well of DENV-2 Ewt (A) and Equad (B) and 160 ng per well of a DENV 1-4 Equad mixture (C) were tested with DENV- WNV- and TBEV- infected and YFV-vaccinated sera compared to negative (NEG) samples in an IgG-ELISA (n=number of individuals). Bottom and top of the boxes are the first and third quartiles. The median signal is depicted as a line inside the box. Whiskers represent the 9^{th} and the 91^{st} percentile. Outliers in B and C are marked with numbers (1-5). Measurements were performed in duplicates in at least two independent experiments.
- **Figure 5:**: Comparison of different antigens for the detection of DENV IgG. Sera positive for IgG against DENV, WNV, TBEV or negative control sera were analyzed with the Panbio Indirect IgG ELISA (black), the DENV-2 Ewt protein (light gray, lined) or the DENV1-4 Equad mix (dark grey, lined). The absolute absorbance is indicated. Cut-Off values for the Panbio test were obtained by calculation of the internal standard of the manufacturer, these are indicated at the right and only refer to this test (horizontal bars: DENV-positive results with an OD-value higher than 1.1^{∗}cut-off, equivocal results having an OD-value between 1.1^{∗}cut-off and 0.9^{∗}cut-off, negative results with an OD-value lower than 0.9*cut-off).

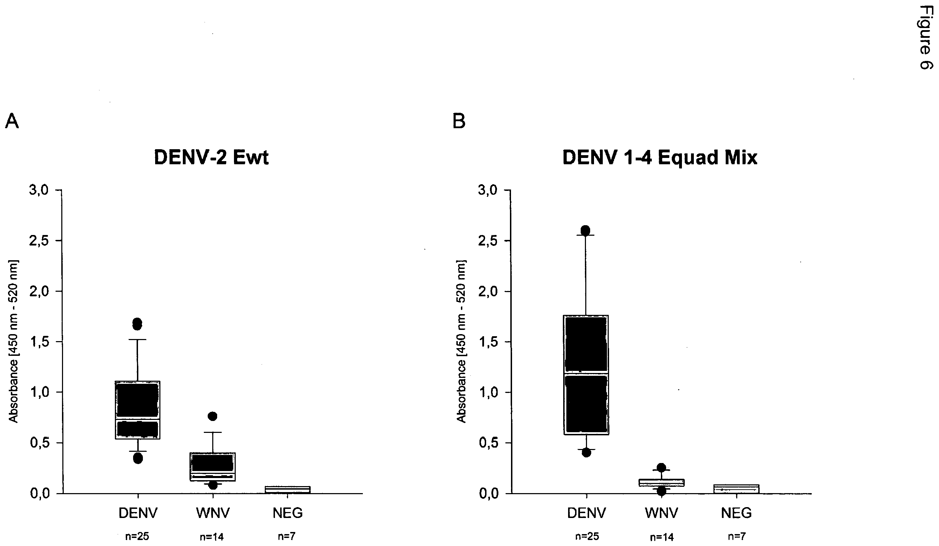

- **Figure 6:**: IgM-ELISA on 300 ng of DENV-2 Ewt (A) and DENV 1-4 Equad mixture (B) with different DENV, WNV and negative (NEG) sera (n= number of individuals). Bottom and top of the boxes are the first and third quartiles. The median signal is depicted as a line inside the box. Whiskers represent the 9^{th} and the 91^{st} percentile. Measurements were performed in duplicates in at least two independent experiments.

### Examples

### Recombinant envelope-proteins with mutations in the conserved fusion loop allow specific serological diagnosis of dengue-infections

Dengue virus (DENV) is a mosquito-borne flavivirus and a major international public health concern in many tropical and sub-tropical areas worldwide. DENV is divided into four major serotypes, and infection with one serotype leads to immunity against the same, but not the other serotypes. The specific diagnosis of DENV-infections via antibody-detection is problematic due to the high degree of cross-reactivity displayed by antibodies against related flaviviruses, such as West Nile virus (WNV), Yellow Fever virus (YFV) or Tick-borne encephalitis virus (TBEV). Especially in areas where several flaviviruses co-circulate or in the context of vaccination e.g. against YFV or TBEV, this severely complicates diagnosis and surveillance. Most flavivirus cross-reactive antibodies are produced against the highly conserved fusion loop (FL) domain in the viral envelope (E) protein.

### Material and Methods

### Cell culture

*Drosophila S2* cells (Invitrogen) were propagated at 28 °C in T-75 cm² flasks in Schneider's medium supplemented with 10 % FCS and 1 % Pen/Strep (complete Schneider's Medium).

### Expression and purification of DENV Envelope proteins

The sequences of the DENV-2 E wild type ectodomain (E-protein amino acid residues 1-399; strain 16681) and the quadruple mutants (Equad: T76R, Q77E, W101R; L107R) of DENV serotypes 1-4 (DENV-1: Nauru/West Pac/1974; E-protein amino acid residues 1-399; DENV-3: Sri Lanka/1266/2000; E-protein amino acid residues 1-397; DENV-4: Dominica/814669/1981 E-protein amino acid residues 1-399;) were synthesized (Centic Biotec) and cloned with BgIII and EcoRI into the pMT/BiP/V5-His vector (Invitrogen). Plasmids were transfected with a Ca-Phosphate transfection kit (Invitrogen) according to manufacturer's instructions into *Drosophila S2* cells. To generate stable cell lines 1 µg of pCoHygro (Invitrogen), containing a hygromycin resistance gene, was co-transfected with each expression vector. Stably transfected polyclonal S2 cell populations were generated after 3 weeks of selection with hygromycin B (300 µg/ml) in complete Schneider's Medium. These cells were then propagated at 28 °C in tissue culture flasks with complete Schneider's medium containing 300 µg/ml hygromycin B and adapted to Sf900II medium containing 600 µg/ml hygromycin B. For an expression culture, cells were seeded at a cell density of 2-3 x10⁶ cells/ml in 600 ml Sf900II medium in 2 l baffled Erlenmeyer shaker flasks at 28 °C and 90 rpm and were induced with 700 µM CuSo4 at a cell density of 6 x 10⁶ cells/ml. After 7 days the suspension culture was centrifuged for 15 min and 4000 g at 4 °C and culture supernatant was concentrated and diafiltrated against His-binding buffer (20 mM sodium phosphate, 500 mM NaCl, 10 mM Imidazole, pH7,4) using Vivaflow 50R TFF cassettes (Sartorius) according to manufacturers' instructions. The DENV E proteins were purified by immobilized metal affinity chromatography (IMAC) with 5 ml HisTrap FF crude columns (GeHealthcare) and size exclusion chromatography with a 16/600 HiLoad Superdex 200 pg column (GeHealthcare) using the ÄKTA pure 25 I chromatography system (GeHealthcare). Purified proteins were quantified using a BCA protein assay (Pierce). Conformation of the E-proteins and loss of the FL-domain structure in the Equad mutants were verified by binding studies of monoclonal antibodies recognizing DENV-2 epitopes in the domains DI-DII (antibody DV2-44), DIII (DV2-76, DV2-96, DV2-106) and FL (DV2-29) and WNV-specific FL-antibodies (E16 and E60) [25,26] (data not shown).

### Serum samples

22 DENV-positive serum samples were obtained from Padova University Hospital (Italy). The confirmed DENV cases were international travelers returning from endemic countries with diagnosis of recent infection15 DENV-positive serum samples and one negative control were obtained from Seracare Life Sciences (USA), and 8 DENV-infected serum samples and two negative controls were obtained from ZeptoMetrix Corporation (USA). Serum samples positive for DENV antibodies all fulfilled one of the following criteria: (a) positive in DENV-specific RT-PCR, (b) positive for both DENV IgM and IgG or (c) positive in a DENV-specific virus neutralization test (VNT). Three DENV-infected and one WNV-infected samples were from the Robert Koch Institute (Berlin) as part of an external quality assessment study [12]. Serum samples from confirmed WNV-infections as well as sera from TBEV-infected individuals and negative controls were derived from Italy (University of Padova). The WNV-positive samples (IgG and IgM) were from seroprevalence studies, blood donors or patients with WNV- neuroinvasive disease. WNV-infections were confirmed by VNT. TBEV IgG-positive serum samples were selected from a seroprevalence study in forest rangers with a history of confirmed TBEV infection and no previous exposure to DENV. Serum samples from YFV-vaccinated individuals (IgG positive) participating in a randomized controlled vaccination study were obtained from the Robert Koch Institute (Berlin, Germany). These were confirmed by VNT.

### Ethics statement

Ethical approval for all serum samples from Padova University was obtained from the Padova University Hospital ethics committee. The randomized controlled vaccination study for YFV from the Robert Koch Institute was approved by the Charité medical ethics committee.

### Antibody measurements

Indicated protein amounts of DENV-2 Ewt and Equad or DENV1-4 Equad mixtures were coated overnight on Nunc polysorb plates (Thermo Scientific) in 100 µl coating buffer (15 mM Na2CO3,35 mMNaHCO3 pH 9.6) at 4 °C. The plates were washed three times with 350 µl per well of PBS-0,05 % Tween and blocked with 200 µl of 5 % non-fat milk powder (blocking solution) for 2 h at room temperature. After a second washing step, human sera were diluted 1:100 in 100 µl blocking solution per well and incubated for 1.5 h at room temperature. Following a third washing step, the HRP-conjugated secondary goat anti human IgG (Fisher Scientific, 1:10000 in 100 µl blocking solution per well) or rabbit anti human µ-chain IgM (Dianova, 1:7500 in 100 µl blocking solution per well) antibody was added for 1 h at room temperature. After a fourth washing step, 100 µl TMB substrate (Biozol) per well were incubated for 30 min at room temperature. The reaction was stopped with 50 µl 1 M H₂SO₄ and signals were read out at 450 nm with background reduction at 520 nm in a micro plate reader (Infinite M200, Tecan). All tests were performed in duplicates and in two independent experiments. The Panbio Dengue IgG Indirect ELISA and was used according to the manufacturer's instructions.

### Statistical analysis

All antibody measurements were performed in duplicates in at least two independent experiments. Graphical and descriptive statistical analysis of the data in boxplots was carried out using SigmaPlot.

### Results

To enhance specificity of serological DENV diagnosis we inserted 4 amino acid point mutations into the conserved fusion loop (FL) domain and an adjacent loop domain of DENV wildtype (wt) E proteins, yielding quadruple mutants for DENV serotypes 1 to 4 (DENV 1-4 Equad-proteins, Figure 1). The mutant proteins as well as the DENV-2 wt E-protein were overexpressed in Drosophila S2 cells and secreted into the culture's supernatant. After purification by immobilized metal affinity chromatography several unspecific proteins were co-purified. Therefore a second purification step using size exclusion chromatography was performed to eliminate the visible unspecific bands (Figure 2A).

To determine the optimal antigen concentration per well for an IgG-ELISA increasing amounts of the proteins DENV-2 Ewt and DENV 1-4 Equad were incubated with three sera of DENV- and WNV- infected or flavivirus-uninfected individuals, respectively (Figure 3). Signal saturation of DENV-positive sera was observed with an antigen amount of approx. 200 ng per well for DENV -1, 3 and -4 Equad and 300 ng per well for DENV-2 Ewt and Equad proteins. Two of the three DENV-positive sera showed a substantial decrease in binding to the DENV-1 and DENV-2 mutant proteins (Figure 3A and B) as compared to the DENV-2 wild type version, but the values were still detectable when 50 ng or more was used. Two WNV-positive samples bound to the wild type antigen as strongly as the DENV-positive sera, and the third started to show such cross-reactivity with more than 200 ng of antigen per well (Figure 3E). However, all WNV sera lost binding almost completely when the mutant antigens were used except for DENV-4 Equad, where two WNV-positive sera still showed substantial signals for 200 ng of antigen per well or more (Figure 3D). Negative sera showed negligible signal intensities for all proteins in all amounts tested. Based on these results an antigen amount of 300 ng per well was chosen for the analysis of a larger serum panel with DENV-2 wt and DENV-2 Equad in an IgG ELISA.

First, sera from 38 DENV-, 13 WNV-, 19 TBEV- infected, 8 YFV vaccinated and 7 uninfected individuals were incubated with DENV-2 Ewt protein (Figure 4A). Strong binding for DENV-positive samples was observed (mean absorbance value 2.36), however, several WNV- and TBEV- positive sera (mean values 1.26 and 0.82, respectively) showed cross-reactive signals which were in the range of DENV-infected sera. Samples from YFV-vaccinated individuals showed a reduced cross-reactivity in comparison to WNV- and TBEV-infected serum samples. When using the DENV-2 Equad protein, the cross-reactivities of WNV- and TBEV-infected samples were significantly reduced (mean values 0.106 and 0.160, respectively) (Figure 4B). In addition, the signal intensities of the DENV-positive samples changed. The mean value was reduced to 1.9 and the overall signal range increased from 2.1 to 2.4 in comparison to DENV-2 Ewt. Next, a mixture of the Equad proteins of all four serotypes was prepared (DENV 1-4 Equad Mix). Based on the titration curves (Figure 3) a total amount of 160 ng was found to be optimal in specificity and sensitivity, consisting of 50 ng of DENV 1-3 Equad respectively and 10 ng of DENV-4 Equad. The proportional amount of DENV-4 Equad was reduced compared to the other serotypes because it elicited a higher cross-reactivity with heterologous flaviviral sera (Figure 3D). The mixture was tested with the same serum panel. This resulted in an increase of the 25^{th} percentile from 1.36 to 1.6, demonstrating a higher sensitivity compared to using only DENV-2 Equad. At the same time, cross-reactivity of WNV- and TBEV-positive sera was even further reduced (mean values 0.063 and 0.084, respectively) showing a higher specificity of the test in comparison to using DENV-2 Equad only. The five DENV-positive sera detected as outliers on the lower end of the DENV-panel using the mutant antigens in Figure 4 were numbered. Whereas sera 1, 2 (both unknown DENV serotype infections) and 3 (DENV-3 infection) showed decreased binding to the DENV-2 Equad-protein as compared to the wild type, their signals increased when using the DENV 1-4 Equad mix. Also the signals of samples 4 and 5 (DENV-1 and -2 infections, respectively) decreased with the DENV-2 Equad compared to the wild type but were even lower with the mutant mixture (Figure 4C).

To compare these results with a state-of-the-art diagnostic method, a number of the serum samples were analyzed with a commercially available DENV-IgG ELISA. The test detected 3/3 DENV-positive sera as positive and 3/3 negative sera as negative. However, 10/10 WNV-infected sera were detected as positive with several OD-values similar to DENV-infected sera. For TBEV-infected sera, 4/10 samples were detected as negative, 3/10 as equivocal and 3/10 as positive (Figure 5). Comparison with the values obtained using DENV Ewt showed a similar cross-reactivity. In contrast, when using the DENV 1-4 Equad mixture high signals were obtained only with the three DENV-infected sera (mean values of 1.5, 1.6 and 1.7, respectively) and cross-reactivity was strongly reduced in all samples from WNV- and TBEV-infections (mean values all >0,3,Figure 5).

Subsequently, the proteins were used to measure IgM antibodies in human sera. Best signal to noise ratios were achieved with 300 ng of DENV 1-4 Equad mix (data not shown), which was then compared to 300 ng of DENV-2 Ewt in binding of IgM-positive DENV and WNV sera. Generally, the cross-reactivity of heterologous flavivirus IgM antibodies was lower than for IgG, as demonstrated by less binding to the Ewt protein (Figure 6A). By using the DENV 1-4 Equad mixture the mean value of signals for DENV sera was enhanced, as compared for DENV-2 Ewt. On the other hand, WNV cross-reactivity was reduced in comparison to DENV-2 Ewt (Figure 6B).

### Discussion

A challenge for current serological dengue diagnosis is the high degree of cross-reactivity between antibodies produced during infections with related flaviviruses [27-29],leading to false positive results in currently available serological test systems [11,12]. Especially in areas with different co-circulating flaviviruses, this is of concern [1,14]. Here, we present insect-cell derived recombinant DENV E proteins bearing mutations in the conserved FL domain to enhance the specificity of serological DENV diagnosis. When using the wild type E protein from serotype 2 (which is the basis for several available test systems) we found all the DENV-positive sera displaying high signals (Figure 4A). However, the cross-reactivity problem was also confirmed, as the signals obtained with many WNV- and TBEV positive samples were in the range of DENV-signals, although the mean intensities were lower (Figures 3E, 4A). As a consequence, several sera would be misdiagnosed as false-positive, which was indeed observed when a commercial assay was used (Figure 5).

It has been shown before that a large proportion of antibodies to DENV-infections are produced against the FL domain [17,18,22]. In accordance, two out of three dengue sera showed a clearly decreased signal when the FL-mutant version of the E-protein from DENV-2 was used (Figure 3B and 3E). However, in the larger serum panel, the signals obtained with the DENV-sera remained detectable (mean absorbance value of 1.9) ((Figure 4B), but most of the binding of the heterologous flavivirus-antibodies was eliminated (Figures 3B, 4B). This result confirms previous studies performed with WNV and JEV, using VLP-based systems with single- or double mutations in the FL domain [20,21] and underlines the role of the conserved FL in cross-reactivity between flavivirus-antibodies. It also demonstrates that the mutations used in the Equad proteins from WNV [24] can also be used to eliminate cross-reactive epitopes in DENV E proteins. When the mixture of all four serotype-specific mutant proteins was used, the 25^{th} percentile of the values increased as compared to using DENV-2 Equad only, although only half the amount of total protein was used (Figure 4C). This increase in sensitivity might result from serum samples that were from non-serotype 2 infections and now bound to their corresponding E-protein, such as the three outliers in Figure 4B. However, in the absence of secure information on the infecting serotype, this interpretation remains speculative. The possibility to perform serotyping analysis with this diagnostic system needs to be evaluated in more detail. Data on the infecting serotypes were present only for a limited subset of the DENV-positive sera in this study. In addition to an increase in sensitivity, the cross-reactivity of TBEV and WNV sera was further reduced with the serotype mixture and lower amount of antigen. Under the conditions used, all DENV-infected sera of the panel led to high absorbance values with the DENV 1-4 mix. Nevertheless, to avoid single outliers (sera 4 and 5 in Figure 4C), the exact relative contribution and amount of each serotype mutant protein in the mixture might still be optimized. The titration curves (Figure 3) suggest that a doubling of the amount of the individual mutant antigens does still not lead to an increase in cross-reactivity.

Using the DENV 1-4 mix for IgM detection resulted in a higher mean value of positive samples as compared to the wildtype antigen (Figure 6). At the same time, the cross-reactivity with WNV-positive sera was decreased. However, due to the generally observed higher specificity of IgM-antibodies [12], this improvement in specificity was less pronounced as for IgG-detection (Figure 6). Nevertheless, our data support the idea that recombinant E proteins bearing four point mutations are suitable for the specific serological dengue-diagnosis. This is also supported by testing samples from a recent external quality assessment on serological dengue diagnosis [12], where the vast majority of participating laboratories (using various ELISA systems) detected a cross-reactive WNV serum as false positive. The serotype mixture used here was able to diagnose the samples 100% correctly (results are included in Figure 4), including diluted samples for sensitivity determination. In addition, the direct comparison of the DENV-1-4 mixture antigen with a commercially available test clearly showed the increased specificity of the mutant antigens (Figure 5). Although no cut-offs were yet defined for the DENV1-4 Equad, it is clear from Figures 4 and 5 that the values obtained with cross-reactive sera are much more similar to negative than to DENV-positive samples.

We recently have performed a study using a bacterially expressed Equad mutant for WNV [24]. These data also showed a reduction in cross-reactivity. However, especially some DENV-positive samples with a high antibody titer still bound the mutant protein to some extent. Therefore, the ratio between signals obtained with the wildtype and mutant protein was calculated for each serum enabling correct diagnosis. In contrast, in the present study the mutant proteins alone were sufficient to exclude cross-reactivity, which might reflect higher sensitivity of the insect-cell derived antigen or specific differences between the antibody responses to WNV and DENV.

In summary, a system for the sensitive and specific serological diagnosis of DENV-infections is presented, which consists of recombinant E-proteins from the four major serotypes, with mutations in the FL and an adjacent loop domain. The binding of cross-reactive antibodies from heterologous flavivirus-infections is strongly decreased. These antigens might form a valuable basis for improved antibody tests, especially in areas where co-circulation of different flaviviruses is observed.

### References

1. Mackenzie JS, Gubler DJ, Petersen LR (2004) Emerging flaviviruses: the spread and resurgence of Japanese encephalitis, West Nile and dengue viruses. Nature medicine 10 (12 Suppl): S98-109. doi:10.1038/nm1144.
2. Bhatt S, Gething PW, Brady OJ, Messina JP, Farlow AW, et al. (2013) The global distribution and burden of dengue. Nature 496 (7446): 504-507. doi:10.1038/nature12060.
3. Guzman MG, Halstead SB, Artsob H, Buchy P, Farrar J, et al. (2010) Dengue: a continuing global threat. Nature reviews. Microbiology 8 (12 Suppl): S7-16. doi:10.1038/nrmicro2460.
4. WHO (2015) Dengue and severe dengue. Available: http://www.who.int/mediacentre/factsheets/fs117/en/.
5. Rothman AL (2011) Immunity to dengue virus: a tale of original antigenic sin and tropical cytokine storms. Nat Rev Immunol 11 (8): 532-543. doi:10.1038/nri3014. Available: http://dx.doi.org/10.1038/nri3014.
6. La Ruche G, Souarès Y, Armengaud A, Peloux-Petiot F, Delaunay P, et al. (2010) First two autochthonous dengue virus infections in metropolitan France, September 2010.. August 2010. Euro Surveill. 15 (39).
7. Gjenero-Margan I, Aleraj B, Krajcar D, Lesnikar V, Klobučar A, et al. (2011) Autochthonous dengue fever in Croatia, August-September 2010. Euro Surveill. 16(9).
8. Lourenço J, Recker M (2014) The 2012 Madeira dengue outbreak: epidemiological determinants and future epidemic potential. PLoS neglected tropical diseases 8 (8): e3083. doi:10.1371/journal.pntd.0003083.
9. Tang KF, Ooi EE (2012) Diagnosis of dengue: an update. Expert review of anti-infective therapy 10 (8): 895-907. doi:10.1586/ERI.12.76.
10. Peeling RW, Artsob H, Pelegrino JL, Buchy P, Cardosa MJ, et al. (2010) Evaluation of diagnostic tests: dengue. Nat Rev Micro 8 (12): S30. doi:10.1038/nrmicro2459.
11. Hunsperger EA, Yoksan S, Buchy P, Nguyen VC, Sekaran SD, et al. (2014) Evaluation of commercially available diagnostic tests for the detection of dengue virus NS1 antigen and anti-dengue virus IgM antibody. PLoS neglected tropical diseases 8 (10): e3171. doi:10.1371/journal.pntd.0003171.
12. Domingo C, Alves MJ, Ory F de, Teichmann A, Schmitz H, et al. (2015) International external quality control assessment for the serological diagnosis of dengue infections. BMC Infect Dis 15 (1): 476. doi:10.1186/s12879-015-0877-0.
13. Papa A, Karabaxoglou D, Kansouzidou A (2011) Acute West Nile virus neuroinvasive infections: Cross-reactivity with dengue virus and tick-borne encephalitis virus. J. Med. Virol. 83 (10): 1861-1865. doi:10.1002/jmv.22180. Available: http://dx.doi.org/10.1002/jmv.22180.
14. Singh KP, Mishra G, Jain P, Pandey N, Nagar R, et al. (2014) Co-positivity of anti-dengue virus and anti-Japanese encephalitis virus IgM in endemic area: co-infection or cross reactivity? Asian Pacific Journal of Tropical Medicine 7 (2): 124-129. doi:10.1016/S1995-7645(14)60007-9.
15. Wahala, Wahala M P B, Silva, Aravinda M de (2011) The human antibody response to dengue virus infection. Viruses 3 (12): 2374-2395. doi:10.3390/v3122374.
16. Crill WD, Chang GJ (2004) Localization and characterization of flavivirus envelope glycoprotein cross-reactive epitopes. Journal of virology 78 (24): 13975-13986. doi:10.1128/JVI.78.24.13975-13986.2004.
17. Crill WD, Hughes HR, Delorey MJ, Chang GJ (2009) Humoral immune responses of dengue fever patients using epitope-specific serotype-2 virus-like particle antigens. PloS one 4 (4): e4991. doi:10.1371/journal.pone.0004991.
18. Lai C, Tsai W, Lin S, Kao C, Hu H, et al. (2008) Antibodies to envelope glycoprotein of dengue virus during the natural course of infection are predominantly cross-reactive and recognize epitopes containing highly conserved residues at the fusion loop of domain II. Journal of virology 82 (13): 6631-6643. doi:10.1128/JVI.00316-08.
19. Seligman SJ (2008) Constancy and diversity in the flavivirus fusion peptide. Virology journal 5: 27. doi:10.1186/1743-422X-5-27.
20. Chiou S, Crill WD, Chen L, Chang GJ (2008) Enzyme-linked immunosorbent assays using novel Japanese encephalitis virus antigen improve the accuracy of clinical diagnosis of flavivirus infections. Clinical and vaccine immunology : CVI 15 (5): 825-835. doi:10.1128/CVI.00004-08.
21. Roberson JA, Crill WD, Chang GJ (2007) Differentiation of West Nile and St. Louis encephalitis virus infections by use of noninfectious virus-like particles with reduced cross-reactivity. Journal of clinical microbiology 45 (10): 3167-3174. doi:10.1128/JCM.01143-07.
22. Vogt MR, Dowd KA, Engle M, Tesh RB, Johnson S, et al. (2011) Poorly neutralizing cross-reactive antibodies against the fusion loop of West Nile virus envelope protein protect in vivo via Fcgamma receptor and complement-dependent effector mechanisms. Journal of virology 85 (22): 11567-11580. doi:10.1128/JVI.05859-11.
23. Cleton NB, Godeke G, Reimerink J, Beersma MF, Doom, H Rogier van, et al. (2015) Spot the difference-development of a syndrome based protein microarray for specific serological detection of multiple flavivirus infections in travelers. PLoS neglected tropical diseases 9 (3): e0003580. doi:10.1371/journal.pntd.0003580.
24. Chabierski S, Barzon L, Papa A, Niedrig M, Bramson JL, et al. (2014) Distinguishing West Nile virus infection using a recombinant envelope protein with mutations in the conserved fusion-loop. BMC infectious diseases 14: 246. doi:10.1186/1471-2334-14-246.
25. Sukupolvi-Petty S, Austin SK, Engle M, Brien JD, Dowd KA, Williams KL et al. (2010) Structure and function analysis of therapeutic monoclonal antibodies against dengue virus type 2. Journal of virology. 84(18):9227-9239. doi: 10.1128/JVI.01087
26. Oliphant T, Nybakken GE, Engle M, Xu Q, Nelson CA, Sukupolvi-Petty S et al. (2006) Antibody recognition and neutralization determinants on domains I and II of West Nile Virus envelope protein. Journal of virology 80(24):12149-12159.
27. Allwinn R, Doerr H, Emmerich P, Schmitz H, Preiser W (2002) Cross-reactivity in flavivirus serology: new implications of an old finding? Med Microbiol Immunol 190 (4): 199-202. doi:10.1007/s00430-001-0107-9.
28. Koraka P, Zeller H, Niedrig M, Osterhaus AD, Groen J (2002) Reactivity of serum samples from patients with a flavivirus infection measured by immunofluorescence assay and ELISA. Microbes and Infection 4 (12): 1209-1215. doi:10.1016/S1286-4579(02)01647-7.
29. Dejnirattisai W, Jumnainsong A, Onsirisakul N, Fitton P, Vasanawathana S, et al. (2010) Cross-reacting antibodies enhance dengue virus infection in humans. Science (New York, N.Y.) 328 (5979): 745-748. doi:10.1126/science.1185181.

## Claims

1. A composition or kit-of-parts comprising the mutant peptides of (a), (b), (c), and (d):
(a) the T76R, Q77E, W101R, L107R quadruple mutant of the peptide which is the wildtype ectodomain of the E protein of a dengue virus (DENV) of serotype 1, or a biologically active variant thereof,
(b) the T76R, Q77E, W101R, L107R quadruple mutant of the peptide which is the wildtype ectodomain of the E protein of a dengue virus (DENV) of serotype 2, or a biologically active variant thereof,
(c) the T76R, Q77E, W101R, L107R quadruple mutant of the peptide which is the wildtype ectodomain of the E protein of a dengue virus (DENV) of serotype 3, or a biologically active variant thereof,
(d) the T76R, Q77E, W101R, L107R quadruple mutant of the peptide representing the wildtype ectodomain of the E protein of a dengue virus (DENV) of serotype 4, or a biologically active variant thereof,
wherein the wildtype ectodomain of the E protein of a dengue virus (DENV) consists of amino acids 1 to 404 of the E protein,
and wherein a biologically active variant of a mutant peptide is a peptide wherein
- 1, 2, 3, 4, or 5 of the N-terminal amino acids of the wildtype ectodomain of the E protein are deleted, and/or
- 1, 2, 3, 4, 5, 6, 7 or 8 of the C-terminal amino acids of the wildtype ectodomain of the E protein are deleted, and/or
- the mutant peptide further contains one or more further moieties selected from 1, 2, 3, 4 or 5 of amino acids of a homologous protein sequence, a purification tag, a labeling moiety, a fluorescent moiety, a separation moiety, and a biotin moiety.

2. The composition or kit-of-parts of claim 1, wherein the wildtype ectodomain of the E protein of a dengue virus (DENV) of serotype 1 comprises the sequence according to SEQ ID No: 5 and/or wherein the wildtype ectodomain of the E protein of a dengue virus (DENV) of serotype 2 comprises the sequence according to SEQ ID No: 6, and/or wherein the wildtype ectodomain of the E protein of a dengue virus (DENV) of serotype 3 comprises the sequence according to SEQ ID No: 7 and/or wherein the wildtype ectodomain of the E protein of a dengue virus (DENV) of serotype 4 comprises the sequence according to SEQ ID No: 8.

3. The composition or kit-of-parts according to claim 1 or 2, wherein the biologically active variant thereof is independently the T76R, Q77E, W101R, L107R quadruple mutant of amino acids 1 to 396, 397, 398, 399, 400, 401, 402 or 403 of the peptide which is the wildtype ectodomain of the E protein of the dengue virus (DENV) serotypes,
wherein optionally the mutant peptides independently contain one or more further moieties, selected from a purification tag, a labeling moiety, a fluorescent moiety, a separation moiety, and a biotin moiety.

4. The composition or kit-of-parts according to any of claims 1 to 3, comprising:
(a) the T76R, Q77E, W101R, L107R quadruple mutant of the peptide which is the wildtype ectodomain of the E protein of a dengue virus (DENV) of serotype 1 or amino acids 1 to 396, 397, 398, 399, 400, 401, 402 or 403 thereof,
preferably wherein the wildtype ectodomain of the E protein of a dengue virus (DENV) of serotype 1 consists of amino acids 1 to 404 of SEQ ID No: 1, and
(b) T76R, Q77E, W101R, L107R quadruple mutant of the peptide which is the wildtype ectodomain of the E protein of a dengue virus (DENV) of serotype 2 or amino acids 1 to 396, 397, 398, 399, 400, 401, 402 or 403 thereof,
preferably wherein the wildtype ectodomain of the E protein of a dengue virus (DENV) of serotype 2 consists of amino acids 1 to 404 of SEQ ID No: 2, and
(c) the T76R, Q77E, W101R, L107R quadruple mutant of the peptide which is the wildtype ectodomain of the E protein of a dengue virus (DENV) of serotype 3 or amino acids 1 to 396, 397, 398, 399, 400, 401, 402 or 403 thereof,
preferably wherein the wildtype ectodomain of the E protein of a dengue virus (DENV) of serotype 3 consists of amino acids 1 to 404 of SEQ ID No: 3, and
(d) the T76R, Q77E, W101R, L107R quadruple mutant of the peptide which is the wildtype ectodomain of the E protein of a dengue virus (DENV) of serotype 4,
preferably wherein the wildtype ectodomain of the E protein of a dengue virus (DENV) of serotype 4 consists of amino acids 1 to 404 of SEQ ID No: 4,
wherein optionally the mutant peptides independently contain one or more further moieties selected from a purification tag, a labeling moiety a fluorescent moiety, a separation moiety, and a biotin moiety.

5. The composition or kit-of-parts according to any of claims 1 to 4, wherein the mutant peptides are present in the composition in a weight ratio of (a):(b):(c):(d) of 5 to 10 : 5 to 10 : 5 to 10 : 1 to 5.

6. The composition or kit-of-parts according to any of claims 1 to 5, wherein the mutant peptides are present in the composition in a weight ratio of 5 to 7,5 : 5 to 7,5 : 5 to 7,5 : 1 to 1,5.

7. The composition or kit-of-parts according to any of claims 1 to 6, wherein the mutant peptides are present in the composition in a weight ratio of 5 +/- 10%: 5 +/- 10%: 5 +/- 10%: 1 +/- 10%.

8. A kit or kit-of-parts comprising, preferably containing:
(a) a composition or kit-of-parts according to any of claims 1 to 7,
and,
(b) peptides which are the ectodomain of the wildtype E protein of dengue virus serotypes 1, 2, 3 and 4, wherein the wildtype ectodomain of the E protein of a dengue virus (DENV) consists of amino acids 1 to 404 of the E protein, wherein optionally
- 1, 2, 3, 4, or 5 of the N-terminal amino acids of the ectodomain sequence are deleted, and/or
- 1, 2, 3, 4, 5, 6, 7 or 8 of the C-terminal amino acids of the ectodomain sequence are deleted, and/or
- the wildtype peptide further contains one or more further moieties, selected from 1, 2, 3, 4 or 5 amino acids of the homologous protein sequence, a purification tag, a labeling moiety, a fluorescent moiety, a separation moiety, and a biotin moiety, preferably wherein the mutant peptides, and wildtype peptides, are bound to one or more solid support(s), more preferably wherein the solid support is selected from a plate, a well plate, an array, a microarray or nanoarray, a bead, or a magnetic bead.

9. Use of a kit or kit-of-parts according to claim 8, or of a composition or kit-of-parts according to any of claims 1 to 7, for discriminating infections with a Dengue virus from infections with other flavivirus(es), preferably from infections with other clinically relevant flavivirus infection(s),
more preferably wherein a DENV infection can be discriminated from infections with TBEV and/or WNV and/or YFV.

10. A method for discriminating infections with a Dengue virus from infections with other flavivirus(es), preferably with other clinically relevant flavivirus infection(s), comprising the steps:
(1) contacting a composition or kit-of-parts according to any of claims 1 to 7, with an animal sample containing or suspected to contain antibodies binding to the specific flavivirus,
(2) determining the amount of mutant peptide-antibody complexes formed in step (1),
wherein the detection of mutant peptide-antibody complexes is indicative of a Dengue virus infection.

11. The method of claim 10, wherein determining the amount of mutant peptide-antibody complexes formed in step (1) is performed by an ELISA assay, preferably wherein
- the mutant peptides of the composition or kit-of-parts are bound to a solid support, and/or
- wherein the amounts of the mutant peptide-antibody complexes are determined using an optionally labeled secondary antibody.

12. The method of claim 10, wherein determining the amount of mutant peptide-antibody complexes formed in step (1) is performed by a capture assay, preferably by performing following steps:
- providing capture antibodies bound to a solid support,
- contacting the animal sample with the capture antibodies bound to a solid support, whereby antibody-antibody complexes bound to a solid support are formed,
- contacting the mutant peptides of the composition or kit-of-parts with the antibody-antibody complexes bound to the solid support, and
- determining the amounts of mutant peptide-antibody complexes by an optionally labeled secondary antibody,
more preferably wherein the optionally labeled secondary antibody specifically recognizes an epitope on the mutant peptides, which is at least 5, 10, or 15 amino acids distant from the fusion loop domain, in particular wherein the fusion loop domain consists of amino acids 63 to 120 of the E protein of a dengue virus,
even more preferably wherein capture antibodies are anti-mammalian IgM antibodies and/or anti-mammalian IgG antibodies, and/or anti-mammalian IgA antibodies and/or anti-avian IgY antibodies and/or wherein the animal suffers from an acute infection or a chronic infection or has suffered from an infection in the past.

13. The method of any of claims 10 to 12, wherein the animal sample is a fluid or solid bodily sample, in particular wherein the fluid bodily sample is selected from blood, whole blood, serum, plasma, cerebrospinal fluid, saliva, urine, spinal fluid, synovial fluid, amniotic fluid, tear fluid and cranial fluid and/or wherein the solid bodily sample is a skin, muscle or mucosal sample, such as a biopsy and/or wherein IgA, IgM and/or IgG antibodies, or IgY antibodies are detected.

## Patentansprüche

1. Zusammensetzung oder kit-of-parts, umfassend die mutierten Peptide von (a), (b), (c) und (d):
(a) die T76R-, Q77E-, W101R-, L107R Vierfach-Mutante des Peptids, das die Wildtyp-Ektodomäne des E-Proteins eines Dengue-Virus (DENV) des Serotyps 1 ist, oder eine biologisch aktive Variante davon,
(b) die T76R, Q77E, W101R, L107R Vierfach-Mutante des Peptids, das die Wildtyp-Ektodomäne des E-Proteins eines Dengue-Virus (DENV) des Serotyps 2 ist, oder eine biologisch aktive Variante davon,
(c) die T76R, Q77E, W101R, L107R Vierfach-Mutante des Peptids, das die Wildtyp-Ektodomäne des E-Proteins eines Dengue-Virus (DENV) des Serotyps 3 ist, oder eine biologisch aktive Variante davon,
(d) die T76R, Q77E, W101R, L107R Vierfach-Mutante des Peptids, das die Wildtyp-Ektodomäne des E-Proteins eines Dengue-Virus (DENV) des Serotyps 4 oder eine biologisch aktive Variante davon,
wobei die Wildtyp-Ektodomäne des E-Proteins eines Dengue-Virus (DENV) aus den Aminosäuren 1 bis 404 des E-Proteins besteht,
und wobei eine biologisch aktive Variante eines mutierten Peptids ein Peptid ist, wobei
- 1, 2, 3, 4 oder 5 der N-terminalen Aminosäuren der Wildtyp-Ektodomäne des E-Proteins deletiert sind und/oder
- 1, 2, 3, 4, 5, 6, 7 oder 8 der C-terminalen Aminosäuren der Wildtyp-Ektodomäne des E-Proteins deletiert sind, und/oder
- das mutierte Peptid ferner eine oder mehrere weitere Gruppen enthält, ausgewählt aus 1, 2, 3, 4 oder 5 Aminosäuren einer homologen Proteinsequenz, einem Aufreinigungs-Tag, einer Markierungs-Gruppe, einer fluoreszierende Gruppe, einer Trenn-Gruppe und einer Biotin-Gruppe.

2. Zusammensetzung oder kit-of-parts nach Anspruch 1, wobei die Wildtyp-Ektodomäne des E-Proteins eines Dengue-Virus (DENV) des Serotyps 1 die Sequenz gemäß SEQ ID Nr.: 5 umfasst und/oder wobei die Wildtyp-Ektodomäne des E-Proteins eines Dengue-Virus (DENV) des Serotyps 2 die Sequenz gemäß SEQ ID Nr.: 6 umfasst, und/oder wobei die Wildtypektodomäne des E-Proteins eines Dengue-Virus (DENV) des Serotyps 3 die Sequenz gemäß SEQ ID Nr.: 7 umfasst und/oder wobei die Wildtypektodomäne des E-Proteins eines Dengue-Virus (DENV) des Serotyps 4 die Sequenz gemäß SEQ ID Nr.: 8 umfasst.

3. Zusammensetzung oder kit-of-parts gemäß Anspruch 1 oder 2, wobei die biologisch aktive Variante davon unabhängig die T76R, Q77E, W101R, L107R Vierfach-Mutante der Aminosäuren 1 bis 396, 397, 398, 399, 400, 401, 402 oder 403 des Peptids ist, das die Wildtyp-Ektodomäne des E-Proteins der Serotypen des Dengue-Virus (DENV) ist,
wobei gegebenenfalls die mutierten Peptide unabhängig voneinander eine oder mehrere weitere Gruppen enthalten, ausgewählt aus einem Reinigungs-Tag, einer Markierungs-Gruppe, einer fluoreszierenden Gruppe, einer Trenn-Gruppe und einer Biotin-Gruppe.

4. Zusammensetzung oder kit-of-parts gemäß einem beliebigen der Ansprüche 1 bis 3, umfassend:
(a) die T76R, Q77E, W101R, L107R Vierfach-Mutante des Peptids, das die Wildtyp-Ektodomäne des E-Proteins eines Dengue-Virus (DENV) des Serotyps 1 ist, oder die Aminosäuren 1 bis 396, 397, 398, 399, 400, 401, 402 oder 403 davon,
vorzugsweise wobei die Wildtyp-Ektodomäne des E-Proteins eines Dengue-Virus (DENV) des Serotyps 1 aus den Aminosäuren 1 bis 404 der SEQ ID Nr.: 1 besteht, und
(b) die T76R, Q77E, W101R, L107R Vierfach-Mutante des Peptids, das die Wildtyp-Ektodomäne des E-Proteins eines Dengue-Virus (DENV) des Serotyps 2 ist, oder der Aminosäuren 1 bis 396, 397, 398, 399, 400, 401, 402 oder 403 davon,
vorzugsweise wobei die Wildtyp-Ektodomäne des E-Proteins eines Dengue-Virus (DENV) des Serotyps 2 aus den Aminosäuren 1 bis 404 der SEQ ID Nr.: 2 besteht, und
c) die T76R, Q77E, W101R, L107R Vierfach-Mutante des Peptids, das die Wildtyp-Ektodomäne des E-Proteins eines Dengue-Virus (DENV) des Serotyps 3 ist, oder die Aminosäuren 1 bis 396, 397, 398, 399, 400, 401, 402 oder 403 davon,
vorzugsweise wobei die Wildtyp-Ektodomäne des E-Proteins eines Dengue-Virus (DENV) des Serotyps 3 aus den Aminosäuren 1 bis 404 der SEQ ID Nr.: 3 besteht, und
(d) die T76R, Q77E, W101R, L107R Vierfach-Mutante des Peptids, das die Wildtyp-Ektodomäne des E-Proteins eines Dengue-Virus (DENV) des Serotyps 4 ist,
vorzugsweise wobei die Wildtyp-Ektodomäne des E-Proteins eines Dengue-Virus (DENV) des Serotyps 4 aus den Aminosäuren 1 bis 404 der SEQ ID Nr.: 4 besteht,
wobei gegebenenfalls die mutierten Peptide unabhängig voneinander eine oder mehrere weitere Gruppen enthalten, ausgewählt aus einem Aufreinigungs-Tag, einer Markierungs-Gruppe, einer fluoreszierenden Gruppe, einer Trenn-Gruppe und einer Biotin-Gruppe.

5. Zusammensetzung oder kit-of-parts gemäß einem beliebigen der Ansprüche 1 bis 4, wobei die mutierten Peptide in der Zusammensetzung in einem Gewichtsverhältnis von (a):(b):(c):(d) von 5 bis 10 : 5 bis 10 : 5 bis 10 : 1 bis 5 vorhanden sind.

6. Zusammensetzung oder kit-of-parts gemäß einem beliebigen der Ansprüche 1 bis 5, wobei die mutierten Peptide in der Zusammensetzung in einem Gewichtsverhältnis von 5 bis 7,5 : 5 bis 7,5 : 5 bis 7,5 : 5 bis 7,5 : 1 bis 1,5 vorhanden sind.

7. Zusammensetzung oder kit-of-parts gemäß einem beliebigen der Ansprüche 1 bis 6, wobei die mutierten Peptide in der Zusammensetzung in einem Gewichtsverhältnis von 5 +/- 10% : 5 +/- 10% : 5 +/- 10% : 1 +/- 10% vorhanden sind.

8. Kit oder kit-of-parts, umfassend, vorzugsweise enthaltend:
(a) eine Zusammensetzung oder kit-of-parts gemäß einem beliebigen der Ansprüche 1 bis 7,
und,
(b) Peptide, die die Ektodomäne des Wildtyp-E-Proteins von Dengue-Virus-Serotypen 1, 2, 3 und 4 sind, wobei die Wildtyp-Ektodomäne des E-Proteins eines Dengue-Virus (DENV) aus den Aminosäuren 1 bis 404 des E-Proteins besteht, wobei gegebenenfalls
- 1, 2, 3, 4 oder 5 der N-terminalen Aminosäuren der Ektodomänensequenz deletiert sind, und/oder
- 1, 2, 3, 4, 5, 6, 7 oder 8 der C-terminalen Aminosäuren der Ektodomänensequenz deletiert sind, und/oder
- Das Wildtyp-Peptid ferner eine oder mehrere weitere Gruppen enthält, ausgewählt aus 1, 2, 3, 4 oder 5 Aminosäuren der homologen Proteinsequenz, einem Reinigungs-Tag, einer Markierungs-Gruppe, einer fluoreszierenden Gruppe, einer Trenn-Gruppe und einer Biotin-Gruppe,
vorzugsweise wobei die mutierten Peptide und Wildtyp-Peptide an einen oder mehrere feste Träger gebunden sind, stärker bevorzugt wobei der feste Träger aus einer Platte, einer Platte mit Vertiefungen (well plate), einem Array, einem Mikroarray oder Nanoarray, einem Kügelchen oder einem magnetischen Kügelchen ausgewählt ist.

9. Verwendung eines Kits oder kit-of-parts gemäß Anspruch 8 oder einer Zusammensetzung oder kit-of-parts gemäß einem beliebigen der Ansprüche 1 bis 7 zur Unterscheidung von Dengue-Virus-Infektionen von Infektionen mit (einem) anderen Flavivirus(en), vorzugsweise von Infektionen mit (einer) anderen klinisch relevanten Flavivirus-Infektion(en), stärker bevorzugt, wobei eine DENV-Infektion von Infektionen mit FSME und/oder WNV und/oder YFV unterschieden werden kann.

10. Verfahren zum Unterscheiden von Dengue-Virus-Infektionen von Infektionen mit (einem) anderen Flavivirus(en), vorzugsweise mit (einer) anderen klinisch relevanten Flavivirus-Infektion(en), umfassend die Schritte:
(1) Inkontaktbringen einer Zusammensetzung oder kit-of-parts gemäß einem beliebigen der Ansprüche 1 bis 7 mit einer Tierprobe, die Antikörper enthält oder bei der der Verdacht besteht, dass sie Antikörper enthält, die an das spezifische Flavivirus binden,
(2) Bestimmen der Menge an mutiertes-Peptid-Antikörper-Komplexen, die in Schritt (1) gebildet wurden,
wobei die Detektion von mutiertes-Peptid-Antikörper-Komplexen eine Dengue-Virus-Infektion anzeigt.

11. Verfahren nach Anspruch 10, wobei das Bestimmen der Menge der in Schritt (1) gebildeten mutiertes-Peptid-Antikörper-Komplexe durch einen ELISA-Test durchgeführt wird, vorzugsweise wobei
- die mutierten Peptide der Zusammensetzung oder kit-of-parts an einen festen Träger gebunden sind, und/oder
- wobei die Mengen der mutiertes-Peptid-Antikörper-Komplexe unter Verwendung eines gegebenenfalls markierten Sekundärantikörpers bestimmt werden.

12. Verfahren nach Anspruch 10, wobei das Bestimmen der Menge der in Schritt (1) gebildeten mutiertes-Peptid-Antikörper-Komplexe durch einen Capture-Assay durchgeführt wird, vorzugsweise durch Durchführen der folgenden Schritte:
- Bereitstellen von Capture-Antikörpern, die an einen festen Träger gebunden sind,
- Inkontaktbringen der Tierprobe mit den an einen festen Träger gebundenen Capture-Antikörpern, wodurch an einen festen Träger gebundene Antikörper-Antikörper-Komplexe gebildet werden,
- Inkontaktbringen der mutierten Peptide der Zusammensetzung oder kit-of-parts mit den an den festen Träger gebundenen Antikörper-Antikörper-Komplexen, und
- Bestimmen der Mengen an mutiertes-Peptid-Antikörper-Komplexen durch einen gegebenenfalls markierten Sekundärantikörper,
stärker bevorzugt wobei der gegebenenfalls markierte Sekundärantikörper spezifisch ein Epitop auf den mutierten Peptiden erkennt, das mindestens 5, 10 oder 15 Aminosäuren von der Fusionsschleifendomäne entfernt ist, insbesondere wobei die Fusionsschleifendomäne aus den Aminosäuren 63 bis 120 des E-Proteins eines Dengue-Virus besteht,
noch stärker bevorzugt wobei Capture-Antikörpern Anti-Säugetier-IgM-Antikörper und/oder Anti-Säugetier-IgG-Antikörper und/oder Anti-Säugetier-IgA-Antikörper und/oder Anti-Aviäre-lgY-Antikörper sind und/oder das Tier an einer akuten oder chronischen Infektion leidet oder in der Vergangenheit eine Infektion erlitten hat.

13. Verfahren nach einem beliebigen der Ansprüche 10 bis 12, wobei die Tierprobe eine flüssige oder feste Körperprobe ist, insbesondere wobei die flüssige Körperprobe aus Blut, Vollblut, Serum, Plasma, Liquor, Speichel, Urin, Rückenmarksflüssigkeit, Synovialflüssigkeit, Fruchtwasser, Tränenflüssigkeit und Schädelflüssigkeit ausgewählt ist und/oder wobei die feste Körperprobe eine Haut-, Muskel- oder Schleimhautprobe ist, wie eine Biopsie und/oder wobei IgA-, IgM- und/oder IgG-Antikörper oder IgY-Antikörper detektiert werden.

## Revendications

1. Composition de kit de parties comprenant les peptides mutants de (a), (b), (c) et (d) :
(a) le quadruple mutant T76R, Q77E, W101R, L107R du peptide qui est l'ectodomaine de type sauvage de la protéine E d'un virus de la dengue (DENV) de sérotype 1, ou d'un de ses variants biologiquement actifs,
(b) le quadruple mutant T76R, Q77E, W101R, L107R du peptide qui est l'ectodomaine de type sauvage de la protéine E d'un virus de la dengue (DENV) de sérotype 2, ou d'un de ses variants biologiquement actifs,
(c) le quadruple mutant T76R, Q77E, W101R, L107R du peptide qui est l'ectodomaine de type sauvage de la protéine E d'un virus de la dengue (DENV) de sérotype 3, ou d'un de ses variants biologiquement actifs,
(d) le quadruple mutant T76R, Q77E, W101R, L107R du peptide qui est l'ectodomaine de type sauvage de la protéine E d'un virus de la dengue (DENV) de sérotype 4, ou d'un de ses variants biologiquement actifs,
où l'ectodomaine de type sauvage de la protéine E d'un virus de la dengue (DENV) est constitué des acides aminés 1 à 404 de la protéine E,
et où un variant biologiquement actif d'un peptide mutant est un peptide dans lequel
- 1, 2, 3, 4 ou 5 des acides aminés N-terminaux de l'ectodomaine de type sauvage de la protéine E sont délétés, et/ou
- 1, 2, 3, 4, 5, 6, 7 ou 8 des acides aminés C-terminaux de l'ectodomaine de type sauvage de la protéine E sont délétés, et/ou
- le peptide mutant contient en outre un ou plusieurs autres groupements choisies parmi 1, 2, 3, 4 ou 5 des acides aminés d'une séquence de protéine homologue, d'une étiquette de purification, d'un groupement de marquage, d'un groupement fluorescent, d'un groupement de séparation et d'un groupement biotine.

2. Composition ou kit de parties selon la revendication 1, où l'ectodomaine de type sauvage de la protéine E d'un virus de la dengue (DENV) de sérotype 1 comprend la séquence selon la SEQ ID NO: 5 et/ou l'ectodomaine de type sauvage de la protéine E d'un virus de la dengue (DENV) de sérotype 2 comprend la séquence selon la SEQ ID NO: 6, et/ou l'ectodomaine de type sauvage de la protéine E d'un virus de la dengue (DENV) de sérotype 3 comprend la séquence selon la SEQ ID NO: 7 et/ou l'ectodomaine de type sauvage de la protéine E d'un virus de la dengue (DENV) de sérotype 4 comprend la séquence selon la SEQ ID NO: 8.

3. Composition ou kit de parties selon la revendication 1 ou 2, où un de ses variants biologiquement actifs est indépendamment le quadruple mutant T76R, Q77E, W101R, L107R des acides aminés 1 à 396, 397, 398, 399, 400, 401, 402 ou 403 du peptide qui est l'ectodomaine de type sauvage de la protéine E des sérotypes du virus de la dengue (DENV),
où éventuellement les peptides mutants contiennent indépendamment un ou plusieurs autres groupements, choisis parmi une étiquette de purification, un groupement de marquage, un groupement fluorescent, un groupement de séparation et un groupement biotine.

4. Composition ou kit de parties selon l'une quelconque des revendications 1 à 3, comprenant
(a) le quadruple mutant T76R, Q77E, W101R, L107R du peptide qui est l'ectodomaine de type sauvage de la protéine E d'un virus de la dengue (DENV) de sérotype 1 ou un de ses acides aminés 1 à 396, 397, 398, 399, 400, 401, 402 ou 403,
de préférence où l'ectodomaine de type sauvage de la protéine E d'un virus de la dengue (DENV) de sérotype 1 est constitué des acides aminés 1 à 404 de la SEQ ID NO: 1, et
(b) le quadruple mutant T76R, Q77E, W101R, L107R du peptide qui est l'ectodomaine de type sauvage de la protéine E d'un virus de la dengue (DENV) de sérotype 2 ou un de ses acides aminés 1 à 396, 397, 398, 399, 400, 401, 402 ou 403,
de préférence où l'ectodomaine de type sauvage de la protéine E d'un virus de la dengue (DENV) de sérotype 2 est constitué des acides aminés 1 à 404 de la SEQ ID NO: 2, et
(c) le quadruple mutant T76R, Q77E, W101R, L107R du peptide qui est l'ectodomaine de type sauvage de la protéine E d'un virus de la dengue (DENV) de sérotype 3 ou un de ses acides aminés 1 à 396, 397, 398, 399, 400, 401, 402 ou 403,
de préférence où l'ectodomaine de type sauvage de la protéine E d'un virus de la dengue (DENV) de sérotype 3 est constitué des acides aminés 1 à 404 de la SEQ ID NO: 3, et
(d) le quadruple mutant T76R, Q77E, W101R, L107R du peptide qui est l'ectodomaine de type sauvage de la protéine E d'un virus de la dengue (DENV) de sérotype 4,
de préférence où l'ectodomaine de type sauvage de la protéine E d'un virus de la dengue (DENV) de sérotype 4 est constitué des acides aminés 1 à 404 de la SEQ ID NO: 4,
éventuellement où les peptides mutants contiennent indépendamment un ou plusieurs autres groupements, choisis parmi une étiquette de purification, un groupement de marquage, un groupement fluorescent, un groupement de séparation et un groupement biotine.

5. Composition ou kit de parties selon l'une quelconque des revendications 1 à 4, où les peptides mutants sont présents dans la composition dans un rapport en poids de (a):(b):(c):(d) de 5 à 10:5 à 10:5 à 10:1 à 5.

6. Composition ou kit de parties selon l'une quelconque des revendications 1 à 5, où les peptides mutants sont présents dans la composition dans un rapport en poids de 5 à 7,5:5 à 7,5:5 à 7,5:1 à 1,5.

7. Composition ou kit de parties selon l'une quelconque des revendications 1 à 6, où les peptides mutants sont présents dans la composition dans un rapport en poids de 5 ± 10 %:5 ± 10 %:5 ± 10 %:1 ± 10 %.

8. Kit ou kit de parties comprenant, de préférence contenant :
(a) une composition ou un kit de parties selon l'une quelconque des revendications 1 à 7,
et,
(b) des peptides qui sont l'ectodomaine de la protéine E de type sauvage des sérotypes 1, 2, 3 et 4 du virus de la dengue, l'ectodomaine de type sauvage de la protéine E d'un virus de la dengue (DENV) étant constitué des acides aminés 1 à 404 de la protéine E, où éventuellement
- 1, 2, 3, 4 ou 5 des acides aminés N-terminaux de la séquence d'ectodomaine sont délétés, et/ou
- 1, 2, 3, 4, 5, 6, 7 ou 8 des acides aminés C-terminaux de la séquence d'ectodomaine sont délétés, et/ou
- le peptide de type sauvage contient en outre un ou plusieurs autres groupements, choisis parmi 1, 2, 3, 4 ou 5 acides aminés de la séquence protéique homologue, une étiquette de purification, un groupement de marquage, un groupement fluorescent, un groupement de séparation et un groupement biotine,
où de préférence les peptides mutants, et les peptides de type sauvages, sont liés à un ou plusieurs supports solides, plus préférablement le support solide est choisi parmi une plaque, une plaque de puits, un réseau, un microréseau ou un nanoréseau, une bille ou une bille magnétique.

9. Utilisation d'un kit ou d'un kit de parties selon la revendication 8, ou d'une composition ou d'un kit de parties selon l'une quelconque des revendications 1 à 7, pour discriminer des infections avec un virus de la dengue d'infections avec un ou plusieurs autres flavivirus, de préférence d'infections avec une ou plusieurs infections par flavivirus cliniquement pertinente(s),
plus préférablement où une infection par DENV peut être discriminée d'infections avec le TBEV et/ou le WNV et/ou l'YFV.

10. Procédé de discrimination d'infections avec un virus de la dengue d'infections avec d'autres flavivirus, de préférence avec une ou plusieurs autres infections par flavivirus cliniquement pertinentes, comprenant les étapes consistant à :
(1) mettre en contact une composition ou un kit de parties selon l'une quelconque des revendications 1 à 7, avec un échantillon animal contenant ou suspecté de contenir des anticorps se liant au flavivirus spécifique,
(2) déterminer la quantité de complexes peptide mutant-anticorps formés dans l'étape (1),
dans lequel la détection des complexes peptide mutant-anticorps est indicatrice d'une infection par le virus de la dengue.

11. Procédé selon la revendication 10, dans lequel la détermination de la quantité de complexes peptide mutant-anticorps formés à l'étape (1) est effectuée par un test ELISA, de préférence dans lequel
- les peptides mutants de la composition ou du kit de parties sont liés à un support solide, et/ou
- les quantités des complexes peptide mutant-anticorps sont déterminées en utilisant un anticorps secondaire éventuellement marqué.

12. Procédé selon la revendication 10, dans lequel la quantité des complexes peptide mutant-anticorps formés à l'étape (1) est effectuée par un test de capture, de préférence en effectuant les étapes suivantes :
- fournir des anticorps de capture liés à un support solide,
- mettre en contact l'échantillon animal avec les anticorps de capture liés à un support solide, moyennant quoi des complexes anticorps-anticorps liés à un support solide sont formés,
- mettre en contact les peptides mutants de la composition ou du kit de parties avec les complexes anticorps-anticorps liés à un support solide, et
- déterminer les quantités de complexes peptide mutant-anticorps par un anticorps secondaire éventuellement marqué,
plus préférablement dans lequel l'anticorps secondaire éventuellement marqué reconnaît spécifiquement un épitope sur les peptides mutants, qui est au moins 5, 10 ou 15 acide aminés distant du domaine de boucle de fusion, en particulier le domaine de la boucle de fusion étant constitué des acides aminés 63 à 120 de la protéine E d'un virus de la dengue,
encore plus préférablement dans lequel les anticorps de capture sont des anticorps IgM anti-mammifère et/ou des anticorps IgG anti-mammifère, et/ou des anticorps IgA anti-mammifère et/ou des anticorps IgY anti-aviaire et/ou dans lequel l'animal souffre d'une infection aiguë ou d'une infection chronique ou a souffert d'une infection dans le passé.

13. Procédé selon l'une quelconque des revendications 10 à 12, dans lequel l'échantillon animal est un fluide ou un échantillon corporel solide, en particulier dans lequel l'échantillon corporel fluide est choisi parmi le sang, le sang entier, le sérum, le plasma, le fluide cérébrospinal, la salive, l'urine, le fluide spinal, le fluide synovial, le fluide amniotique, le fluide de larmes et le fluide crânien et/ou dans lequel l'échantillon corporel solide est une peau, un muscle ou un échantillon mucosal, tel qu'une biopsie et/ou dans lequel des anticorps IgA, IgM et/ou IgG ou des anticorps IgY sont détectés.


### SEQUENCE LISTING

<110> Fraunhofer-Gesellschaft zur Förderung der angewandten Forschung e.V.
<120> Distinguishing Dengue virus infections from other flaviviral infections using a recombinant mutant peptide
<130> F69931EP
<160> 25
<170> PatentIn version 3.5
<210> 1
   <211> 404
   <212> PRT
   <213> Dengue virus
<400> 1
   Met Arg Cys Val Gly Ile Gly Asn Arg Asp Phe Val Glu Gly Leu Ser
   1               5                   10                  15
   Gly Ala Thr Trp Val Asp Val Val Leu Glu His Gly Ser Cys Val Thr
               20                  25                  30
   Thr Met Ala Lys Asp Lys Pro Thr Leu Asp Ile Glu Leu Leu Lys Thr
           35                  40                  45
   Glu Val Thr Asn Pro Ala Val Leu Arg Lys Leu Cys Ile Glu Ala Lys
       50                  55                  60
   Ile Ser Asn Thr Thr Thr Asp Ser Arg Cys Pro Thr Gln Gly Glu Ala
   65                  70                  75                  80
   Thr Leu Val Glu Glu Gln Asp Thr Asn Phe Val Cys Arg Arg Thr Phe
                   85                  90                  95
   Val Asp Arg Gly Trp Gly Asn Gly Cys Gly Leu Phe Gly Lys Gly Ser
               100                 105                 110
   Leu Ile Thr Cys Ala Lys Phe Lys Cys Val Thr Lys Leu Glu Gly Lys
           115                 120                 125
   Ile Val Gln Tyr Glu Asn Leu Lys Tyr Ser Val Ile Val Thr Val His
       130                 135                 140
   Thr Gly Asp Gln His Gln Val Gly Asn Glu Thr Thr Glu His Gly Thr
   145                 150                 155                 160
   Thr Ala Thr Ile Thr Pro Gln Ala Pro Thr Ser Glu Ile Gln Leu Thr
                   165                 170                 175
   Asp Tyr Gly Ala Leu Thr Leu Asp Cys Ser Pro Arg Thr Gly Leu Asp
               180                 185                 190
   Phe Asn Glu Met Val Leu Leu Thr Met Lys Lys Lys Ser Trp Leu Val
           195                 200                 205
   His Lys Gln Trp Phe Leu Asp Leu Pro Leu Pro Trp Thr Ser Gly Ala
       210                 215                 220
   Ser Thr Ser Gln Glu Thr Trp Asn Arg Gln Asp Leu Leu Val Thr Phe
   225                 230                 235                 240
   Lys Thr Ala His Ala Lys Lys Gln Glu Val Val Val Leu Gly Ser Gln
                   245                 250                 255
   Glu Gly Ala Met His Thr Ala Leu Thr Gly Ala Thr Glu Ile Gln Thr
               260                 265                 270
   Ser Gly Thr Thr Thr Ile Phe Ala Gly His Leu Lys Cys Arg Leu Lys
           275                 280                 285
   Met Asp Lys Leu Ile Leu Lys Gly Met Ser Tyr Val Met Cys Thr Gly
       290                 295                 300
   Ser Phe Lys Leu Glu Lys Glu Val Ala Glu Thr Gln His Gly Thr Val
   305                 310                 315                 320
   Leu Val Gln Val Lys Tyr Glu Gly Thr Asp Ala Pro Cys Lys Ile Pro
                   325                 330                 335
   Phe Ser Ser Gln Asp Glu Lys Gly Val Thr Gln Asn Gly Arg Leu Ile
               340                 345                 350
   Thr Ala Asn Pro Ile Val Thr Asp Lys Glu Lys Pro Val Asn Ile Glu
           355                 360                 365
   Ala Glu Pro Pro Phe Gly Glu Ser Tyr Ile Val Val Gly Ala Gly Glu
       370                 375                 380
   Lys Ala Leu Lys Leu Ser Trp Phe Lys Lys Gly Ser Ser Ile Gly Lys
   385                 390                 395                 400
   Met Phe Glu Ala
<210> 2
   <211> 404
   <212> PRT
   <213> Dengue virus
<400> 2
   Met Arg Cys Ile Gly Met Ser Asn Arg Asp Phe Val Glu Gly Val Ser
   1               5                   10                  15
   Gly Gly Ser Trp Val Asp Ile Val Leu Glu His Gly Ser Cys Val Thr
               20                  25                  30
   Thr Met Ala Lys Asn Lys Pro Thr Leu Asp Phe Glu Leu Ile Lys Thr
           35                  40                  45
   Glu Ala Lys Gln Pro Ala Thr Leu Arg Lys Tyr Cys Ile Glu Ala Lys
       50                  55                  60
   Leu Thr Asn Thr Thr Thr Glu Ser Arg Cys Pro Thr Gln Gly Glu Pro
   65                  70                  75                  80
   Ser Leu Asn Glu Glu Gln Asp Lys Arg Phe Val Cys Lys His Ser Met
                   85                  90                  95
   Val Asp Arg Gly Trp Gly Asn Gly Cys Gly Leu Phe Gly Lys Gly Gly
               100                 105                 110
   Ile Val Thr Cys Ala Met Phe Arg Cys Lys Lys Asn Met Glu Gly Lys
           115                 120                 125
   Val Val Gln Pro Glu Asn Leu Glu Tyr Thr Ile Val Ile Thr Pro His
       130                 135                 140
   Ser Gly Glu Glu His Ala Val Gly Asn Asp Thr Gly Lys His Gly Lys
   145                 150                 155                 160
   Glu Ile Lys Ile Thr Pro Gln Ser Ser Ile Thr Glu Ala Glu Leu Thr
                   165                 170                 175
   Gly Tyr Gly Thr Val Thr Met Glu Cys Ser Pro Arg Thr Gly Leu Asp
               180                 185                 190
   Phe Asn Glu Met Val Leu Leu Gln Met Glu Asn Lys Ala Trp Leu Val
           195                 200                 205
   His Arg Gln Trp Phe Leu Asp Leu Pro Leu Pro Trp Leu Pro Gly Ala
       210                 215                 220
   Asp Thr Gln Gly Ser Asn Trp Ile Gln Lys Glu Thr Leu Val Thr Phe
   225                 230                 235                 240
   Lys Asn Pro His Ala Lys Lys Gln Asp Val Val Val Leu Gly Ser Gln
                   245                 250                 255
   Glu Gly Ala Met His Thr Ala Leu Thr Gly Ala Thr Glu Ile Gln Met
               260                 265                 270
   Ser Ser Gly Asn Leu Leu Phe Thr Gly His Leu Lys Cys Arg Leu Arg
           275                 280                 285
   Met Asp Lys Leu Gln Leu Lys Gly Met Ser Tyr Ser Met Cys Thr Gly
       290                 295                 300
   Lys Phe Lys Val Val Lys Glu Ile Ala Glu Thr Gln His Gly Thr Ile
   305                 310                 315                 320
   Val Ile Arg Val Gln Tyr Glu Gly Asp Gly Ser Pro Cys Lys Ile Pro
                   325                 330                 335
   Phe Glu Ile Met Asp Leu Glu Lys Arg His Val Leu Gly Arg Leu Ile
               340                 345                 350
   Thr Val Asn Pro Ile Val Thr Glu Lys Asp Ser Pro Val Asn Ile Glu
           355                 360                 365
   Ala Glu Pro Pro Phe Gly Asp Ser Tyr Ile Ile Ile Gly Val Glu Pro
       370                 375                 380
   Gly Gln Leu Lys Leu Asn Trp Phe Lys Lys Gly Ser Ser Ile Gly Gln
   385                 390                 395                 400
   Met Phe Glu Thr
<210> 3
   <211> 404
   <212> PRT
   <213> Dengue virus
<400> 3
   Met Arg Cys Val Gly Val Gly Asn Arg Asp Phe Val Glu Gly Leu Ser
   1               5                   10                  15
   Gly Ala Thr Trp Val Asp Val Val Leu Glu His Gly Gly Cys Val Thr
               20                  25                  30
   Thr Met Ala Lys Asn Lys Pro Thr Leu Asp Ile Glu Leu Gln Lys Thr
           35                  40                  45
   Glu Ala Thr Gln Leu Ala Thr Leu Arg Lys Leu Cys Ile Glu Gly Lys
       50                  55                  60
   Ile Thr Asn Ile Thr Thr Asp Ser Arg Cys Pro Thr Gln Gly Glu Ala
   65                  70                  75                  80
   Val Leu Pro Glu Glu Gln Asp Gln Asn Tyr Val Cys Lys His Thr Tyr
                   85                  90                  95
   Val Asp Arg Gly Trp Gly Asn Gly Cys Gly Leu Phe Gly Lys Gly Ser
               100                 105                 110
   Leu Val Thr Cys Ala Lys Phe Gln Cys Leu Glu Pro Ile Glu Gly Lys
           115                 120                 125
   Val Val Gln Tyr Glu Asn Leu Lys Tyr Thr Val Ile Ile Thr Val His
       130                 135                 140
   Thr Gly Asp Gln His Gln Val Gly Asn Glu Thr Gln Gly Val Thr Ala
   145                 150                 155                 160
   Glu Ile Thr Pro Gln Ala Ser Thr Thr Glu Ala Ile Leu Pro Glu Tyr
                   165                 170                 175
   Gly Thr Leu Gly Leu Glu Cys Ser Pro Arg Thr Gly Leu Asp Phe Asn
               180                 185                 190
   Glu Met Ile Leu Leu Thr Met Lys Asn Lys Ala Trp Met Val His Arg
           195                 200                 205
   Gln Trp Phe Phe Asp Leu Pro Leu Pro Trp Ala Ser Gly Ala Thr Thr
       210                 215                 220
   Glu Thr Pro Thr Trp Asn Arg Lys Glu Leu Leu Val Thr Phe Lys Asn
   225                 230                 235                 240
   Ala His Ala Lys Lys Gln Glu Val Val Val Leu Gly Ser Gln Glu Gly
                   245                 250                 255
   Ala Met His Thr Ala Leu Thr Gly Ala Thr Glu Ile Gln Asn Ser Gly
               260                 265                 270
   Gly Thr Ser Ile Phe Ala Gly His Leu Lys Cys Arg Leu Lys Met Asp
           275                 280                 285
   Lys Leu Glu Leu Lys Gly Met Ser Tyr Ala Met Cys Thr Asn Thr Phe
   290 295 300
   Val Leu Lys Lys Glu Val Ser Glu Thr Gln His Gly Thr Ile Leu Ile
   305 310 315 320
   Lys Val Glu Tyr Lys Gly Glu Asp Ala Pro Cys Lys Ile Pro Phe Ser
   325 330 335
   Thr Glu Asp Gly Gln Gly Lys Ala His Asn Gly Arg Leu Ile Thr Ala
   340 345 350
   Asn Pro Val Val Thr Lys Lys Glu Glu Pro Val Asn Ile Glu Ala Glu
   355 360 365
   Pro Pro Phe Gly Glu Ser Asn Ile Val Ile Gly Ile Gly Asp Asn Ala
   370 375 380
   Leu Lys Ile Asn Trp Tyr Lys Lys Gly Ser Ser Ile Gly Lys Met Phe
   385 390 395 400
   Glu Ala Thr Glu
<210> 4
   <211> 404
   <212> PRT
   <213> Dengue virus
<400> 4
   Met Arg Cys Val Gly Val Gly Asn Arg Asp Phe Val Glu Gly Val Ser
   1 5 10 15
   Gly Gly Ala Trp Val Asp Leu Val Leu Glu His Gly Gly Cys Val Thr
   20 25 30
   Thr Met Ala Gln Gly Lys Pro Thr Leu Asp Phe Glu Leu Thr Lys Thr
   35 40 45
   Thr Ala Lys Glu Val Ala Leu Leu Arg Thr Tyr Cys Ile Glu Ala Ser
   50 55 60
   Ile Ser Asn Ile Thr Thr Ala Thr Arg Cys Pro Thr Gln Gly Glu Pro
   65 70 75 80
   Tyr Leu Lys Glu Glu Gln Asp Gln Gln Tyr Ile Cys Arg Arg Asp Val
   85 90 95
   Val Asp Arg Gly Trp Gly Asn Gly Cys Gly Leu Phe Gly Lys Gly Gly
               100                 105                 110
   Val Val Thr Cys Ala Lys Phe Ser Cys Ser Gly Lys Ile Thr Gly Asn
           115                 120                 125
   Leu Val Gln Ile Glu Asn Leu Glu Tyr Thr Val Val Val Thr Val His
       130                 135                 140
   Asn Gly Asp Thr His Ala Val Gly Asn Asp Thr Ser Asn His Gly Val
   145                 150                 155                 160
   Thr Ala Met Ile Thr Pro Arg Ser Pro Ser Val Glu Val Lys Leu Pro
                   165                 170                 175
   Asp Tyr Gly Glu Leu Thr Leu Asp Cys Glu Pro Arg Ser Gly Ile Asp
               180                 185                 190
   Phe Asn Glu Met Ile Leu Met Lys Met Lys Lys Lys Thr Trp Leu Val
           195                 200                 205
   His Lys Gln Trp Phe Leu Asp Leu Pro Leu Pro Trp Thr Ala Gly Ala
       210                 215                 220
   Asp Thr Ser Glu Val His Trp Asn Tyr Lys Glu Arg Met Val Thr Phe
   225                 230                 235                 240
   Lys Val Pro His Ala Lys Arg Gln Asp Val Thr Val Leu Gly Ser Gln
                   245                 250                 255
   Glu Gly Ala Met His Ser Ala Leu Ala Gly Ala Thr Glu Val Asp Ser
               260                 265                 270
   Gly Asp Gly Asn His Met Phe Ala Gly His Leu Lys Cys Lys Val Arg
           275                 280                 285
   Met Glu Lys Leu Arg Ile Lys Gly Met Ser Tyr Thr Met Cys Ser Gly
       290                 295                 300
   Lys Phe Ser Ile Asp Lys Glu Met Ala Glu Thr Gln His Gly Thr Thr
   305                 310                 315                 320
   Val Val Lys Val Lys Tyr Glu Gly Ala Gly Ala Pro Cys Lys Val Pro
                   325                 330                 335
   Ile Glu Ile Arg Asp Val Asn Lys Glu Lys Val Val Gly Arg Ile Ile
               340                 345                 350
   Ser Ser Thr Pro Leu Ala Glu Asn Thr Asn Ser Val Thr Asn Ile Glu
           355                 360                 365
   Leu Glu Pro Pro Phe Gly Asp Ser Tyr Ile Val Ile Gly Val Gly Asn
       370                 375                 380
   Ser Ala Leu Thr Leu His Trp Phe Arg Lys Gly Ser Ser Ile Gly Lys
   385                 390                 395                 400
   Met Phe Glu Ser
<210> 5
   <211> 56
   <212> PRT
   <213> Dengue virus
<400> 5
   Asn Thr Thr Thr Asp Ser Arg Cys Pro Thr Gln Gly Glu Ala Thr Leu
   1               5                   10                  15
   Val Glu Glu Gln Asp Thr Asn Phe Val Cys Arg Arg Thr Phe Val Asp
               20                  25                  30
   Arg Gly Trp Gly Asn Gly Cys Gly Leu Phe Gly Lys Gly Ser Leu Ile
           35                  40                  45
   Thr Cys Ala Lys Phe Lys Cys Val
       50                  55
<210> 6
   <211> 56
   <212> PRT
   <213> Dengue virus
<400> 6
   Asn Thr Thr Thr Glu Ser Arg Cys Pro Thr Gln Gly Glu Pro Ser Leu
   1               5                   10                  15
   Asn Glu Glu Gln Asp Lys Arg Phe Val Cys Lys His Ser Met Val Asp
               20                  25                  30
   Arg Gly Trp Gly Asn Gly Cys Gly Leu Phe Gly Lys Gly Gly Ile Val
           35                  40                  45
   Thr Cys Ala Met Phe Arg Cys Lys
       50                  55
<210> 7
   <211> 56
   <212> PRT
   <213> Dengue virus
<400> 7
   Asn Ile Thr Thr Asp Ser Arg Cys Pro Thr Gln Gly Glu Ala Val Leu
   1               5                   10                  15
   Pro Glu Glu Gln Asp Gln Asn Tyr Val Cys Lys His Thr Tyr Val Asp
               20                  25                  30
   Arg Gly Trp Gly Asn Gly Cys Gly Leu Phe Gly Lys Gly Ser Leu Val
           35                  40                  45
   Thr Cys Ala Lys Phe Gln Cys Leu
       50                  55
<210> 8
   <211> 56
   <212> PRT
   <213> Dengue virus
<400> 8
   Asn Ile Thr Thr Ala Thr Arg Cys Pro Thr Gln Gly Glu Pro Tyr Leu
   1               5                   10                  15
   Lys Glu Glu Gln Asp Gln Gln Tyr Ile Cys Arg Arg Asp Val Val Asp
               20                  25                  30
   Arg Gly Trp Gly Asn Gly Cys Gly Leu Phe Gly Lys Gly Gly Val Val
           35                  40                  45
   Thr Cys Ala Lys Phe Ser Cys Ser
       50                  55
<210> 9
   <211> 9
   <212> PRT
   <213> Artificial Sequence
<220>
   <223> His tag with linker
<400> 9
   Gly Ser Gly His His His His His His
   1               5
<210> 10
   <211> 399
   <212> PRT
   <213> Artificial Sequence
<220>
   <223> mutated form of DENV-1 accession: NP_722460.2; aa 1-399
<400> 10
   Met Arg Cys Val Gly Ile Gly Asn Arg Asp Phe Val Glu Gly Leu Ser
   1               5                   10                  15
   Gly Ala Thr Trp Val Asp Val Val Leu Glu His Gly Ser Cys Val Thr
               20                  25                  30
   Thr Met Ala Lys Asp Lys Pro Thr Leu Asp Ile Glu Leu Leu Lys Thr
           35                  40                  45
   Glu Val Thr Asn Pro Ala Val Leu Arg Lys Leu Cys Ile Glu Ala Lys
       50                  55                  60
   Ile Ser Asn Thr Thr Thr Asp Ser Arg Cys Pro Arg Glu Gly Glu Ala
   65                  70                  75                  80
   Thr Leu Val Glu Glu Gln Asp Thr Asn Phe Val Cys Arg Arg Thr Phe
                   85                  90                  95
   Val Asp Arg Gly Arg Gly Asn Gly Cys Gly Arg Phe Gly Lys Gly Ser
               100                 105                 110
   Leu Ile Thr Cys Ala Lys Phe Lys Cys Val Thr Lys Leu Glu Gly Lys
           115                 120                 125
   Ile Val Gln Tyr Glu Asn Leu Lys Tyr Ser Val Ile Val Thr Val His
       130                 135                 140
   Thr Gly Asp Gln His Gln Val Gly Asn Glu Thr Thr Glu His Gly Thr
   145                 150                 155                 160
   Thr Ala Thr Ile Thr Pro Gln Ala Pro Thr Ser Glu Ile Gln Leu Thr
                   165                 170                 175
   Asp Tyr Gly Ala Leu Thr Leu Asp Cys Ser Pro Arg Thr Gly Leu Asp
               180                 185                 190
   Phe Asn Glu Met Val Leu Leu Thr Met Lys Lys Lys Ser Trp Leu Val
           195                 200                 205
   His Lys Gln Trp Phe Leu Asp Leu Pro Leu Pro Trp Thr Ser Gly Ala
       210                 215                 220
   Ser Thr Ser Gln Glu Thr Trp Asn Arg Gln Asp Leu Leu Val Thr Phe
   225                 230                 235                 240
   Lys Thr Ala His Ala Lys Lys Gln Glu Val Val Val Leu Gly Ser Gln
                   245                 250                 255
   Glu Gly Ala Met His Thr Ala Leu Thr Gly Ala Thr Glu Ile Gln Thr
               260                 265                 270
   Ser Gly Thr Thr Thr Ile Phe Ala Gly His Leu Lys Cys Arg Leu Lys
           275                 280                 285
   Met Asp Lys Leu Ile Leu Lys Gly Met Ser Tyr Val Met Cys Thr Gly
       290                 295                 300
   Ser Phe Lys Leu Glu Lys Glu Val Ala Glu Thr Gln His Gly Thr Val
   305                 310                 315                 320
   Leu Val Gln Val Lys Tyr Glu Gly Thr Asp Ala Pro Cys Lys Ile Pro
                   325                 330                 335
   Phe Ser Ser Gln Asp Glu Lys Gly Val Thr Gln Asn Gly Arg Leu Ile
               340                 345                 350
   Thr Ala Asn Pro Ile Val Thr Asp Lys Glu Lys Pro Val Asn Ile Glu
           355                 360                 365
   Ala Glu Pro Pro Phe Gly Glu Ser Tyr Ile Val Val Gly Ala Gly Glu
       370                 375                 380
   Lys Ala Leu Lys Leu Ser Trp Phe Lys Lys Gly Ser Ser Ile Gly
   385                 390                 395
<210> 11
   <211> 399
   <212> PRT
   <213> Artificial Sequence
<220>
   <223> mutated form of DENV-2 Accession: NP_056776.2 ; aa 1-399
<400> 11
   Met Arg Cys Ile Gly Met Ser Asn Arg Asp Phe Val Glu Gly Val Ser
   1               5                   10                  15
   Gly Gly Ser Trp Val Asp Ile Val Leu Glu His Gly Ser Cys Val Thr
               20                  25                  30
   Thr Met Ala Lys Asn Lys Pro Thr Leu Asp Phe Glu Leu Ile Lys Thr
           35                  40                  45
   Glu Ala Lys Gln Pro Ala Thr Leu Arg Lys Tyr Cys Ile Glu Ala Lys
       50                  55                  60
   Leu Thr Asn Thr Thr Thr Glu Ser Arg Cys Pro Arg Glu Gly Glu Pro
   65                  70                  75                  80
   Ser Leu Asn Glu Glu Gln Asp Lys Arg Phe Val Cys Lys His Ser Met
                   85                  90                  95
   Val Asp Arg Gly Arg Gly Asn Gly Cys Gly Arg Phe Gly Lys Gly Gly
               100                 105                 110
   Ile Val Thr Cys Ala Met Phe Arg Cys Lys Lys Asn Met Glu Gly Lys
           115                 120                 125
   Val Val Gln Pro Glu Asn Leu Glu Tyr Thr Ile Val Ile Thr Pro His
       130                 135                 140
   Ser Gly Glu Glu His Ala Val Gly Asn Asp Thr Gly Lys His Gly Lys
   145                 150                 155                 160
   Glu Ile Lys Ile Thr Pro Gln Ser Ser Ile Thr Glu Ala Glu Leu Thr
                   165                 170                 175
   Gly Tyr Gly Thr Val Thr Met Glu Cys Ser Pro Arg Thr Gly Leu Asp
               180                 185                 190
   Phe Asn Glu Met Val Leu Leu Gln Met Glu Asn Lys Ala Trp Leu Val
           195                 200                 205
   His Arg Gln Trp Phe Leu Asp Leu Pro Leu Pro Trp Leu Pro Gly Ala
       210                 215                 220
   Asp Thr Gln Gly Ser Asn Trp Ile Gln Lys Glu Thr Leu Val Thr Phe
   225                 230                 235                 240
   Lys Asn Pro His Ala Lys Lys Gln Asp Val Val Val Leu Gly Ser Gln
                   245                 250                 255
   Glu Gly Ala Met His Thr Ala Leu Thr Gly Ala Thr Glu Ile Gln Met
               260                 265                 270
   Ser Ser Gly Asn Leu Leu Phe Thr Gly His Leu Lys Cys Arg Leu Arg
           275                 280                 285
   Met Asp Lys Leu Gln Leu Lys Gly Met Ser Tyr Ser Met Cys Thr Gly
       290                 295                 300
   Lys Phe Lys Val Val Lys Glu Ile Ala Glu Thr Gln His Gly Thr Ile
   305                 310                 315                 320
   Val Ile Arg Val Gln Tyr Glu Gly Asp Gly Ser Pro Cys Lys Ile Pro
                   325                 330                 335
   Phe Glu Ile Met Asp Leu Glu Lys Arg His Val Leu Gly Arg Leu Ile
               340                 345                 350
   Thr Val Asn Pro Ile Val Thr Glu Lys Asp Ser Pro Val Asn Ile Glu
           355                 360                 365
   Ala Glu Pro Pro Phe Gly Asp Ser Tyr Ile Ile Ile Gly Val Glu Pro
       370                 375                 380
   Gly Gln Leu Lys Leu Asn Trp Phe Lys Lys Gly Ser Ser Ile Gly
   385                 390                 395
<210> 12
   <211> 397
   <212> PRT
   <213> Artificial Sequence
<220>
   <223> mutated form of DENV-3 accession: YP_001531168.2
<400> 12
   Met Arg Cys Val Gly Val Gly Asn Arg Asp Phe Val Glu Gly Leu Ser
   1               5                   10                  15
   Gly Ala Thr Trp Val Asp Val Val Leu Glu His Gly Gly Cys Val Thr
               20                  25                  30
   Thr Met Ala Lys Asn Lys Pro Thr Leu Asp Ile Glu Leu Gln Lys Thr
           35                  40                  45
   Glu Ala Thr Gln Leu Ala Thr Leu Arg Lys Leu Cys Ile Glu Gly Lys
       50                  55                  60
   Ile Thr Asn Ile Thr Thr Asp Ser Arg Cys Pro Arg Glu Gly Glu Ala
   65                  70                  75                  80
   Val Leu Pro Glu Glu Gln Asp Gln Asn Tyr Val Cys Lys His Thr Tyr
                   85                  90                  95
   Val Asp Arg Gly Arg Gly Asn Gly Cys Gly Arg Phe Gly Lys Gly Ser
               100                 105                 110
   Leu Val Thr Cys Ala Lys Phe Gln Cys Leu Glu Pro Ile Glu Gly Lys
           115                 120                 125
   Val Val Gln Tyr Glu Asn Leu Lys Tyr Thr Val Ile Ile Thr Val His
       130                 135                 140
   Thr Gly Asp Gln His Gln Val Gly Asn Glu Thr Gln Gly Val Thr Ala
   145                 150                 155                 160
   Glu Ile Thr Pro Gln Ala Ser Thr Thr Glu Ala Ile Leu Pro Glu Tyr
                   165                 170                 175
   Gly Thr Leu Gly Leu Glu Cys Ser Pro Arg Thr Gly Leu Asp Phe Asn
               180                 185                 190
   Glu Met Ile Leu Leu Thr Met Lys Asn Lys Ala Trp Met Val His Arg
           195                 200                 205
   Gln Trp Phe Phe Asp Leu Pro Leu Pro Trp Ala Ser Gly Ala Thr Thr
       210                 215                 220
   Glu Thr Pro Thr Trp Asn Arg Lys Glu Leu Leu Val Thr Phe Lys Asn
   225                 230                 235                 240
   Ala His Ala Lys Lys Gln Glu Val Val Val Leu Gly Ser Gln Glu Gly
                   245                 250                 255
   Ala Met His Thr Ala Leu Thr Gly Ala Thr Glu Ile Gln Asn Ser Gly
               260                 265                 270
   Gly Thr Ser Ile Phe Ala Gly His Leu Lys Cys Arg Leu Lys Met Asp
           275                 280                 285
   Lys Leu Glu Leu Lys Gly Met Ser Tyr Ala Met Cys Thr Asn Thr Phe
       290                 295                 300
   Val Leu Lys Lys Glu Val Ser Glu Thr Gln His Gly Thr Ile Leu Ile
   305                 310                 315                 320
   Lys Val Glu Tyr Lys Gly Glu Asp Ala Pro Cys Lys Ile Pro Phe Ser
                   325                 330                 335
   Thr Glu Asp Gly Gln Gly Lys Ala His Asn Gly Arg Leu Ile Thr Ala
               340                 345                 350
   Asn Pro Val Val Thr Lys Lys Glu Glu Pro Val Asn Ile Glu Ala Glu
           355                 360                 365
   Pro Pro Phe Gly Glu Ser Asn Ile Val Ile Gly Ile Gly Asp Asn Ala
       370                 375                 380
   Leu Lys Ile Asn Trp Tyr Lys Lys Gly Ser Ser Ile Gly
   385                 390                 395
<210> 13
   <211> 399
   <212> PRT
   <213> Artificial Sequence
<220>
   <223> mutated form of DENV-4 accession: NP_740317.1
<400> 13
   Met Arg Cys Val Gly Val Gly Asn Arg Asp Phe Val Glu Gly Val Ser
   1               5                   10                  15
   Gly Gly Ala Trp Val Asp Leu Val Leu Glu His Gly Gly Cys Val Thr
               20                  25                  30
   Thr Met Ala Gln Gly Lys Pro Thr Leu Asp Phe Glu Leu Thr Lys Thr
           35                  40                  45
   Thr Ala Lys Glu Val Ala Leu Leu Arg Thr Tyr Cys Ile Glu Ala Ser
       50                  55                  60
   Ile Ser Asn Ile Thr Thr Ala Thr Arg Cys Pro Arg Glu Gly Glu Pro
   65                  70                  75                  80
   Tyr Leu Lys Glu Glu Gln Asp Gln Gln Tyr Ile Cys Arg Arg Asp Val
                   85                  90                  95
   Val Asp Arg Gly Arg Gly Asn Gly Cys Gly Arg Phe Gly Lys Gly Gly
               100                 105                 110
   Val Val Thr Cys Ala Lys Phe Ser Cys Ser Gly Lys Ile Thr Gly Asn
           115                 120                 125
   Leu Val Gln Ile Glu Asn Leu Glu Tyr Thr Val Val Val Thr Val His
       130                 135                 140
   Asn Gly Asp Thr His Ala Val Gly Asn Asp Thr Ser Asn His Gly Val
   145                 150                 155                 160
   Thr Ala Met Ile Thr Pro Arg Ser Pro Ser Val Glu Val Lys Leu Pro
                   165                 170                 175
   Asp Tyr Gly Glu Leu Thr Leu Asp Cys Glu Pro Arg Ser Gly Ile Asp
               180                 185                 190
   Phe Asn Glu Met Ile Leu Met Lys Met Lys Lys Lys Thr Trp Leu Val
           195                 200                 205
   His Lys Gln Trp Phe Leu Asp Leu Pro Leu Pro Trp Thr Ala Gly Ala
       210                 215                 220
   Asp Thr Ser Glu Val His Trp Asn Tyr Lys Glu Arg Met Val Thr Phe
   225                 230                 235                 240
   Lys Val Pro His Ala Lys Arg Gln Asp Val Thr Val Leu Gly Ser Gln
                   245                 250                 255
   Glu Gly Ala Met His Ser Ala Leu Ala Gly Ala Thr Glu Val Asp Ser
               260                 265                 270
   Gly Asp Gly Asn His Met Phe Ala Gly His Leu Lys Cys Lys Val Arg
           275                 280                 285
   Met Glu Lys Leu Arg Ile Lys Gly Met Ser Tyr Thr Met Cys Ser Gly
       290                 295                 300
   Lys Phe Ser Ile Asp Lys Glu Met Ala Glu Thr Gln His Gly Thr Thr
   305                 310                 315                 320
   Val Val Lys Val Lys Tyr Glu Gly Ala Gly Ala Pro Cys Lys Val Pro
                   325                 330                 335
   Ile Glu Ile Arg Asp Val Asn Lys Glu Lys Val Val Gly Arg Ile Ile
               340                 345                 350
   Ser Ser Thr Pro Leu Ala Glu Asn Thr Asn Ser Val Thr Asn Ile Glu
           355                 360                 365
   Leu Glu Pro Pro Phe Gly Asp Ser Tyr Ile Val Ile Gly Val Gly Asn
       370                 375                 380
   Ser Ala Leu Thr Leu His Trp Phe Arg Lys Gly Ser Ser Ile Gly
   385                 390                 395
<210> 14
   <211> 495
   <212> PRT
   <213> Dengue virus
<400> 14
   Met Arg Cys Val Gly Ile Gly Asn Arg Asp Phe Val Glu Gly Leu Ser
   1               5                   10                  15
   Gly Ala Thr Trp Val Asp Val Val Leu Glu His Gly Ser Cys Val Thr
               20                  25                  30
   Thr Met Ala Lys Asp Lys Pro Thr Leu Asp Ile Glu Leu Leu Lys Thr
           35                  40                  45
   Glu Val Thr Asn Pro Ala Val Leu Arg Lys Leu Cys Ile Glu Ala Lys
       50                  55                  60
   Ile Ser Asn Thr Thr Thr Asp Ser Arg Cys Pro Thr Gln Gly Glu Ala
   65                  70                  75                  80
   Thr Leu Val Glu Glu Gln Asp Thr Asn Phe Val Cys Arg Arg Thr Phe
                   85                  90                  95
   Val Asp Arg Gly Trp Gly Asn Gly Cys Gly Leu Phe Gly Lys Gly Ser
               100                 105                 110
   Leu Ile Thr Cys Ala Lys Phe Lys Cys Val Thr Lys Leu Glu Gly Lys
           115                 120                 125
   Ile Val Gln Tyr Glu Asn Leu Lys Tyr Ser Val Ile Val Thr Val His
       130                 135                 140
   Thr Gly Asp Gln His Gln Val Gly Asn Glu Thr Thr Glu His Gly Thr
   145                 150                 155                 160
   Thr Ala Thr Ile Thr Pro Gln Ala Pro Thr Ser Glu Ile Gln Leu Thr
                   165                 170                 175
   Asp Tyr Gly Ala Leu Thr Leu Asp Cys Ser Pro Arg Thr Gly Leu Asp
               180                 185                 190
   Phe Asn Glu Met Val Leu Leu Thr Met Lys Lys Lys Ser Trp Leu Val
           195                 200                 205
   His Lys Gln Trp Phe Leu Asp Leu Pro Leu Pro Trp Thr Ser Gly Ala
       210                 215                 220
   Ser Thr Ser Gln Glu Thr Trp Asn Arg Gln Asp Leu Leu Val Thr Phe
   225                 230                 235                 240
   Lys Thr Ala His Ala Lys Lys Gln Glu Val Val Val Leu Gly Ser Gln
                   245                 250                 255
   Glu Gly Ala Met His Thr Ala Leu Thr Gly Ala Thr Glu Ile Gln Thr
               260                 265                 270
   Ser Gly Thr Thr Thr Ile Phe Ala Gly His Leu Lys Cys Arg Leu Lys
           275                 280                 285
   Met Asp Lys Leu Ile Leu Lys Gly Met Ser Tyr Val Met Cys Thr Gly
       290                 295                 300
   Ser Phe Lys Leu Glu Lys Glu Val Ala Glu Thr Gln His Gly Thr Val
   305                 310                 315                 320
   Leu Val Gln Val Lys Tyr Glu Gly Thr Asp Ala Pro Cys Lys Ile Pro
                   325                 330                 335
   Phe Ser Ser Gln Asp Glu Lys Gly Val Thr Gln Asn Gly Arg Leu Ile
               340                 345                 350
   Thr Ala Asn Pro Ile Val Thr Asp Lys Glu Lys Pro Val Asn Ile Glu
           355                 360                 365
   Ala Glu Pro Pro Phe Gly Glu Ser Tyr Ile Val Val Gly Ala Gly Glu
       370                 375                 380
   Lys Ala Leu Lys Leu Ser Trp Phe Lys Lys Gly Ser Ser Ile Gly Lys
   385                 390                 395                 400
   Met Phe Glu Ala Thr Ala Arg Gly Ala Arg Arg Met Ala Ile Leu Gly
                   405                 410                 415
   Asp Thr Ala Trp Asp Phe Gly Ser Ile Gly Gly Val Phe Thr Ser Val
               420                 425                 430
   Gly Lys Leu Ile His Gln Ile Phe Gly Thr Ala Tyr Gly Val Leu Phe
           435                 440                 445
   Ser Gly Val Ser Trp Thr Met Lys Ile Gly Ile Gly Ile Leu Leu Thr
       450                 455                 460
   Trp Leu Gly Leu Asn Ser Arg Ser Thr Ser Leu Ser Met Thr Cys Ile
   465                 470                 475                 480
   Ala Val Gly Met Val Thr Leu Tyr Leu Gly Val Met Val Gln Ala
                   485                 490                 495
<210> 15
   <211> 495
   <212> PRT
   <213> Dengue virus
<400> 15
   Met Arg Cys Ile Gly Met Ser Asn Arg Asp Phe Val Glu Gly Val Ser
   1               5                   10                  15
   Gly Gly Ser Trp Val Asp Ile Val Leu Glu His Gly Ser Cys Val Thr
               20                  25                  30
   Thr Met Ala Lys Asn Lys Pro Thr Leu Asp Phe Glu Leu Ile Lys Thr
           35                  40                  45
   Glu Ala Lys Gln Pro Ala Thr Leu Arg Lys Tyr Cys Ile Glu Ala Lys
       50                  55                  60
   Leu Thr Asn Thr Thr Thr Glu Ser Arg Cys Pro Thr Gln Gly Glu Pro
   65                  70                  75                  80
   Ser Leu Asn Glu Glu Gln Asp Lys Arg Phe Val Cys Lys His Ser Met
                   85                  90                  95
   Val Asp Arg Gly Trp Gly Asn Gly Cys Gly Leu Phe Gly Lys Gly Gly
               100                 105                 110
   Ile Val Thr Cys Ala Met Phe Arg Cys Lys Lys Asn Met Glu Gly Lys
           115                 120                 125
   Val Val Gln Pro Glu Asn Leu Glu Tyr Thr Ile Val Ile Thr Pro His
       130                 135                 140
   Ser Gly Glu Glu His Ala Val Gly Asn Asp Thr Gly Lys His Gly Lys
   145                 150                 155                 160
   Glu Ile Lys Ile Thr Pro Gln Ser Ser Ile Thr Glu Ala Glu Leu Thr
                   165                 170                 175
   Gly Tyr Gly Thr Val Thr Met Glu Cys Ser Pro Arg Thr Gly Leu Asp
               180                 185                 190
   Phe Asn Glu Met Val Leu Leu Gln Met Glu Asn Lys Ala Trp Leu Val
           195                 200                 205
   His Arg Gln Trp Phe Leu Asp Leu Pro Leu Pro Trp Leu Pro Gly Ala
       210                 215                 220
   Asp Thr Gln Gly Ser Asn Trp Ile Gln Lys Glu Thr Leu Val Thr Phe
   225 230 235 240
   Lys Asn Pro His Ala Lys Lys Gln Asp Val Val Val Leu Gly Ser Gln
   245 250 255
   Glu Gly Ala Met His Thr Ala Leu Thr Gly Ala Thr Glu Ile Gln Met
   260 265 270
   Ser Ser Gly Asn Leu Leu Phe Thr Gly His Leu Lys Cys Arg Leu Arg
   275 280 285
   Met Asp Lys Leu Gln Leu Lys Gly Met Ser Tyr Ser Met Cys Thr Gly
   290 295 300
   Lys Phe Lys Val Val Lys Glu Ile Ala Glu Thr Gln His Gly Thr Ile
   305 310 315 320
   Val Ile Arg Val Gln Tyr Glu Gly Asp Gly Ser Pro Cys Lys Ile Pro
   325 330 335
   Phe Glu Ile Met Asp Leu Glu Lys Arg His Val Leu Gly Arg Leu Ile
   340 345 350
   Thr Val Asn Pro Ile Val Thr Glu Lys Asp Ser Pro Val Asn Ile Glu
   355 360 365
   Ala Glu Pro Pro Phe Gly Asp Ser Tyr Ile Ile Ile Gly Val Glu Pro
   370 375 380
   Gly Gln Leu Lys Leu Asn Trp Phe Lys Lys Gly Ser Ser Ile Gly Gln
   385 390 395 400
   Met Phe Glu Thr Thr Met Arg Gly Ala Lys Arg Met Ala Ile Leu Gly
   405 410 415
   Asp Thr Ala Trp Asp Phe Gly Ser Leu Gly Gly Val Phe Thr Ser Ile
   420 425 430
   Gly Lys Ala Leu His Gln Val Phe Gly Ala Ile Tyr Gly Ala Ala Phe
   435 440 445
   Ser Gly Val Ser Trp Thr Met Lys Ile Leu Ile Gly Val Ile Ile Thr
   450 455 460
   Trp Ile Gly Met Asn Ser Arg Ser Thr Ser Leu Ser Val Thr Leu Val
   465 470 475 480
   Leu Val Gly Ile Val Thr Leu Tyr Leu Gly Val Met Val Gln Ala
   485 490 495
<210> 16
   <211> 493
   <212> PRT
   <213> Dengue virus
<400> 16
   Met Arg Cys Val Gly Val Gly Asn Arg Asp Phe Val Glu Gly Leu Ser
   1 5 10 15
   Gly Ala Thr Trp Val Asp Val Val Leu Glu His Gly Gly Cys Val Thr
   20 25 30
   Thr Met Ala Lys Asn Lys Pro Thr Leu Asp Ile Glu Leu Gln Lys Thr
   35 40 45
   Glu Ala Thr Gln Leu Ala Thr Leu Arg Lys Leu Cys Ile Glu Gly Lys
   50 55 60
   Ile Thr Asn Ile Thr Thr Asp Ser Arg Cys Pro Thr Gln Gly Glu Ala
   65 70 75 80
   Val Leu Pro Glu Glu Gln Asp Gln Asn Tyr Val Cys Lys His Thr Tyr
   85 90 95
   Val Asp Arg Gly Trp Gly Asn Gly Cys Gly Leu Phe Gly Lys Gly Ser
   100 105 110
   Leu Val Thr Cys Ala Lys Phe Gln Cys Leu Glu Pro Ile Glu Gly Lys
   115 120 125
   Val Val Gln Tyr Glu Asn Leu Lys Tyr Thr Val Ile Ile Thr Val His
   130 135 140
   Thr Gly Asp Gln His Gln Val Gly Asn Glu Thr Gln Gly Val Thr Ala
   145 150 155 160
   Glu Ile Thr Pro Gln Ala Ser Thr Thr Glu Ala Ile Leu Pro Glu Tyr
   165 170 175
   Gly Thr Leu Gly Leu Glu Cys Ser Pro Arg Thr Gly Leu Asp Phe Asn
   180 185 190
   Glu Met Ile Leu Leu Thr Met Lys Asn Lys Ala Trp Met Val His Arg
   195 200 205
   Gln Trp Phe Phe Asp Leu Pro Leu Pro Trp Ala Ser Gly Ala Thr Thr
       210                 215                 220
   Glu Thr Pro Thr Trp Asn Arg Lys Glu Leu Leu Val Thr Phe Lys Asn
   225                 230                 235                 240
   Ala His Ala Lys Lys Gln Glu Val Val Val Leu Gly Ser Gln Glu Gly
                   245                 250                 255
   Ala Met His Thr Ala Leu Thr Gly Ala Thr Glu Ile Gln Asn Ser Gly
               260                 265                 270
   Gly Thr Ser Ile Phe Ala Gly His Leu Lys Cys Arg Leu Lys Met Asp
           275                 280                 285
   Lys Leu Glu Leu Lys Gly Met Ser Tyr Ala Met Cys Thr Asn Thr Phe
       290                 295                 300
   Val Leu Lys Lys Glu Val Ser Glu Thr Gln His Gly Thr Ile Leu Ile
   305                 310                 315                 320
   Lys Val Glu Tyr Lys Gly Glu Asp Ala Pro Cys Lys Ile Pro Phe Ser
                   325                 330                 335
   Thr Glu Asp Gly Gln Gly Lys Ala His Asn Gly Arg Leu Ile Thr Ala
               340                 345                 350
   Asn Pro Val Val Thr Lys Lys Glu Glu Pro Val Asn Ile Glu Ala Glu
           355                 360                 365
   Pro Pro Phe Gly Glu Ser Asn Ile Val Ile Gly Ile Gly Asp Asn Ala
       370                 375                 380
   Leu Lys Ile Asn Trp Tyr Lys Lys Gly Ser Ser Ile Gly Lys Met Phe
   385                 390                 395                 400
   Glu Ala Thr Glu Arg Gly Ala Arg Arg Met Ala Ile Leu Gly Asp Thr
                   405                 410                 415
   Ala Trp Asp Phe Gly Ser Val Gly Gly Val Leu Asn Ser Leu Gly Lys
               420                 425                 430
   Met Val His Gln Ile Phe Gly Ser Ala Tyr Thr Ala Leu Phe Ser Gly
           435                 440                 445
   Val Ser Trp Val Met Lys Ile Gly Ile Gly Val Leu Leu Thr Trp Ile
   450 455 460
   Gly Leu Asn Ser Lys Asn Thr Ser Met Ser Phe Ser Cys Ile Ala Ile
   465 470 475 480
   Gly Ile Ile Thr Leu Tyr Leu Gly Ala Val Val Gln Ala
   485 490
<210> 17
   <211> 495
   <212> PRT
   <213> Dengue virus
<400> 17
   Met Arg Cys Val Gly Val Gly Asn Arg Asp Phe Val Glu Gly Val Ser
   1               5                   10                  15
   Gly Gly Ala Trp Val Asp Leu Val Leu Glu His Gly Gly Cys Val Thr
               20                  25                  30
   Thr Met Ala Gln Gly Lys Pro Thr Leu Asp Phe Glu Leu Thr Lys Thr
           35                  40                  45
   Thr Ala Lys Glu Val Ala Leu Leu Arg Thr Tyr Cys Ile Glu Ala Ser
       50                  55                  60
   Ile Ser Asn Ile Thr Thr Ala Thr Arg Cys Pro Thr Gln Gly Glu Pro
   65                  70                  75                  80
   Tyr Leu Lys Glu Glu Gln Asp Gln Gln Tyr Ile Cys Arg Arg Asp Val
                   85                  90                  95
   Val Asp Arg Gly Trp Gly Asn Gly Cys Gly Leu Phe Gly Lys Gly Gly
               100                 105                 110
   Val Val Thr Cys Ala Lys Phe Ser Cys Ser Gly Lys Ile Thr Gly Asn
           115                 120                 125
   Leu Val Gln Ile Glu Asn Leu Glu Tyr Thr Val Val Val Thr Val His
       130                 135                 140
   Asn Gly Asp Thr His Ala Val Gly Asn Asp Thr Ser Asn His Gly Val
   145                 150                 155                 160
   Thr Ala Met Ile Thr Pro Arg Ser Pro Ser Val Glu Val Lys Leu Pro
                   165                 170                 175
   Asp Tyr Gly Glu Leu Thr Leu Asp Cys Glu Pro Arg Ser Gly Ile Asp
               180                 185                 190
   Phe Asn Glu Met Ile Leu Met Lys Met Lys Lys Lys Thr Trp Leu Val
           195                 200                 205
   His Lys Gln Trp Phe Leu Asp Leu Pro Leu Pro Trp Thr Ala Gly Ala
       210                 215                 220
   Asp Thr Ser Glu Val His Trp Asn Tyr Lys Glu Arg Met Val Thr Phe
   225                 230                 235                 240
   Lys Val Pro His Ala Lys Arg Gln Asp Val Thr Val Leu Gly Ser Gln
                   245                 250                 255
   Glu Gly Ala Met His Ser Ala Leu Ala Gly Ala Thr Glu Val Asp Ser
               260                 265                 270
   Gly Asp Gly Asn His Met Phe Ala Gly His Leu Lys Cys Lys Val Arg
           275                 280                 285
   Met Glu Lys Leu Arg Ile Lys Gly Met Ser Tyr Thr Met Cys Ser Gly
       290                 295                 300
   Lys Phe Ser Ile Asp Lys Glu Met Ala Glu Thr Gln His Gly Thr Thr
   305                 310                 315                 320
   Val Val Lys Val Lys Tyr Glu Gly Ala Gly Ala Pro Cys Lys Val Pro
                   325                 330                 335
   Ile Glu Ile Arg Asp Val Asn Lys Glu Lys Val Val Gly Arg Ile Ile
               340                 345                 350
   Ser Ser Thr Pro Leu Ala Glu Asn Thr Asn Ser Val Thr Asn Ile Glu
           355                 360                 365
   Leu Glu Pro Pro Phe Gly Asp Ser Tyr Ile Val Ile Gly Val Gly Asn
       370                 375                 380
   Ser Ala Leu Thr Leu His Trp Phe Arg Lys Gly Ser Ser Ile Gly Lys
   385                 390                 395                 400
   Met Phe Glu Ser Thr Tyr Arg Gly Ala Lys Arg Met Ala Ile Leu Gly
                   405                 410                 415
   Glu Thr Ala Trp Asp Phe Gly Ser Val Gly Gly Leu Phe Thr Ser Leu
               420                 425                 430
   Gly Lys Ala Val His Gln Val Phe Gly Ser Val Tyr Thr Thr Met Phe
           435                 440                 445
   Gly Gly Val Ser Trp Met Ile Arg Ile Leu Ile Gly Phe Leu Val Leu
       450                 455                 460
   Trp Ile Gly Thr Asn Ser Arg Asn Thr Ser Met Ala Met Thr Cys Ile
   465                 470                 475                 480
   Ala Val Gly Gly Ile Thr Leu Phe Leu Gly Phe Thr Val Gln Ala
                   485                 490                 495
<210> 18
   <211> 495
   <212> PRT
   <213> Dengue virus
<400> 18
   Met Arg Cys Val Gly Ile Gly Ser Arg Asp Phe Val Glu Gly Leu Ser
   1               5                   10                  15
   Gly Ala Thr Trp Val Asp Val Val Leu Glu His Gly Ser Cys Val Thr
               20                  25                  30
   Thr Met Ala Lys Asp Lys Pro Thr Leu Asp Ile Glu Leu Leu Lys Thr
           35                  40                  45
   Glu Val Thr Asn Pro Ala Val Leu Arg Lys Leu Cys Ile Glu Ala Lys
       50                  55                  60
   Ile Ser Asn Thr Thr Thr Asp Ser Arg Cys Pro Thr Gln Gly Glu Ala
   65                  70                  75                  80
   Thr Leu Val Glu Glu Gln Asp Ala Asn Phe Val Cys Arg Arg Thr Phe
                   85                  90                  95
   Val Asp Arg Gly Trp Gly Asn Gly Cys Gly Leu Phe Gly Lys Gly Ser
               100                 105                 110
   Leu Ile Thr Cys Ala Lys Phe Lys Cys Val Thr Lys Leu Glu Gly Lys
           115                 120                 125
   Ile Val Gln Tyr Glu Asn Leu Lys Tyr Ser Val Ile Val Thr Val His
       130                 135                 140
   Thr Gly Asp Gln His Gln Val Gly Asn Glu Ser Thr Glu His Gly Thr
   145                 150                 155                 160
   Thr Ala Thr Ile Thr Pro Gln Ala Pro Thr Thr Glu Ile Gln Leu Thr
   165 170 175
   Asp Tyr Gly Ala Leu Thr Leu Asp Cys Ser Pro Arg Thr Gly Leu Asp
   180 185 190
   Phe Asn Glu Met Val Leu Leu Thr Met Lys Glu Lys Ser Trp Leu Val
   195 200 205
   His Lys Gln Trp Phe Leu Asp Leu Pro Leu Pro Trp Thr Ser Gly Ala
   210 215 220
   Ser Thr Ser Gln Glu Thr Trp Asn Arg Gln Asp Leu Leu Val Thr Phe
   225 230 235 240
   Lys Thr Ala His Ala Lys Lys Gln Glu Val Val Val Leu Gly Ser Gln
   245 250 255
   Glu Gly Ala Met His Thr Ala Leu Thr Gly Ala Thr Glu Ile Gln Thr
   260 265 270
   Ser Gly Thr Thr Thr Ile Phe Ala Gly His Leu Lys Cys Arg Leu Lys
   275 280 285
   Met Asp Lys Leu Thr Leu Lys Gly Met Ser Tyr Val Met Cys Thr Gly
   290 295 300
   Ser Phe Lys Leu Glu Lys Glu Leu Ala Glu Thr Gln His Gly Thr Val
   305 310 315 320
   Leu Val Gln Ile Lys Tyr Glu Gly Thr Asp Ala Pro Cys Lys Ile Pro
   325 330 335
   Phe Ser Thr Gln Asp Glu Lys Gly Val Thr Gln Asn Gly Arg Leu Ile
   340 345 350
   Thr Ala Asn Pro Ile Val Thr Asp Lys Glu Lys Pro Val Asn Ile Glu
   355 360 365
   Ala Glu Pro Pro Phe Gly Glu Ser Tyr Ile Val Ile Gly Ala Gly Glu
   370 375 380
   Lys Ala Leu Lys Leu Ser Trp Phe Lys Lys Gly Ser Ser Ile Gly Lys
   385 390 395 400
   Met Phe Glu Ala Thr Ala Arg Gly Ala Arg Arg Met Ala Ile Leu Gly
   405 410 415
   Asp Thr Ala Trp Asp Phe Gly Ser Ile Gly Gly Val Phe Thr Ser Val
               420                 425                 430
   Gly Lys Leu Val His Gln Ile Phe Gly Thr Ala Tyr Gly Val Leu Phe
           435                 440                 445
   Ser Gly Val Ser Trp Thr Met Lys Ile Gly Ile Gly Val Leu Leu Thr
       450                 455                 460
   Trp Leu Gly Leu Asn Ser Arg Ser Thr Ser Leu Ser Met Thr Cys Ile
   465                 470                 475                 480
   Ala Val Gly Leu Val Thr Leu Tyr Leu Gly Val Met Val Gln Ala
                   485                 490                 495
<210> 19
   <211> 495
   <212> PRT
   <213> Dengue virus
<400> 19
   Met Arg Cys Ile Gly Ile Ser Asn Arg Asp Phe Val Glu Gly Val Ser
   1               5                   10                  15
   Gly Gly Ser Trp Val Asp Ile Val Leu Glu His Gly Ser Cys Val Thr
               20                  25                  30
   Thr Met Ala Lys Asn Lys Pro Thr Leu Asp Phe Glu Leu Ile Lys Thr
           35                  40                  45
   Glu Ala Lys Gln Pro Ala Thr Leu Arg Lys Tyr Cys Ile Glu Ala Lys
       50                  55                  60
   Leu Thr Asn Thr Thr Thr Glu Ser Arg Cys Pro Thr Gln Gly Glu Pro
   65                  70                  75                  80
   Ser Leu Lys Glu Glu Gln Asp Lys Arg Phe Val Cys Lys His Ser Met
                   85                  90                  95
   Val Asp Arg Gly Trp Gly Asn Gly Cys Gly Leu Phe Gly Lys Gly Gly
               100                 105                 110
   Ile Val Thr Cys Ala Met Phe Thr Cys Lys Lys Asn Met Glu Gly Lys
           115                 120                 125
   Ile Val Gln Pro Glu Asn Leu Glu Tyr Thr Ile Val Val Thr Pro His
       130                 135                 140
   Ser Gly Glu Glu His Ala Val Gly Asn Asp Thr Gly Lys His Gly Lys
   145 150 155 160
   Glu Ile Lys Val Thr Pro Gln Ser Ser Ile Thr Glu Ala Glu Leu Thr
   165 170 175
   Gly Tyr Gly Thr Val Thr Met Glu Cys Ser Pro Arg Thr Gly Leu Asp
   180 185 190
   Phe Asn Glu Met Val Leu Leu Gln Met Glu Asn Lys Ala Trp Leu Val
   195 200 205
   His Arg Gln Trp Phe Leu Asp Leu Pro Leu Pro Trp Leu Pro Gly Ala
   210 215 220
   Asp Lys Gln Glu Ser Asn Trp Ile Gln Lys Glu Thr Leu Val Thr Phe
   225 230 235 240
   Lys Asn Pro His Ala Lys Lys Gln Asp Val Val Val Leu Gly Ser Gln
   245 250 255
   Glu Gly Ala Met His Thr Ala Leu Thr Gly Ala Thr Glu Ile Gln Met
   260 265 270
   Ser Ser Gly Asn Leu Leu Phe Thr Gly His Leu Lys Cys Arg Leu Arg
   275 280 285
   Met Asp Lys Leu Gln Leu Lys Gly Met Ser Tyr Ser Met Cys Thr Gly
   290 295 300
   Lys Phe Lys Val Val Lys Glu Ile Ala Glu Thr Gln His Gly Thr Ile
   305 310 315 320
   Val Ile Arg Val Gln Tyr Glu Gly Asp Gly Ser Pro Cys Lys Ile Pro
   325 330 335
   Phe Glu Ile Met Asp Leu Glu Lys Arg Tyr Val Leu Gly Arg Leu Ile
   340 345 350
   Thr Val Asn Pro Ile Val Thr Glu Lys Asp Ser Pro Val Asn Ile Glu
   355 360 365
   Ala Glu Pro Pro Phe Gly Asp Ser Tyr Ile Ile Ile Gly Val Glu Pro
   370 375 380
   Gly Gln Leu Lys Leu Asn Trp Phe Lys Lys Gly Ser Ser Ile Gly Gln
   385 390 395 400
   Met Phe Glu Thr Thr Met Arg Gly Ala Lys Arg Met Ala Ile Leu Gly
                   405                 410                 415
   Asp Thr Ala Trp Asp Phe Gly Ser Leu Gly Gly Val Phe Thr Ser Ile
               420                 425                 430
   Gly Lys Ala Leu His Gln Val Phe Gly Ala Ile Tyr Gly Ala Ala Phe
           435                 440                 445
   Ser Gly Val Ser Trp Thr Met Lys Ile Leu Ile Gly Val Ile Ile Thr
       450                 455                 460
   Trp Ile Gly Met Asn Ser Arg Ser Thr Ser Leu Ser Val Ser Leu Val
   465                 470                 475                 480
   Leu Val Gly Ile Val Thr Leu Tyr Leu Gly Val Met Val Gln Ala
                   485                 490                 495
<210> 20
   <211> 493
   <212> PRT
   <213> Dengue virus
<400> 20
   Met Arg Cys Val Gly Val Gly Asn Arg Asp Phe Val Glu Gly Leu Ser
   1               5                   10                  15
   Gly Ala Ser Trp Val Asp Val Val Leu Glu His Gly Gly Cys Val Thr
               20                  25                  30
   Thr Met Ala Lys Asn Lys Pro Thr Leu Asp Ile Glu Leu Gln Lys Thr
           35                  40                  45
   Glu Ala Thr Gln Leu Ala Thr Leu Arg Lys Leu Cys Ile Glu Gly Lys
       50                  55                  60
   Ile Thr Asn Ile Thr Thr Asp Ser Arg Cys Pro Thr Gln Gly Glu Ala
   65                  70                  75                  80
   Ile Leu Pro Glu Glu Gln Asp Gln Asn Tyr Val Cys Lys His Thr Tyr
                   85                  90                  95
   Val Asp Arg Gly Trp Gly Asn Gly Cys Gly Leu Phe Gly Lys Gly Ser
               100                 105                 110
   Leu Val Thr Cys Ala Lys Phe Gln Cys Leu Glu Pro Ile Glu Gly Lys
           115                 120                 125
   Val Val Gln His Glu Asn Leu Lys Tyr Thr Val Ile Ile Thr Val His
       130                 135                 140
   Thr Gly Asp Gln His Gln Val Gly Asn Asp Thr Gln Gly Val Thr Val
   145                 150                 155                 160
   Glu Ile Thr Pro Gln Ala Ser Thr Val Glu Ala Ile Leu Pro Glu Tyr
                   165                 170                 175
   Gly Thr Leu Gly Leu Glu Cys Ser Pro Arg Thr Gly Leu Asp Phe Asn
               180                 185                 190
   Glu Met Ile Leu Leu Thr Met Lys Asn Lys Ala Trp Met Val His Arg
           195                 200                 205
   Gln Trp Phe Phe Asp Leu Pro Leu Pro Trp Thr Ser Gly Ala Thr Thr
       210                 215                 220
   Glu Thr Pro Thr Trp Asn Arg Lys Glu Leu Leu Val Thr Phe Lys Asn
   225                 230                 235                 240
   Ala His Ala Lys Lys Gln Glu Val Val Val Leu Gly Ser Gln Glu Gly
                   245                 250                 255
   Ala Met His Thr Ala Leu Thr Gly Ala Thr Glu Ile Gln Asn Ser Gly
               260                 265                 270
   Gly Thr Ser Ile Phe Ala Gly His Leu Lys Cys Arg Leu Lys Met Asp
           275                 280                 285
   Lys Leu Glu Leu Lys Gly Met Ser Tyr Ala Met Cys Leu Asn Thr Phe
       290                 295                 300
   Val Leu Lys Lys Glu Val Ser Glu Thr Gln His Gly Thr Ile Leu Ile
   305                 310                 315                 320
   Lys Val Glu Tyr Lys Gly Glu Asp Ala Pro Cys Lys Ile Pro Phe Ser
                   325                 330                 335
   Thr Glu Asp Gly Gln Gly Lys Ala His Asn Gly Arg Leu Ile Thr Ala
               340                 345                 350
   Asn Pro Val Val Thr Lys Lys Glu Glu Pro Val Asn Ile Glu Ala Glu
           355                 360                 365
   Pro Pro Phe Gly Glu Ser Asn Ile Val Ile Gly Ile Gly Asp Lys Ala
       370                 375                 380
   Leu Lys Ile Asn Trp Tyr Lys Lys Gly Ser Ser Ile Gly Lys Met Phe
   385                 390                 395                 400
   Glu Ala Thr Ala Arg Gly Ala Arg Arg Met Ala Ile Leu Gly Asp Thr
                   405                 410                 415
   Ala Trp Asp Phe Gly Ser Val Gly Gly Val Leu Asn Ser Leu Gly Lys
               420                 425                 430
   Met Val His Gln Ile Phe Gly Ser Ala Tyr Thr Ala Leu Phe Ser Gly
           435                 440                 445
   Val Ser Trp Ile Met Lys Ile Gly Ile Gly Val Leu Leu Thr Trp Ile
       450                 455                 460
   Gly Leu Asn Ser Lys Asn Thr Ser Met Ser Phe Ser Cys Ile Ala Ile
   465                 470                 475                 480
   Gly Ile Ile Thr Leu Tyr Leu Gly Val Val Val Gln Ala
                   485                 490
<210> 21
   <211> 495
   <212> PRT
   <213> Dengue virus
<400> 21
   Met Arg Cys Val Gly Val Gly Asn Arg Asp Phe Val Glu Gly Val Ser
   1               5                   10                  15
   Gly Gly Ala Trp Val Asp Leu Val Leu Glu His Gly Gly Cys Val Thr
               20                  25                  30
   Thr Met Ala Gln Gly Lys Pro Thr Leu Asp Phe Glu Leu Ile Lys Thr
           35                  40                  45
   Thr Ala Lys Glu Val Ala Leu Leu Arg Thr Tyr Cys Ile Glu Ala Ser
       50                  55                  60
   Ile Ser Asn Ile Thr Thr Ala Thr Arg Cys Pro Thr Gln Gly Glu Pro
   65                  70                  75                  80
   Tyr Leu Lys Glu Glu Gln Asp Gln Gln Tyr Ile Cys Arg Arg Asp Val
                   85                  90                  95
   Val Asp Arg Gly Trp Gly Asn Gly Cys Gly Leu Phe Gly Lys Gly Gly
               100                 105                 110
   Val Val Thr Cys Ala Lys Phe Ser Cys Ser Gly Lys Ile Thr Gly Asn
           115                 120                 125
   Leu Val Gln Ile Glu Asn Leu Glu Tyr Thr Val Val Val Thr Val His
       130                 135                 140
   Asn Gly Asp Thr His Ala Val Gly Asn Asp Thr Ser Asn His Gly Glu
   145                 150                 155                 160
   Thr Ala Thr Ile Thr Pro Arg Ser Pro Ser Val Glu Val Lys Leu Pro
                   165                 170                 175
   Asp Tyr Gly Glu Leu Thr Leu Asp Cys Glu Pro Arg Ser Gly Ile Asp
               180                 185                 190
   Phe Asn Glu Met Ile Leu Met Lys Met Lys Thr Lys Thr Trp Leu Val
           195                 200                 205
   His Lys Gln Trp Phe Leu Asp Leu Pro Leu Pro Trp Thr Ala Gly Ala
       210                 215                 220
   Asp Thr Ser Glu Val His Trp Asn His Lys Glu Arg Met Val Thr Phe
   225                 230                 235                 240
   Lys Val Pro His Ala Lys Arg Gln Asp Val Thr Val Leu Gly Ser Gln
                   245                 250                 255
   Glu Gly Ala Met His Ser Ala Leu Ala Gly Ala Thr Glu Val Asp Ser
               260                 265                 270
   Gly Asp Gly Asn His Met Phe Ala Gly His Leu Lys Cys Lys Val Arg
           275                 280                 285
   Met Glu Lys Leu Arg Ile Lys Gly Met Ser Tyr Thr Met Cys Ser Gly
       290                 295                 300
   Lys Phe Ser Ile Asp Lys Glu Met Ala Glu Thr Gln His Gly Thr Thr
   305                 310                 315                 320
   Val Val Lys Val Lys Tyr Glu Gly Thr Gly Ala Pro Cys Lys Val Pro
                   325                 330                 335
   Ile Glu Ile Arg Asp Val Asn Lys Glu Lys Val Val Gly Arg Ile Ile
               340                 345                 350
   Ser Ser Thr Pro Phe Ala Glu Asn Thr Asn Ser Val Thr Asn Ile Glu
   355 360 365
   Leu Glu Pro Pro Phe Gly Asp Ser Tyr Ile Val Ile Gly Val Gly Asp
   370 375 380
   Ser Ala Leu Thr Leu His Trp Phe Arg Lys Gly Ser Ser Ile Gly Lys
   385 390 395 400
   Met Phe Glu Ser Thr Tyr Arg Gly Ala Lys Arg Met Ala Ile Leu Gly
   405 410 415
   Glu Thr Ala Trp Asp Phe Gly Ser Val Gly Gly Leu Leu Thr Ser Leu
   420 425 430
   Gly Lys Ala Val His Gln Val Phe Gly Ser Val Tyr Thr Thr Met Phe
   435 440 445
   Gly Gly Val Ser Trp Met Val Arg Ile Leu Ile Gly Leu Leu Val Leu
   450 455 460
   Trp Ile Gly Thr Asn Ser Arg Asn Thr Ser Met Ala Met Ser Cys Ile
   465 470 475 480
   Ala Val Gly Gly Ile Thr Leu Phe Leu Gly Phe Thr Val His Ala
   485 490 495
<210> 22
   <211> 56
   <212> PRT
   <213> West Nile virus
<400> 22
   Asp Leu Ser Thr Lys Ala Ala Cys Pro Thr Met Gly Glu Ala His Asn
   1 5 10 15
   Asp Lys Arg Ala Asp Pro Ala Phe Val Cys Arg Gln Gly Val Val Asp
   20 25 30
   Arg Gly Trp Gly Asn Gly Cys Gly Leu Phe Gly Lys Gly Ser Ile Asp
   35 40 45
   Thr Cys Ala Lys Phe Ala Cys Ser
   50 55
<210> 23
   <211> 56
   <212> PRT
   <213> Japanese encephalitis virus
<400> 23
   Asp Ile Ser Thr Val Ala Arg Cys Pro Thr Thr Gly Glu Ala His Asn
   1               5                   10                  15
   Glu Lys Arg Ala Asp Ser Ser Tyr Val Cys Lys Gln Gly Phe Thr Asp
               20                  25                  30
   Arg Gly Trp Gly Asn Gly Cys Gly Leu Phe Gly Lys Gly Ser Ile Asp
           35                  40                  45
   Thr Cys Ala Lys Phe Ser Cys Thr
       50                  55
<210> 24
   <211> 56
   <212> PRT
   <213> Yellow fever virus
<400> 24
   His Val Lys Ile Asn Asp Lys Cys Pro Ser Thr Gly Glu Ala His Leu
   1               5                   10                  15
   Ala Glu Glu Asn Glu Gly Asp Asn Ala Cys Lys Arg Thr Tyr Ser Asp
               20                  25                  30
   Arg Gly Trp Gly Asn Gly Cys Gly Leu Phe Gly Lys Gly Ser Ile Val
           35                  40                  45
   Ala Cys Ala Lys Phe Thr Cys Ala
       50                  55
<210> 25
   <211> 56
   <212> PRT
   <213> Tick-borne encephalitis virus
<400> 25
   Asp Thr Lys Val Ala Ala Arg Cys Pro Thr Met Gly Pro Ala Thr Leu
   1               5                   10                  15
   Ala Glu Glu His Gln Gly Gly Thr Val Cys Lys Arg Asp Gln Ser Asp
               20                  25                  30
   Arg Gly Trp Gly Asn His Cys Gly Leu Phe Gly Lys Gly Ser Ile Val
           35                  40                  45
   Ala Cys Val Lys Ala Ala Cys Glu
       50                  55